(19) Europäisches Patentamt
European Patent Office
Office européen des brevets

(11) EP 2 785 847 B1

(12) **EUROPEAN PATENT SPECIFICATION**

(45) Date of publication and mention of the grant of the patent:
**26.07.2017 Bulletin 2017/30**

(21) Application number: **12799456.4**

(22) Date of filing: **30.11.2012**

(51) Int Cl.:
*C12P 1/04* (2006.01) *C12P 7/06* (2006.01)
*C12N 9/54* (2006.01)

(86) International application number:
**PCT/US2012/067380**

(87) International publication number:
**WO 2013/082486 (06.06.2013 Gazette 2013/23)**

(54) **Liquefaction process with selected alpha-amylases and proteases**

Verflüssigungsverfahren mit ausgewählten Alpha-amylasen und Proteasen

Procédé de liquéfaction avec des alpha-amylases et proteases sélectionnées

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **02.12.2011 US 201161566281 P**

(43) Date of publication of application:
**08.10.2014 Bulletin 2014/41**

(73) Proprietors:
- **Novozymes A/S**
  **2880 Bagsvaerd (DK)**
- **Novozymes North America, Inc.**
  **Franklinton, NC 27525 (US)**

(72) Inventors:
- **DEINHAMMER, Randall**
  **Wake Forest, North Carolina 27587 (US)**
- **CRAIG, Joyce**
  **Pittsboro, North Carolina 27312 (US)**
- **MATSUI, Tomoko**
  **Chiba-shi,**
  **Chiba 261-0004 (JP)**
- **TAKAGI, Shinobu**
  **Funabashi,**
  **Chiba, 274-0825 (JP)**
- **CLARK, Suzanne**
  **Youngsville, North Carolina 27596 (US)**
- **MATTHEWS, John**
  **Louisberg, North Carolina 27549 (US)**
- **HJULMAND, Anne Glud**
  **DK-3070 Snekkersten (DK)**
- **SOONG, Chee-Leong**
  **Raleigh, North Carolina 27614 (US)**

(74) Representative: **Shenker, Paul Dhan**
**Novozymes A/S**
**Patents**
**Krogshoejvej 36**
**2880 Bagsværd (DK)**

(56) References cited:
**WO-A2-2006/086792 WO-A2-2011/072191**
**WO-A2-2012/088303 DE-A1-102005 062 984**
**US-B1- 6 358 726 US-B2- 8 048 657**

- **PEREZ-CARRILLO ET AL: "Addition of protease during starch liquefaction affects free amino nitrogen, fusel alcohols and ethanol production of fermented maizeand whole and decorticated sorghum mashes", BIOCHEMICAL ENGINEERING JOURNAL, vol. 67, 2 May 2012 (2012-05-02), pages 1-9, XP028408603,**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 785 847 B1

Printed by Jouve, 75001 PARIS (FR)

SEQUENCE LISTING

<110> Novozymes A/S
Deinhammer, Randall
Craig, Joyce
Matsui, Tomoko
Takagi, Shinobu
Clark, Suzanne
Matthews, John
Hjulmand, Anne Glud
Soong, Chee-Leong

<120> Processes for Producing Fermentation Products

<130> 12075-WO-PCT

<160> 21

<170> PatentIn version 3.5

<210> 1
<211> 515
<212> PRT
<213> Bacillus stearothermophilus

<220>
<221> mat_peptide
<222> (1)..(515)

<400> 1

```
Ala Ala Pro Phe Asn Gly Thr Met Met Gln Tyr Phe Glu Trp Tyr Leu
1               5                   10                  15

Pro Asp Asp Gly Thr Leu Trp Thr Lys Val Ala Asn Glu Ala Asn Asn
            20                  25                  30

Leu Ser Ser Leu Gly Ile Thr Ala Leu Trp Leu Pro Pro Ala Tyr Lys
        35                  40                  45

Gly Thr Ser Arg Ser Asp Val Gly Tyr Gly Val Tyr Asp Leu Tyr Asp
    50                  55                  60

Leu Gly Glu Phe Asn Gln Lys Gly Thr Val Arg Thr Lys Tyr Gly Thr
65                  70                  75                  80

Lys Ala Gln Tyr Leu Gln Ala Ile Gln Ala Ala His Ala Ala Gly Met
                85                  90                  95

Gln Val Tyr Ala Asp Val Val Phe Asp His Lys Gly Gly Ala Asp Gly
            100                 105                 110

Thr Glu Trp Val Asp Ala Val Glu Val Asn Pro Ser Asp Arg Asn Gln
        115                 120                 125
```

```
Glu Ile Ser Gly Thr Tyr Gln Ile Gln Ala Trp Thr Lys Phe Asp Phe
    130                 135                 140

Pro Gly Arg Gly Asn Thr Tyr Ser Ser Phe Lys Trp Arg Trp Tyr His
145                 150                 155                 160

Phe Asp Gly Val Asp Trp Asp Glu Ser Arg Lys Leu Ser Arg Ile Tyr
                165                 170                 175

Lys Phe Arg Gly Ile Gly Lys Ala Trp Asp Trp Glu Val Asp Thr Glu
            180                 185                 190

Asn Gly Asn Tyr Asp Tyr Leu Met Tyr Ala Asp Leu Asp Met Asp His
        195                 200                 205

Pro Glu Val Val Thr Glu Leu Lys Asn Trp Gly Lys Trp Tyr Val Asn
    210                 215                 220

Thr Thr Asn Ile Asp Gly Phe Arg Leu Asp Ala Val Lys His Ile Lys
225                 230                 235                 240

Phe Ser Phe Phe Pro Asp Trp Leu Ser Tyr Val Arg Ser Gln Thr Gly
                245                 250                 255

Lys Pro Leu Phe Thr Val Gly Glu Tyr Trp Ser Tyr Asp Ile Asn Lys
            260                 265                 270

Leu His Asn Tyr Ile Thr Lys Thr Asn Gly Thr Met Ser Leu Phe Asp
        275                 280                 285

Ala Pro Leu His Asn Lys Phe Tyr Thr Ala Ser Lys Ser Gly Gly Ala
    290                 295                 300

Phe Asp Met Arg Thr Leu Met Thr Asn Thr Leu Met Lys Asp Gln Pro
305                 310                 315                 320

Thr Leu Ala Val Thr Phe Val Asp Asn His Asp Thr Glu Pro Gly Gln
                325                 330                 335

Ala Leu Gln Ser Trp Val Asp Pro Trp Phe Lys Pro Leu Ala Tyr Ala
            340                 345                 350

Phe Ile Leu Thr Arg Gln Glu Gly Tyr Pro Cys Val Phe Tyr Gly Asp
        355                 360                 365

Tyr Tyr Gly Ile Pro Gln Tyr Asn Ile Pro Ser Leu Lys Ser Lys Ile
    370                 375                 380
```

```
Asp Pro Leu Leu Ile Ala Arg Arg Asp Tyr Ala Tyr Gly Thr Gln His
385                 390                 395                 400

Asp Tyr Leu Asp His Ser Asp Ile Ile Gly Trp Thr Arg Glu Gly Val
                405                 410                 415

Thr Glu Lys Pro Gly Ser Gly Leu Ala Ala Leu Ile Thr Asp Gly Pro
            420                 425                 430

Gly Gly Ser Lys Trp Met Tyr Val Gly Lys Gln His Ala Gly Lys Val
        435                 440                 445

Phe Tyr Asp Leu Thr Gly Asn Arg Ser Asp Thr Val Thr Ile Asn Ser
    450                 455                 460

Asp Gly Trp Gly Glu Phe Lys Val Asn Gly Gly Ser Val Ser Val Trp
465                 470                 475                 480

Val Pro Arg Lys Thr Thr Val Ser Thr Ile Ala Arg Pro Ile Thr Thr
                485                 490                 495

Arg Pro Trp Thr Gly Glu Phe Val Arg Trp Thr Glu Pro Arg Leu Val
            500                 505                 510

Ala Trp Pro
        515
```

<210> 2
<211> 1068
<212> DNA
<213> Thermoascus aurantiacus

<220>
<221> CDS
<222> (1)..(1065)

<220>
<221> misc_signal
<222> (1)..(57)

<220>
<221> misc_feature
<222> (58)..(534)

<220>
<221> mat_peptide
<222> (535)..(1068)

<400> 2

```
atg cgg ctc gtt  gct tcc cta acg gcc  ttg gtg gcc ttg tcc  gta          45
Met Arg Leu Val  Ala Ser Leu Thr Ala  Leu Val Ala Leu Ser  Val
```

```
             -175                -170                -165

cct gtc ttt ccc  gct gct gtc aac gtg  aag cgt gct tcg tcc  tac          90
Pro Val Phe Pro  Ala Ala Val Asn Val  Lys Arg Ala Ser Ser  Tyr
             -160                -155                -150

ctg gag atc act  ctg agc cag gtc agc  aac act ctg atc aag  gcc         135
Leu Glu Ile Thr  Leu Ser Gln Val Ser  Asn Thr Leu Ile Lys  Ala
             -145                -140                -135

gtg gtc cag aac  act ggt agc gac gag  ttg tcc ttc gtt cac  ctg         180
Val Val Gln Asn  Thr Gly Ser Asp Glu  Leu Ser Phe Val His  Leu
             -130                -125                -120

aac ttc ttc aag  gac ccc gct cct gtc  aaa aag gta tcg gtc  tat         225
Asn Phe Phe Lys  Asp Pro Ala Pro Val  Lys Lys Val Ser Val  Tyr
             -115                -110                -105

cgc gat ggg tct  gaa gtg cag ttc gag ggc att ttg agc cgc tac aaa       273
Arg Asp Gly Ser  Glu Val Gln Phe Glu Gly Ile Leu Ser Arg Tyr Lys
             -100                -95                -90

tcg act ggc ctc tct cgt gac gcc ttt act tat ctg gct ccc gga gag        321
Ser Thr Gly Leu Ser Arg Asp Ala Phe Thr Tyr Leu Ala Pro Gly Glu
        -85                 -80                 -75

tcc gtc gag gac gtt ttt gat att gct tcg act tac gat ctg acc agc        369
Ser Val Glu Asp Val Phe Asp Ile Ala Ser Thr Tyr Asp Leu Thr Ser
    -70                 -65                 -60

ggc ggc cct gta act atc cgt act gag gga gtt gtt ccc tac gcc acg        417
Gly Gly Pro Val Thr Ile Arg Thr Glu Gly Val Val Pro Tyr Ala Thr
-55                 -50                 -45                 -40

gct aac agc act gat att gcc ggc tac atc tca tac tcg tct aat gtg        465
Ala Asn Ser Thr Asp Ile Ala Gly Tyr Ile Ser Tyr Ser Ser Asn Val
                -35                 -30                 -25

ttg acc att gat gtc gat ggc gcc gct gct gcc act gtc tcc aag gca        513
Leu Thr Ile Asp Val Asp Gly Ala Ala Ala Ala Thr Val Ser Lys Ala
            -20                 -15                 -10

atc act cct ttg gac cgc cgc act agg atc agt tcc tgc tcc ggc agc        561
Ile Thr Pro Leu Asp Arg Arg Thr Arg Ile Ser Ser Cys Ser Gly Ser
        -5                  -1  1               5

aga cag agc gct ctt act acg gct ctc aga aac gct gct tct ctt gcc        609
Arg Gln Ser Ala Leu Thr Thr Ala Leu Arg Asn Ala Ala Ser Leu Ala
10                  15                  20                  25

aac gca gct gcc gac gcg gct cag tct gga tca gct tca aag ttc agc        657
Asn Ala Ala Ala Asp Ala Ala Gln Ser Gly Ser Ala Ser Lys Phe Ser
                30                  35                  40

gag tac ttc aag act act tct agc tct acc cgc cag acc gtg gct gcg        705
Glu Tyr Phe Lys Thr Thr Ser Ser Ser Thr Arg Gln Thr Val Ala Ala
            45                  50                  55

cgt ctt cgg gct gtt gcg cgg gag gca tct tcg tct tct tcg gga gcc        753
Arg Leu Arg Ala Val Ala Arg Glu Ala Ser Ser Ser Ser Ser Gly Ala
        60                  65                  70

acc acg tac tac tgc gac gat ccc tac ggc tac tgt tcc tcc aac gtc        801
```

```
Thr Thr Tyr Tyr Cys Asp Asp Pro Tyr Gly Tyr Cys Ser Ser Asn Val
    75                  80                  85

ctg gct tac acc ctg cct tca tac aac ata atc gcc aac tgt gac att        849
Leu Ala Tyr Thr Leu Pro Ser Tyr Asn Ile Ile Ala Asn Cys Asp Ile
90                  95                  100                 105

ttc tat act tac ctg ccg gct ctg acc agt acc tgt cac gct cag gat        897
Phe Tyr Thr Tyr Leu Pro Ala Leu Thr Ser Thr Cys His Ala Gln Asp
                110                 115                 120

caa gcg acc act gcc ctt cac gag ttc acc cat gcg cct ggc gtc tac        945
Gln Ala Thr Thr Ala Leu His Glu Phe Thr His Ala Pro Gly Val Tyr
            125                 130                 135

agc cct ggc acg gac gac ctg gcg tat ggc tac cag gct gcg atg ggt        993
Ser Pro Gly Thr Asp Asp Leu Ala Tyr Gly Tyr Gln Ala Ala Met Gly
        140                 145                 150

ctc agc agc agc cag gct gtc atg aac gct gac acc tac gct ctc tat       1041
Leu Ser Ser Ser Gln Ala Val Met Asn Ala Asp Thr Tyr Ala Leu Tyr
    155                 160                 165

gcg aat gcc ata tac ctt ggt tgc taa                                   1068
Ala Asn Ala Ile Tyr Leu Gly Cys
170                 175
```

<210> 3
<211> 355
<212> PRT
<213> Thermoascus aurantiacus

<400> 3

```
Met Arg Leu Val  Ala Ser Leu Thr Ala  Leu Val Ala Leu Ser  Val
            -175                 -170                 -165

Pro Val Phe Pro  Ala Ala Val Asn Val  Lys Arg Ala Ser Ser  Tyr
            -160                 -155                 -150

Leu Glu Ile Thr  Leu Ser Gln Val Ser  Asn Thr Leu Ile Lys  Ala
            -145                 -140                 -135

Val Val Gln Asn  Thr Gly Ser Asp Glu  Leu Ser Phe Val His  Leu
            -130                 -125                 -120

Asn Phe Phe Lys  Asp Pro Ala Pro Val  Lys Lys Val Ser Val  Tyr
            -115                 -110                 -105

Arg Asp Gly Ser  Glu Val Gln Phe Glu Gly Ile Leu Ser Arg Tyr Lys
            -100                 -95                 -90

Ser Thr Gly Leu Ser Arg Asp Ala Phe Thr Tyr Leu Ala Pro Gly Glu
        -85                 -80                 -75
```

```
Ser Val Glu Asp Val Phe Asp Ile Ala Ser Thr Tyr Asp Leu Thr Ser
    -70                 -65                 -60

Gly Gly Pro Val Thr Ile Arg Thr Glu Gly Val Val Pro Tyr Ala Thr
-55                 -50                 -45                 -40

Ala Asn Ser Thr Asp Ile Ala Gly Tyr Ile Ser Tyr Ser Ser Asn Val
                -35                 -30                 -25

Leu Thr Ile Asp Val Asp Gly Ala Ala Ala Ala Thr Val Ser Lys Ala
            -20                 -15                 -10

Ile Thr Pro Leu Asp Arg Arg Thr Arg Ile Ser Ser Cys Ser Gly Ser
        -5                  -1  1               5

Arg Gln Ser Ala Leu Thr Thr Ala Leu Arg Asn Ala Ala Ser Leu Ala
10                  15                  20                  25

Asn Ala Ala Ala Asp Ala Ala Gln Ser Gly Ser Ala Ser Lys Phe Ser
                30                  35                  40

Glu Tyr Phe Lys Thr Thr Ser Ser Ser Thr Arg Gln Thr Val Ala Ala
            45                  50                  55

Arg Leu Arg Ala Val Ala Arg Glu Ala Ser Ser Ser Ser Ser Gly Ala
        60                  65                  70

Thr Thr Tyr Tyr Cys Asp Asp Pro Tyr Gly Tyr Cys Ser Ser Asn Val
    75                  80                  85

Leu Ala Tyr Thr Leu Pro Ser Tyr Asn Ile Ile Ala Asn Cys Asp Ile
90                  95                  100                 105

Phe Tyr Thr Tyr Leu Pro Ala Leu Thr Ser Thr Cys His Ala Gln Asp
                110                 115                 120

Gln Ala Thr Thr Ala Leu His Glu Phe Thr His Ala Pro Gly Val Tyr
            125                 130                 135

Ser Pro Gly Thr Asp Asp Leu Ala Tyr Gly Tyr Gln Ala Ala Met Gly
        140                 145                 150

Leu Ser Ser Ser Gln Ala Val Met Asn Ala Asp Thr Tyr Ala Leu Tyr
    155                 160                 165

Ala Asn Ala Ile Tyr Leu Gly Cys
170                 175
```

<210> 4
<211> 49
<212> DNA
<213> Artificial Sequence

<220>
<223> Synthetic Construct

<400> 4
aacgacggta cccggggatc ggatccatgc ggctcgttgc ttccctaac 49

<210> 5
<211> 48
<212> DNA
<213> Artificial Sequence

<220>
<223> Artificial Construct

<400> 5
ctaattacat gatgcggccc ttaattaatt agcaaccaag gtatatgg 48

<210> 6
<211> 20
<212> DNA
<213> Artificial Sequence

<220>
<223> Artificial Construct

<400> 6
taggagttta gtgaacttgc 20

<210> 7
<211> 18
<212> DNA
<213> Artificial Sequence

<220>
<223> Artificial Construct

<400> 7
ttcgagcgtc ccaaaacc 18

<210> 8
<211> 1851
<212> DNA
<213> Penicillium oxalicum

<220>
<221> CDS
<222> (1)..(1851)

<400> 8

```
atg cgt ctc act cta tta tca ggt gta gcc ggc gtt ctc tgc gca gga         48
Met Arg Leu Thr Leu Leu Ser Gly Val Ala Gly Val Leu Cys Ala Gly
1               5                   10                  15
```

```
cag ctg acg gcg gcg cgt cct gat ccc aag ggt ggg aat ctg acg ccg         96
Gln Leu Thr Ala Ala Arg Pro Asp Pro Lys Gly Gly Asn Leu Thr Pro
            20                  25                  30

ttc atc cac aaa gag ggc gag cgg tcg ctc caa ggc atc ttg gac aat        144
Phe Ile His Lys Glu Gly Glu Arg Ser Leu Gln Gly Ile Leu Asp Asn
        35                  40                  45

ctc ggt ggg cga ggt aag aaa aca ccc ggc act gcc gca ggg ttg ttt        192
Leu Gly Gly Arg Gly Lys Lys Thr Pro Gly Thr Ala Ala Gly Leu Phe
    50                  55                  60

att gcc agt cca aac aca gag aat cca aac tat tat tat aca tgg act        240
Ile Ala Ser Pro Asn Thr Glu Asn Pro Asn Tyr Tyr Tyr Thr Trp Thr
65                  70                  75                  80

cgt gac tca gct ttg act gcc aag tgc ttg atc gac ctg ttc gaa gac        288
Arg Asp Ser Ala Leu Thr Ala Lys Cys Leu Ile Asp Leu Phe Glu Asp
                85                  90                  95

tct cgg gca aag ttt cca att gac cgc aaa tac ttg gaa aca gga att        336
Ser Arg Ala Lys Phe Pro Ile Asp Arg Lys Tyr Leu Glu Thr Gly Ile
            100                 105                 110

cgg gac tac gtg tcg tcc caa gca atc ctc cag agt gtg tct aat cct        384
Arg Asp Tyr Val Ser Ser Gln Ala Ile Leu Gln Ser Val Ser Asn Pro
        115                 120                 125

tct gga acc ctg aag gat ggc tct ggt ctg ggt gaa ccc aag ttt gag        432
Ser Gly Thr Leu Lys Asp Gly Ser Gly Leu Gly Glu Pro Lys Phe Glu
    130                 135                 140

att gac ctg aat ccc ttt tcg ggt gcc tgg ggt cgg cct cag cgg gat        480
Ile Asp Leu Asn Pro Phe Ser Gly Ala Trp Gly Arg Pro Gln Arg Asp
145                 150                 155                 160

ggc cca gcg ctg cga gcg acc gct atg atc acc tac gcc aac tac ctg        528
Gly Pro Ala Leu Arg Ala Thr Ala Met Ile Thr Tyr Ala Asn Tyr Leu
                165                 170                 175

ata tcc cat ggt cag aaa tcg gat gtg tca cag gtc atg tgg ccg att        576
Ile Ser His Gly Gln Lys Ser Asp Val Ser Gln Val Met Trp Pro Ile
            180                 185                 190

att gcc aat gat cta gca tat gtt ggt caa tac tgg aat aat acc gga        624
Ile Ala Asn Asp Leu Ala Tyr Val Gly Gln Tyr Trp Asn Asn Thr Gly
        195                 200                 205

ttt gac ctg tgg gaa gag gtg gat ggg tca agc ttt ttc acg att gcg        672
Phe Asp Leu Trp Glu Glu Val Asp Gly Ser Ser Phe Phe Thr Ile Ala
    210                 215                 220

gtc cag cac cga gcc ctt gtt gaa ggc tcg caa ctg gcg aaa aag ctc        720
Val Gln His Arg Ala Leu Val Glu Gly Ser Gln Leu Ala Lys Lys Leu
225                 230                 235                 240

ggc aag tcc tgc gat gcc tgt gat tct cag cct ccc cag ata ttg tgt        768
Gly Lys Ser Cys Asp Ala Cys Asp Ser Gln Pro Pro Gln Ile Leu Cys
                245                 250                 255

ttc ctg cag agt ttc tgg aac gga aag tac atc acc tcc aac atc aac        816
Phe Leu Gln Ser Phe Trp Asn Gly Lys Tyr Ile Thr Ser Asn Ile Asn
```

```
            260                 265                 270

acg caa gca agc cgc tct ggt atc gac ctg gac tct gtc ctg gga agc      864
Thr Gln Ala Ser Arg Ser Gly Ile Asp Leu Asp Ser Val Leu Gly Ser
        275                 280                 285

att cat acc ttt gat ccc gaa gca gcc tgt gac gat gca act ttc cag      912
Ile His Thr Phe Asp Pro Glu Ala Ala Cys Asp Asp Ala Thr Phe Gln
    290                 295                 300

cct tgt tct gcc cgc gct ctg gcg aac cac aag gtc tat gtg gat tcc      960
Pro Cys Ser Ala Arg Ala Leu Ala Asn His Lys Val Tyr Val Asp Ser
305                 310                 315                 320

ttc cgc tct atc tac aag att aat gcg ggt ctt gca gag gga tcg gct     1008
Phe Arg Ser Ile Tyr Lys Ile Asn Ala Gly Leu Ala Glu Gly Ser Ala
                325                 330                 335

gcc aac gtt ggc cgc tac ccc gag gat gtt tac caa gga ggc aat cca     1056
Ala Asn Val Gly Arg Tyr Pro Glu Asp Val Tyr Gln Gly Gly Asn Pro
            340                 345                 350

tgg tat ctc gcc acc cta ggc gca tct gaa ttg ctt tac gac gcc ttg     1104
Trp Tyr Leu Ala Thr Leu Gly Ala Ser Glu Leu Leu Tyr Asp Ala Leu
        355                 360                 365

tac cag tgg gac aga ctt ggc aaa ctt gaa gtc tcg gag acc tcg ttg     1152
Tyr Gln Trp Asp Arg Leu Gly Lys Leu Glu Val Ser Glu Thr Ser Leu
    370                 375                 380

tca ttc ttc aaa gac ttt gac gcg acc gtg aaa att ggc tcg tac tcg     1200
Ser Phe Phe Lys Asp Phe Asp Ala Thr Val Lys Ile Gly Ser Tyr Ser
385                 390                 395                 400

agg aac agc aag acc tac aag aaa ttg acc cag tcc atc aag tcg tac     1248
Arg Asn Ser Lys Thr Tyr Lys Lys Leu Thr Gln Ser Ile Lys Ser Tyr
                405                 410                 415

gcg gac ggg ttc atc cag tta gtg cag cag tac act cct tct aat gga     1296
Ala Asp Gly Phe Ile Gln Leu Val Gln Gln Tyr Thr Pro Ser Asn Gly
            420                 425                 430

tct ctg gcc gag caa tac gat cgc aat acg gct gct cct ctc tct gca     1344
Ser Leu Ala Glu Gln Tyr Asp Arg Asn Thr Ala Ala Pro Leu Ser Ala
        435                 440                 445

aac gat ctg act tgg tca ttt gcc tct ttc ttg acg gct acg caa cgc     1392
Asn Asp Leu Thr Trp Ser Phe Ala Ser Phe Leu Thr Ala Thr Gln Arg
    450                 455                 460

cgc gat gcc gtg gtt cct ccc tcc tgg ggc gca aag tcg gca aac aaa     1440
Arg Asp Ala Val Val Pro Pro Ser Trp Gly Ala Lys Ser Ala Asn Lys
465                 470                 475                 480

gtc cca acc act tgt tca gcc tcc cct gtt gtg ggt act tat aag gcg     1488
Val Pro Thr Thr Cys Ser Ala Ser Pro Val Val Gly Thr Tyr Lys Ala
                485                 490                 495

ccc acg gca act ttc tca tcc aag act aag tgc gtc ccc gct aaa gat     1536
Pro Thr Ala Thr Phe Ser Ser Lys Thr Lys Cys Val Pro Ala Lys Asp
            500                 505                 510

att gtg cct atc acg ttc tac ctg att gag aac act tac tat gga gag     1584
```

```
Ile Val Pro Ile Thr Phe Tyr Leu Ile Glu Asn Thr Tyr Tyr Gly Glu
        515                 520                 525

aac gtc ttc atg agt ggc aac att act gcg ctg ggt aac tgg gac gcc      1632
Asn Val Phe Met Ser Gly Asn Ile Thr Ala Leu Gly Asn Trp Asp Ala
    530                 535                 540

aag aaa ggc ttc cca ctc acc gca aac ctc tac acg caa gat caa aac      1680
Lys Lys Gly Phe Pro Leu Thr Ala Asn Leu Tyr Thr Gln Asp Gln Asn
545                 550                 555                 560

ttg tgg ttc gcc agt gtc gag ttc atc cca gca ggc aca ccc ttt gag      1728
Leu Trp Phe Ala Ser Val Glu Phe Ile Pro Ala Gly Thr Pro Phe Glu
                565                 570                 575

tac aag tac tac aag gtc gag ccc aat ggc gat att act tgg gag aag      1776
Tyr Lys Tyr Tyr Lys Val Glu Pro Asn Gly Asp Ile Thr Trp Glu Lys
            580                 585                 590

ggt ccc aac cgg gtg ttc gtc gct ccc acg gga tgc cca gtt cag cct      1824
Gly Pro Asn Arg Val Phe Val Ala Pro Thr Gly Cys Pro Val Gln Pro
        595                 600                 605

cac tcc aac gac gtg tgg cag ttt tga                                  1851
His Ser Asn Asp Val Trp Gln Phe
    610                 615
```

<210> 9
<211> 616
<212> PRT
<213> Penicillium oxalicum

<400> 9

```
Met Arg Leu Thr Leu Leu Ser Gly Val Ala Gly Val Leu Cys Ala Gly
1               5                   10                  15

Gln Leu Thr Ala Ala Arg Pro Asp Pro Lys Gly Gly Asn Leu Thr Pro
            20                  25                  30

Phe Ile His Lys Glu Gly Glu Arg Ser Leu Gln Gly Ile Leu Asp Asn
        35                  40                  45

Leu Gly Gly Arg Gly Lys Lys Thr Pro Gly Thr Ala Ala Gly Leu Phe
    50                  55                  60

Ile Ala Ser Pro Asn Thr Glu Asn Pro Asn Tyr Tyr Tyr Thr Trp Thr
65                  70                  75                  80

Arg Asp Ser Ala Leu Thr Ala Lys Cys Leu Ile Asp Leu Phe Glu Asp
                85                  90                  95

Ser Arg Ala Lys Phe Pro Ile Asp Arg Lys Tyr Leu Glu Thr Gly Ile
            100                 105                 110
```

Arg Asp Tyr Val Ser Ser Gln Ala Ile Leu Gln Ser Val Ser Asn Pro
        115                 120                 125

Ser Gly Thr Leu Lys Asp Gly Ser Gly Leu Gly Glu Pro Lys Phe Glu
    130                 135                 140

Ile Asp Leu Asn Pro Phe Ser Gly Ala Trp Gly Arg Pro Gln Arg Asp
145                 150                 155                 160

Gly Pro Ala Leu Arg Ala Thr Ala Met Ile Thr Tyr Ala Asn Tyr Leu
                165                 170                 175

Ile Ser His Gly Gln Lys Ser Asp Val Ser Gln Val Met Trp Pro Ile
            180                 185                 190

Ile Ala Asn Asp Leu Ala Tyr Val Gly Gln Tyr Trp Asn Asn Thr Gly
        195                 200                 205

Phe Asp Leu Trp Glu Glu Val Asp Gly Ser Ser Phe Phe Thr Ile Ala
    210                 215                 220

Val Gln His Arg Ala Leu Val Glu Gly Ser Gln Leu Ala Lys Lys Leu
225                 230                 235                 240

Gly Lys Ser Cys Asp Ala Cys Asp Ser Gln Pro Pro Gln Ile Leu Cys
                245                 250                 255

Phe Leu Gln Ser Phe Trp Asn Gly Lys Tyr Ile Thr Ser Asn Ile Asn
            260                 265                 270

Thr Gln Ala Ser Arg Ser Gly Ile Asp Leu Asp Ser Val Leu Gly Ser
        275                 280                 285

Ile His Thr Phe Asp Pro Glu Ala Ala Cys Asp Asp Ala Thr Phe Gln
    290                 295                 300

Pro Cys Ser Ala Arg Ala Leu Ala Asn His Lys Val Tyr Val Asp Ser
305                 310                 315                 320

Phe Arg Ser Ile Tyr Lys Ile Asn Ala Gly Leu Ala Glu Gly Ser Ala
                325                 330                 335

Ala Asn Val Gly Arg Tyr Pro Glu Asp Val Tyr Gln Gly Gly Asn Pro
            340                 345                 350

Trp Tyr Leu Ala Thr Leu Gly Ala Ser Glu Leu Leu Tyr Asp Ala Leu
        355                 360                 365

Tyr Gln Trp Asp Arg Leu Gly Lys Leu Glu Val Ser Glu Thr Ser Leu
370 375 380

Ser Phe Phe Lys Asp Phe Asp Ala Thr Val Lys Ile Gly Ser Tyr Ser
385 390 395 400

Arg Asn Ser Lys Thr Tyr Lys Lys Leu Thr Gln Ser Ile Lys Ser Tyr
405 410 415

Ala Asp Gly Phe Ile Gln Leu Val Gln Gln Tyr Thr Pro Ser Asn Gly
420 425 430

Ser Leu Ala Glu Gln Tyr Asp Arg Asn Thr Ala Ala Pro Leu Ser Ala
435 440 445

Asn Asp Leu Thr Trp Ser Phe Ala Ser Phe Leu Thr Ala Thr Gln Arg
450 455 460

Arg Asp Ala Val Val Pro Pro Ser Trp Gly Ala Lys Ser Ala Asn Lys
465 470 475 480

Val Pro Thr Thr Cys Ser Ala Ser Pro Val Val Gly Thr Tyr Lys Ala
485 490 495

Pro Thr Ala Thr Phe Ser Ser Lys Thr Lys Cys Val Pro Ala Lys Asp
500 505 510

Ile Val Pro Ile Thr Phe Tyr Leu Ile Glu Asn Thr Tyr Tyr Gly Glu
515 520 525

Asn Val Phe Met Ser Gly Asn Ile Thr Ala Leu Gly Asn Trp Asp Ala
530 535 540

Lys Lys Gly Phe Pro Leu Thr Ala Asn Leu Tyr Thr Gln Asp Gln Asn
545 550 555 560

Leu Trp Phe Ala Ser Val Glu Phe Ile Pro Ala Gly Thr Pro Phe Glu
565 570 575

Tyr Lys Tyr Tyr Lys Val Glu Pro Asn Gly Asp Ile Thr Trp Glu Lys
580 585 590

Gly Pro Asn Arg Val Phe Val Ala Pro Thr Gly Cys Pro Val Gln Pro
595 600 605

His Ser Asn Asp Val Trp Gln Phe
610 615

<210> 10
<211> 4014
<212> DNA
<213> Thermococcus hydrothermalis

<220>
<221> CDS
<222> (1)..(4011)

<220>
<221> misc_signal
<222> (1)..(81)

<220>
<221> mat_peptide
<222> (82)..(4014)

<400> 10

```
atg agg cgg gtg gtt gcc ctc ttc att gca att ttg atg ctt gga agc          48
Met Arg Arg Val Val Ala Leu Phe Ile Ala Ile Leu Met Leu Gly Ser
        -25                 -20                 -15

atc gtt gga gcg aac gtt aag agc gtt ggc gcg gcg gag ccg aag ccg          96
Ile Val Gly Ala Asn Val Lys Ser Val Gly Ala Ala Glu Pro Lys Pro
    -10                 -5                  -1  1               5

ctc aac gtc ata ata gtc tgg cac cag cac cag ccc tac tac tac gac         144
Leu Asn Val Ile Ile Val Trp His Gln His Gln Pro Tyr Tyr Tyr Asp
                10                  15                  20

cct gtc cag gac gtc tac acc agg ccc tgg gtc agg ctc cac gcg gcg         192
Pro Val Gln Asp Val Tyr Thr Arg Pro Trp Val Arg Leu His Ala Ala
            25                  30                  35

aac aac tac tgg aag atg gcc cac tac ctg agc cag tac ccg gag gtt         240
Asn Asn Tyr Trp Lys Met Ala His Tyr Leu Ser Gln Tyr Pro Glu Val
        40                  45                  50

cac gcc acc att gac ctc tcg ggt tcg ctg ata gcc cag ctt gcc gac         288
His Ala Thr Ile Asp Leu Ser Gly Ser Leu Ile Ala Gln Leu Ala Asp
    55                  60                  65

tac atg aac ggc aag aag gac acc tac cag ata atc acc gag aag ata         336
Tyr Met Asn Gly Lys Lys Asp Thr Tyr Gln Ile Ile Thr Glu Lys Ile
70                  75                  80                  85

gcc aac ggg gaa ccc ctc acc gtc gac gag aag tgg ttc atg ctc cag         384
Ala Asn Gly Glu Pro Leu Thr Val Asp Glu Lys Trp Phe Met Leu Gln
                90                  95                  100

gca ccg gga ggg ttc ttc gac aac acc atc ccc tgg aac ggt gaa ccg         432
Ala Pro Gly Gly Phe Phe Asp Asn Thr Ile Pro Trp Asn Gly Glu Pro
            105                 110                 115

ata acc gac ccc aac ggc aac ccg ata agg gac ttc tgg gac cgc tac         480
Ile Thr Asp Pro Asn Gly Asn Pro Ile Arg Asp Phe Trp Asp Arg Tyr
        120                 125                 130

acg gag ctg aag aac aag atg ctc agc gca aag gcc aag tac gca aac         528
Thr Glu Leu Lys Asn Lys Met Leu Ser Ala Lys Ala Lys Tyr Ala Asn
```

```
    135                 140                 145

ttc gtg act gag agc cag aag gtc gct gtg acg aac gag ttc aca gag          576
Phe Val Thr Glu Ser Gln Lys Val Ala Val Thr Asn Glu Phe Thr Glu
150                 155                 160                 165

cag gac tac ata gac cta gcg gtt ctc ttc aat ctc gct tgg att gac          624
Gln Asp Tyr Ile Asp Leu Ala Val Leu Phe Asn Leu Ala Trp Ile Asp
                170                 175                 180

tac aat tac atc acg agc acg ccg gag ttc aag gcc ctc tac gac aag          672
Tyr Asn Tyr Ile Thr Ser Thr Pro Glu Phe Lys Ala Leu Tyr Asp Lys
            185                 190                 195

gtt gac gag ggc ggc tat aca agg gcg gac gtc aaa acc gtt ctc gac          720
Val Asp Glu Gly Gly Tyr Thr Arg Ala Asp Val Lys Thr Val Leu Asp
        200                 205                 210

gcc cag atc tgg ctt ctc aac cac acc ttc gag gag cac gag aag ata          768
Ala Gln Ile Trp Leu Leu Asn His Thr Phe Glu Glu His Glu Lys Ile
    215                 220                 225

aac ctc ctc ctc gga aac ggc aac gtc gag gtc acg gtc gtt ccc tac          816
Asn Leu Leu Leu Gly Asn Gly Asn Val Glu Val Thr Val Val Pro Tyr
230                 235                 240                 245

gcc cac ccg ata ggc ccg ata ctc aac gac ttc ggc tgg gac agc gac          864
Ala His Pro Ile Gly Pro Ile Leu Asn Asp Phe Gly Trp Asp Ser Asp
                250                 255                 260

ttc aac gac cag gtc aag aag gcc gac gaa ctg tac aag ccg tac ctc          912
Phe Asn Asp Gln Val Lys Lys Ala Asp Glu Leu Tyr Lys Pro Tyr Leu
            265                 270                 275

ggc ggc ggc acc gcg gtt cca aaa ggc gga tgg gcg gct gag agc gcc          960
Gly Gly Gly Thr Ala Val Pro Lys Gly Gly Trp Ala Ala Glu Ser Ala
        280                 285                 290

ctc aac gac aaa act ctg gag atc ctc gcc gag aac ggc tgg gag tgg         1008
Leu Asn Asp Lys Thr Leu Glu Ile Leu Ala Glu Asn Gly Trp Glu Trp
    295                 300                 305

gtc atg acc gac cag atg gtt ctc gga aag ctc ggc att gag gga acc         1056
Val Met Thr Asp Gln Met Val Leu Gly Lys Leu Gly Ile Glu Gly Thr
310                 315                 320                 325

gtc gag aac tac cac aag ccc tgg gtg gcc gag ttc aac gga aag aag         1104
Val Glu Asn Tyr His Lys Pro Trp Val Ala Glu Phe Asn Gly Lys Lys
                330                 335                 340

ata tac ctc ttc cca aga aat cac gat cta agt gac aga gtt ggc ttt         1152
Ile Tyr Leu Phe Pro Arg Asn His Asp Leu Ser Asp Arg Val Gly Phe
            345                 350                 355

acc tac agc gga atg aac cag cag cag gcc gtt gag gac ttc gtc aac         1200
Thr Tyr Ser Gly Met Asn Gln Gln Gln Ala Val Glu Asp Phe Val Asn
        360                 365                 370

gag ctc ctc aag ctc cag aag cag aac tac gat ggc tcg ctg gtt tac         1248
Glu Leu Leu Lys Leu Gln Lys Gln Asn Tyr Asp Gly Ser Leu Val Tyr
    375                 380                 385

gtg gtc acg ctc gac ggc gag aac ccc gtg gag aac tac ccc tac gac         1296
```

```
Val Val Thr Leu Asp Gly Glu Asn Pro Val Glu Asn Tyr Pro Tyr Asp
390                 395                 400                 405

ggg gag ctc ttc ctc acc gaa ctc tac aag aag ctg acc gaa ctc cag        1344
Gly Glu Leu Phe Leu Thr Glu Leu Tyr Lys Lys Leu Thr Glu Leu Gln
                410                 415                 420

gag cag ggt ctc ata aga acc ctc acc ccg agc gag tac atc cag ctc        1392
Glu Gln Gly Leu Ile Arg Thr Leu Thr Pro Ser Glu Tyr Ile Gln Leu
            425                 430                 435

tac ggc gac aag gcc aac aag ctc aca cct cgg atg atg gag cgc ctt        1440
Tyr Gly Asp Lys Ala Asn Lys Leu Thr Pro Arg Met Met Glu Arg Leu
        440                 445                 450

gac ctc acc gga gac aac gtt aac gcc ctc ctc aag gcc cag agc ctc        1488
Asp Leu Thr Gly Asp Asn Val Asn Ala Leu Leu Lys Ala Gln Ser Leu
    455                 460                 465

ggc gaa ctc tac gac atg acc ggc gtt aag gag gag atg cag tgg ccc        1536
Gly Glu Leu Tyr Asp Met Thr Gly Val Lys Glu Glu Met Gln Trp Pro
470                 475                 480                 485

gag agc agc tgg ata gac gga acc ctc tcc acg tgg ata ggc gag ccc        1584
Glu Ser Ser Trp Ile Asp Gly Thr Leu Ser Thr Trp Ile Gly Glu Pro
                490                 495                 500

cag gag aac tac ggc tgg tac tgg ctc tac atg gcc agg aag gcc ctt        1632
Gln Glu Asn Tyr Gly Trp Tyr Trp Leu Tyr Met Ala Arg Lys Ala Leu
            505                 510                 515

atg gag aac aag gat aaa atg agc cag gcg gac tgg gag aag gcc tac        1680
Met Glu Asn Lys Asp Lys Met Ser Gln Ala Asp Trp Glu Lys Ala Tyr
        520                 525                 530

gag tac ctg ctc cgc gcc gag gca agc gac tgg ttc tgg tgg tac gga        1728
Glu Tyr Leu Leu Arg Ala Glu Ala Ser Asp Trp Phe Trp Trp Tyr Gly
    535                 540                 545

agc gac cag gac agc ggc cag gac tac acc ttc gac cgc tac ctg aag        1776
Ser Asp Gln Asp Ser Gly Gln Asp Tyr Thr Phe Asp Arg Tyr Leu Lys
550                 555                 560                 565

acc tac ctc tac gag atg tac aag ctg gca gga gtc gag ccg ccg agc        1824
Thr Tyr Leu Tyr Glu Met Tyr Lys Leu Ala Gly Val Glu Pro Pro Ser
                570                 575                 580

tac ctc ttc ggc aac tac ttc ccg gac gga gag ccc tac acc acg agg        1872
Tyr Leu Phe Gly Asn Tyr Phe Pro Asp Gly Glu Pro Tyr Thr Thr Arg
            585                 590                 595

ggc ctg gtc gga ctc aag gac ggc gag atg aag aac ttc tcc agc atg        1920
Gly Leu Val Gly Leu Lys Asp Gly Glu Met Lys Asn Phe Ser Ser Met
        600                 605                 610

tcc ccg ctg gca aag ggc gtg agc gtc tat ttc gac ggc gag ggg ata        1968
Ser Pro Leu Ala Lys Gly Val Ser Val Tyr Phe Asp Gly Glu Gly Ile
    615                 620                 625

cac ttc ata gtg aaa ggg aac ctg gac agg ttc gag gtg agc atc tgg        2016
His Phe Ile Val Lys Gly Asn Leu Asp Arg Phe Glu Val Ser Ile Trp
630                 635                 640                 645
```

gag aag gat gag cgc gtt ggc aac acg ttc acc cgc ctc caa gag aag 2064
Glu Lys Asp Glu Arg Val Gly Asn Thr Phe Thr Arg Leu Gln Glu Lys
650 655 660

ccg gac gag ttg agc tat ttc atg ttc cca ttc tca agg gac agc gtt 2112
Pro Asp Glu Leu Ser Tyr Phe Met Phe Pro Phe Ser Arg Asp Ser Val
665 670 675

ggt ctc ctc ata acc aag cac gtc gtg tac gag aac gga aag gcc gag 2160
Gly Leu Leu Ile Thr Lys His Val Val Tyr Glu Asn Gly Lys Ala Glu
680 685 690

ata tac ggc gcc acc gac tac gag aag agc gag aag ctt ggg gaa gcc 2208
Ile Tyr Gly Ala Thr Asp Tyr Glu Lys Ser Glu Lys Leu Gly Glu Ala
695 700 705

acc gtc aag aac acg agc gaa gga atc gaa gtc gtc ctt ccc ttt gac 2256
Thr Val Lys Asn Thr Ser Glu Gly Ile Glu Val Val Leu Pro Phe Asp
710 715 720 725

tac ata gaa aac ccc tcc gac ttc tac ttc gct gtc tcg acg gtc aaa 2304
Tyr Ile Glu Asn Pro Ser Asp Phe Tyr Phe Ala Val Ser Thr Val Lys
730 735 740

gat gga gac ctt gag gtg ata agc act cct gtg gag ctc aag ctc ccg 2352
Asp Gly Asp Leu Glu Val Ile Ser Thr Pro Val Glu Leu Lys Leu Pro
745 750 755

acc gag gtc aag gga gtc gtc ata gcc gat ata acc gac cca gaa ggc 2400
Thr Glu Val Lys Gly Val Val Ile Ala Asp Ile Thr Asp Pro Glu Gly
760 765 770

gac gac cat ggg ccc gga aac tac act tat ccc acg gac aag gtc ttc 2448
Asp Asp His Gly Pro Gly Asn Tyr Thr Tyr Pro Thr Asp Lys Val Phe
775 780 785

aag cca ggt gtt ttc gac ctc ctc cgc ttc agg atg ctc gaa cag acg 2496
Lys Pro Gly Val Phe Asp Leu Leu Arg Phe Arg Met Leu Glu Gln Thr
790 795 800 805

gag agc tac gtc atg gag ttc tac ttc aag gac cta ggt ggt aac ccg 2544
Glu Ser Tyr Val Met Glu Phe Tyr Phe Lys Asp Leu Gly Gly Asn Pro
810 815 820

tgg aac gga ccc aac ggc ttc agc ctc cag ata atc gag gtc tac ctc 2592
Trp Asn Gly Pro Asn Gly Phe Ser Leu Gln Ile Ile Glu Val Tyr Leu
825 830 835

gac ttc aag gac ggt gga aac agt tcg gcc att aag atg ttc ccc gac 2640
Asp Phe Lys Asp Gly Gly Asn Ser Ser Ala Ile Lys Met Phe Pro Asp
840 845 850

gga ccg gga gcc aac gtc aac ctc gac ccc gag cat cca tgg gac gtt 2688
Gly Pro Gly Ala Asn Val Asn Leu Asp Pro Glu His Pro Trp Asp Val
855 860 865

gcc ttc agg ata gcg ggc tgg gac tac gga aac ctc atc atc ctg ccg 2736
Ala Phe Arg Ile Ala Gly Trp Asp Tyr Gly Asn Leu Ile Ile Leu Pro
870 875 880 885

aac gga acg gcc atc cag ggc gag atg cag att tcc gca gat ccg gtt 2784
Asn Gly Thr Ala Ile Gln Gly Glu Met Gln Ile Ser Ala Asp Pro Val
890 895 900

```
aag aac gcc ata ata gtc aag gtt cca aag aag tac atc gcc ata aac        2832
Lys Asn Ala Ile Ile Val Lys Val Pro Lys Lys Tyr Ile Ala Ile Asn
            905                 910                 915

gag gac tac ggc ctc tgg gga gac gtc ctc gtc ggc tcg cag gac ggc        2880
Glu Asp Tyr Gly Leu Trp Gly Asp Val Leu Val Gly Ser Gln Asp Gly
        920                 925                 930

tac ggc ccg gac aag tgg aga acg gcg gca gtg gat gcg gag cag tgg        2928
Tyr Gly Pro Asp Lys Trp Arg Thr Ala Ala Val Asp Ala Glu Gln Trp
    935                 940                 945

aag ctt gga ggt gcg gac ccg cag gca gtc ata aac ggc gtg gcc ccg        2976
Lys Leu Gly Gly Ala Asp Pro Gln Ala Val Ile Asn Gly Val Ala Pro
950                 955                 960                 965

cgc gtc att gat gag ctg gtt ccg cag ggc ttt gaa ccg acc cag gag        3024
Arg Val Ile Asp Glu Leu Val Pro Gln Gly Phe Glu Pro Thr Gln Glu
                970                 975                 980

gag cag ctg agc agc tac gat gca aac gac atg aag ctc gcc act gtc        3072
Glu Gln Leu Ser Ser Tyr Asp Ala Asn Asp Met Lys Leu Ala Thr Val
            985                 990                 995

aag gcg ctg  cta ctc ctc aag cag  ggc atc gtt gtg acc  gac ccg         3117
Lys Ala Leu  Leu Leu Leu Lys Gln  Gly Ile Val Val Thr  Asp Pro
        1000                 1005                 1010

gag gga gac  gac cac ggg ccg gga  acg tac acc tat ccg  acg gac         3162
Glu Gly Asp  Asp His Gly Pro Gly  Thr Tyr Thr Tyr Pro  Thr Asp
        1015                 1020                 1025

aaa gtt ttc  aag ccc ggt gtt ttc  gac ctc ctc aag ttc  aag gtg         3207
Lys Val Phe  Lys Pro Gly Val Phe  Asp Leu Leu Lys Phe  Lys Val
        1030                 1035                 1040

acc gag gga  agc gac gac tgg acg  ctg gag ttc cac ttc  aaa gac         3252
Thr Glu Gly  Ser Asp Asp Trp Thr  Leu Glu Phe His Phe  Lys Asp
        1045                 1050                 1055

ctc ggt gga  aac ccg tgg aac ggg  ccg aac ggc ttc agc  ctg cag         3297
Leu Gly Gly  Asn Pro Trp Asn Gly  Pro Asn Gly Phe Ser  Leu Gln
        1060                 1065                 1070

ata atc gag  gta tac ttc gac ttc  aag gag ggc ggg aac  gtc tcg         3342
Ile Ile Glu  Val Tyr Phe Asp Phe  Lys Glu Gly Gly Asn  Val Ser
        1075                 1080                 1085

gcc att aag  atg ttc ccg gat ggg  ccc gga agc aac gtc  cgt ctt         3387
Ala Ile Lys  Met Phe Pro Asp Gly  Pro Gly Ser Asn Val  Arg Leu
        1090                 1095                 1100

gat cca aat  cac cca tgg gac ctg  gcg ctt agg ata gcc  ggc tgg         3432
Asp Pro Asn  His Pro Trp Asp Leu  Ala Leu Arg Ile Ala  Gly Trp
        1105                 1110                 1115

gac tac gga  aac ctg ata att ctg  ccc gac gga acc gcc  tac caa         3477
Asp Tyr Gly  Asn Leu Ile Ile Leu  Pro Asp Gly Thr Ala  Tyr Gln
        1120                 1125                 1130

ggc gag atg  cag att tcc gca gat  ccg gtt aag aac gcc  ata ata         3522
Gly Glu Met  Gln Ile Ser Ala Asp  Pro Val Lys Asn Ala  Ile Ile
```

```
        1135                1140                1145

gtc aag gtt  cca aag aag tac ctg  aac ata tcc gac tac  gga ctc    3567
Val Lys Val  Pro Lys Lys Tyr Leu  Asn Ile Ser Asp Tyr  Gly Leu
        1150                1155                1160

tac acc gcc  gtc atc gtg ggt tcc  caa gac ggg tac ggc  ccg gac    3612
Tyr Thr Ala  Val Ile Val Gly Ser  Gln Asp Gly Tyr Gly  Pro Asp
        1165                1170                1175

aag tgg agg  ccc gtg gcc gct gag  gcc gag cag tgg aag  ctc gga    3657
Lys Trp Arg  Pro Val Ala Ala Glu  Ala Glu Gln Trp Lys  Leu Gly
        1180                1185                1190

ggc gca gac  ccc cag gcg gtc ata  gac aac ctc gta cca  agg gtc    3702
Gly Ala Asp  Pro Gln Ala Val Ile  Asp Asn Leu Val Pro  Arg Val
        1195                1200                1205

gtt gat gaa  ctc gtg ccg gag ggc  ttc aag cca acg cag  gag gag    3747
Val Asp Glu  Leu Val Pro Glu Gly  Phe Lys Pro Thr Gln  Glu Glu
        1210                1215                1220

cag ctg agc  agc tac gac ctt gag  aag aag acc ctg gcg  acg gtg    3792
Gln Leu Ser  Ser Tyr Asp Leu Glu  Lys Lys Thr Leu Ala  Thr Val
        1225                1230                1235

ctc atg gta  ccg ctc gtc aat ggg  act ggc ggc gag gaa  cca acg    3837
Leu Met Val  Pro Leu Val Asn Gly  Thr Gly Gly Glu Glu  Pro Thr
        1240                1245                1250

ccg acg gag  agc cca acg gaa acg  acg aca acc aca ccc  agc gaa    3882
Pro Thr Glu  Ser Pro Thr Glu Thr  Thr Thr Thr Thr Pro  Ser Glu
        1255                1260                1265

aca acc acc  aca act tca acg acc  acc ggc cca agc tca  acg acc    3927
Thr Thr Thr  Thr Thr Ser Thr Thr  Thr Gly Pro Ser Ser  Thr Thr
        1270                1275                1280

acc agc aca  ccc ggc gga gga atc  tgc ggc cca ggc att  ata gcg    3972
Thr Ser Thr  Pro Gly Gly Gly Ile  Cys Gly Pro Gly Ile  Ile Ala
        1285                1290                1295

ggc ctg gcc  ctg ata ccg ctc ctc  ctc aag agg agg aac  tga        4014
Gly Leu Ala  Leu Ile Pro Leu Leu  Leu Lys Arg Arg Asn
        1300                1305                1310
```

<210> 11
<211> 1337
<212> PRT
<213> Thermococcus hydrothermalis

<400> 11

```
Met Arg Arg Val Val Ala Leu Phe Ile Ala Ile Leu Met Leu Gly Ser
        -25                 -20                 -15

Ile Val Gly Ala Asn Val Lys Ser Val Gly Ala Ala Glu Pro Lys Pro
    -10                 -5              -1  1               5

Leu Asn Val Ile Ile Val Trp His Gln His Gln Pro Tyr Tyr Tyr Asp
```

```
                10                  15                  20

Pro Val Gln Asp Val Tyr Thr Arg Pro Trp Val Arg Leu His Ala Ala
            25                  30                  35

Asn Asn Tyr Trp Lys Met Ala His Tyr Leu Ser Gln Tyr Pro Glu Val
        40                  45                  50

His Ala Thr Ile Asp Leu Ser Gly Ser Leu Ile Ala Gln Leu Ala Asp
    55                  60                  65

Tyr Met Asn Gly Lys Lys Asp Thr Tyr Gln Ile Ile Thr Glu Lys Ile
70                  75                  80                  85

Ala Asn Gly Glu Pro Leu Thr Val Asp Glu Lys Trp Phe Met Leu Gln
                90                  95                  100

Ala Pro Gly Gly Phe Phe Asp Asn Thr Ile Pro Trp Asn Gly Glu Pro
            105                 110                 115

Ile Thr Asp Pro Asn Gly Asn Pro Ile Arg Asp Phe Trp Asp Arg Tyr
        120                 125                 130

Thr Glu Leu Lys Asn Lys Met Leu Ser Ala Lys Ala Lys Tyr Ala Asn
    135                 140                 145

Phe Val Thr Glu Ser Gln Lys Val Ala Val Thr Asn Glu Phe Thr Glu
150                 155                 160                 165

Gln Asp Tyr Ile Asp Leu Ala Val Leu Phe Asn Leu Ala Trp Ile Asp
                170                 175                 180

Tyr Asn Tyr Ile Thr Ser Thr Pro Glu Phe Lys Ala Leu Tyr Asp Lys
            185                 190                 195

Val Asp Glu Gly Gly Tyr Thr Arg Ala Asp Val Lys Thr Val Leu Asp
        200                 205                 210

Ala Gln Ile Trp Leu Leu Asn His Thr Phe Glu Glu His Glu Lys Ile
    215                 220                 225

Asn Leu Leu Leu Gly Asn Gly Asn Val Glu Val Thr Val Val Pro Tyr
230                 235                 240                 245

Ala His Pro Ile Gly Pro Ile Leu Asn Asp Phe Gly Trp Asp Ser Asp
                250                 255                 260
```

```
Phe Asn Asp Gln Val Lys Lys Ala Asp Glu Leu Tyr Lys Pro Tyr Leu
            265                 270                 275

Gly Gly Gly Thr Ala Val Pro Lys Gly Gly Trp Ala Ala Glu Ser Ala
        280                 285                 290

Leu Asn Asp Lys Thr Leu Glu Ile Leu Ala Glu Asn Gly Trp Glu Trp
    295                 300                 305

Val Met Thr Asp Gln Met Val Leu Gly Lys Leu Gly Ile Glu Gly Thr
310                 315                 320                 325

Val Glu Asn Tyr His Lys Pro Trp Val Ala Glu Phe Asn Gly Lys Lys
                330                 335                 340

Ile Tyr Leu Phe Pro Arg Asn His Asp Leu Ser Asp Arg Val Gly Phe
            345                 350                 355

Thr Tyr Ser Gly Met Asn Gln Gln Gln Ala Val Glu Asp Phe Val Asn
        360                 365                 370

Glu Leu Leu Lys Leu Gln Lys Gln Asn Tyr Asp Gly Ser Leu Val Tyr
    375                 380                 385

Val Val Thr Leu Asp Gly Glu Asn Pro Val Glu Asn Tyr Pro Tyr Asp
390                 395                 400                 405

Gly Glu Leu Phe Leu Thr Glu Leu Tyr Lys Lys Leu Thr Glu Leu Gln
                410                 415                 420

Glu Gln Gly Leu Ile Arg Thr Leu Thr Pro Ser Glu Tyr Ile Gln Leu
            425                 430                 435

Tyr Gly Asp Lys Ala Asn Lys Leu Thr Pro Arg Met Met Glu Arg Leu
        440                 445                 450

Asp Leu Thr Gly Asp Asn Val Asn Ala Leu Leu Lys Ala Gln Ser Leu
    455                 460                 465

Gly Glu Leu Tyr Asp Met Thr Gly Val Lys Glu Glu Met Gln Trp Pro
470                 475                 480                 485

Glu Ser Ser Trp Ile Asp Gly Thr Leu Ser Thr Trp Ile Gly Glu Pro
                490                 495                 500

Gln Glu Asn Tyr Gly Trp Tyr Trp Leu Tyr Met Ala Arg Lys Ala Leu
            505                 510                 515
```

```
Met Glu Asn Lys Asp Lys Met Ser Gln Ala Asp Trp Glu Lys Ala Tyr
        520                 525                 530

Glu Tyr Leu Leu Arg Ala Glu Ala Ser Asp Trp Phe Trp Trp Tyr Gly
    535                 540                 545

Ser Asp Gln Asp Ser Gly Gln Asp Tyr Thr Phe Asp Arg Tyr Leu Lys
550                 555                 560                 565

Thr Tyr Leu Tyr Glu Met Tyr Lys Leu Ala Gly Val Glu Pro Pro Ser
                570                 575                 580

Tyr Leu Phe Gly Asn Tyr Phe Pro Asp Gly Glu Pro Tyr Thr Thr Arg
            585                 590                 595

Gly Leu Val Gly Leu Lys Asp Gly Glu Met Lys Asn Phe Ser Ser Met
        600                 605                 610

Ser Pro Leu Ala Lys Gly Val Ser Val Tyr Phe Asp Gly Glu Gly Ile
    615                 620                 625

His Phe Ile Val Lys Gly Asn Leu Asp Arg Phe Glu Val Ser Ile Trp
630                 635                 640                 645

Glu Lys Asp Glu Arg Val Gly Asn Thr Phe Thr Arg Leu Gln Glu Lys
                650                 655                 660

Pro Asp Glu Leu Ser Tyr Phe Met Phe Pro Phe Ser Arg Asp Ser Val
            665                 670                 675

Gly Leu Leu Ile Thr Lys His Val Val Tyr Glu Asn Gly Lys Ala Glu
        680                 685                 690

Ile Tyr Gly Ala Thr Asp Tyr Glu Lys Ser Glu Lys Leu Gly Glu Ala
    695                 700                 705

Thr Val Lys Asn Thr Ser Glu Gly Ile Glu Val Val Leu Pro Phe Asp
710                 715                 720                 725

Tyr Ile Glu Asn Pro Ser Asp Phe Tyr Phe Ala Val Ser Thr Val Lys
                730                 735                 740

Asp Gly Asp Leu Glu Val Ile Ser Thr Pro Val Glu Leu Lys Leu Pro
            745                 750                 755

Thr Glu Val Lys Gly Val Val Ile Ala Asp Ile Thr Asp Pro Glu Gly
        760                 765                 770
```

```
Asp Asp His Gly Pro Gly Asn Tyr Thr Tyr Pro Thr Asp Lys Val Phe
    775                 780                 785

Lys Pro Gly Val Phe Asp Leu Leu Arg Phe Arg Met Leu Glu Gln Thr
790                 795                 800                 805

Glu Ser Tyr Val Met Glu Phe Tyr Phe Lys Asp Leu Gly Gly Asn Pro
                810                 815                 820

Trp Asn Gly Pro Asn Gly Phe Ser Leu Gln Ile Ile Glu Val Tyr Leu
            825                 830                 835

Asp Phe Lys Asp Gly Gly Asn Ser Ser Ala Ile Lys Met Phe Pro Asp
        840                 845                 850

Gly Pro Gly Ala Asn Val Asn Leu Asp Pro Glu His Pro Trp Asp Val
    855                 860                 865

Ala Phe Arg Ile Ala Gly Trp Asp Tyr Gly Asn Leu Ile Ile Leu Pro
870                 875                 880                 885

Asn Gly Thr Ala Ile Gln Gly Glu Met Gln Ile Ser Ala Asp Pro Val
                890                 895                 900

Lys Asn Ala Ile Ile Val Lys Val Pro Lys Lys Tyr Ile Ala Ile Asn
            905                 910                 915

Glu Asp Tyr Gly Leu Trp Gly Asp Val Leu Val Gly Ser Gln Asp Gly
        920                 925                 930

Tyr Gly Pro Asp Lys Trp Arg Thr Ala Ala Val Asp Ala Glu Gln Trp
    935                 940                 945

Lys Leu Gly Gly Ala Asp Pro Gln Ala Val Ile Asn Gly Val Ala Pro
950                 955                 960                 965

Arg Val Ile Asp Glu Leu Val Pro Gln Gly Phe Glu Pro Thr Gln Glu
                970                 975                 980

Glu Gln Leu Ser Ser Tyr Asp Ala Asn Asp Met Lys Leu Ala Thr Val
            985                 990                 995

Lys Ala Leu  Leu Leu Leu Lys Gln  Gly Ile Val Val Thr  Asp Pro
        1000                 1005                 1010

Glu Gly Asp  Asp His Gly Pro Gly  Thr Tyr Thr Tyr Pro  Thr Asp
```

1015 1020 1025

Lys Val Phe Lys Pro Gly Val Phe Asp Leu Leu Lys Phe Lys Val
1030 1035 1040

Thr Glu Gly Ser Asp Asp Trp Thr Leu Glu Phe His Phe Lys Asp
1045 1050 1055

Leu Gly Gly Asn Pro Trp Asn Gly Pro Asn Gly Phe Ser Leu Gln
1060 1065 1070

Ile Ile Glu Val Tyr Phe Asp Phe Lys Glu Gly Gly Asn Val Ser
1075 1080 1085

Ala Ile Lys Met Phe Pro Asp Gly Pro Gly Ser Asn Val Arg Leu
1090 1095 1100

Asp Pro Asn His Pro Trp Asp Leu Ala Leu Arg Ile Ala Gly Trp
1105 1110 1115

Asp Tyr Gly Asn Leu Ile Ile Leu Pro Asp Gly Thr Ala Tyr Gln
1120 1125 1130

Gly Glu Met Gln Ile Ser Ala Asp Pro Val Lys Asn Ala Ile Ile
1135 1140 1145

Val Lys Val Pro Lys Lys Tyr Leu Asn Ile Ser Asp Tyr Gly Leu
1150 1155 1160

Tyr Thr Ala Val Ile Val Gly Ser Gln Asp Gly Tyr Gly Pro Asp
1165 1170 1175

Lys Trp Arg Pro Val Ala Ala Glu Ala Glu Gln Trp Lys Leu Gly
1180 1185 1190

Gly Ala Asp Pro Gln Ala Val Ile Asp Asn Leu Val Pro Arg Val
1195 1200 1205

Val Asp Glu Leu Val Pro Glu Gly Phe Lys Pro Thr Gln Glu Glu
1210 1215 1220

Gln Leu Ser Ser Tyr Asp Leu Glu Lys Lys Thr Leu Ala Thr Val
1225 1230 1235

Leu Met Val Pro Leu Val Asn Gly Thr Gly Gly Glu Glu Pro Thr
1240 1245 1250

```
Pro Thr Glu  Ser Pro Thr Glu Thr  Thr Thr Thr Thr Pro  Ser Glu
        1255                 1260                 1265

Thr Thr Thr  Thr Thr Ser Thr Thr  Thr Gly Pro Ser Ser  Thr Thr
        1270                 1275                 1280

Thr Ser Thr  Pro Gly Gly Gly Ile  Cys Gly Pro Gly Ile  Ile Ala
        1285                 1290                 1295

Gly Leu Ala  Leu Ile Pro Leu Leu  Leu Lys Arg Arg Asn
        1300                 1305                 1310
```

<210> 12
<211> 809
<212> PRT
<213> Artificial Sequence

<220>
<223> Hybrid pullulanase of Thermoccus hydrothermalis and Thermococcus litoralis

<220>
<221> SIGNAL
<222> (1)..(27)

<220>
<221> mat_peptide
<222> (28)..(809)

<400> 12

```
Met Lys Lys Pro Leu Gly Lys Ile Val Ala Ser Thr Ala Leu Leu Ile
        -25                 -20                 -15

Ser Val Ala Phe Ser Ser Ser Ile Ala Ser Ala Glu Glu Pro Lys Pro
    -10                 -5              -1  1               5

Leu Asn Val Ile Ile Val Trp His Gln His Gln Pro Tyr Tyr Tyr Asp
                10                  15                  20

Pro Ile Gln Asp Ile Tyr Thr Arg Pro Trp Val Arg Leu His Ala Ala
            25                  30                  35

Asn Asn Tyr Trp Lys Met Ala Asn Tyr Leu Ser Lys Tyr Pro Asp Val
        40                  45                  50

His Val Ala Ile Asp Leu Ser Gly Ser Leu Ile Ala Gln Leu Ala Asp
    55                  60                  65

Tyr Met Asn Gly Lys Lys Asp Thr Tyr Gln Ile Val Thr Glu Lys Ile
70                  75                  80                  85
```

```
Ala Asn Gly Glu Pro Leu Thr Leu Glu Asp Lys Trp Phe Met Leu Gln
                90                  95                  100

Ala Pro Gly Gly Phe Phe Asp His Thr Ile Pro Trp Asn Gly Glu Pro
            105                 110                 115

Val Ala Asp Glu Asn Gly Asn Pro Tyr Arg Glu Gln Trp Asp Arg Tyr
        120                 125                 130

Ala Glu Leu Lys Asp Lys Arg Asn Asn Ala Phe Lys Lys Tyr Ala Asn
    135                 140                 145

Leu Pro Leu Asn Glu Gln Lys Val Lys Ile Thr Ala Glu Phe Thr Glu
150                 155                 160                 165

Gln Asp Tyr Ile Asp Leu Ala Val Leu Phe Asn Leu Ala Trp Ile Asp
                170                 175                 180

Tyr Asn Tyr Ile Ile Asn Thr Pro Glu Leu Lys Ala Leu Tyr Asp Lys
            185                 190                 195

Val Asp Val Gly Gly Tyr Thr Lys Glu Asp Val Ala Thr Val Leu Lys
        200                 205                 210

His Gln Met Trp Leu Leu Asn His Thr Phe Glu Glu His Glu Lys Ile
    215                 220                 225

Asn Tyr Leu Leu Gly Asn Gly Asn Val Glu Val Thr Val Val Pro Tyr
230                 235                 240                 245

Ala His Pro Ile Gly Pro Leu Leu Asn Asp Phe Gly Trp Tyr Glu Asp
                250                 255                 260

Phe Asp Ala His Val Lys Lys Ala His Glu Leu Tyr Lys Lys Tyr Leu
            265                 270                 275

Gly Asp Asn Arg Val Glu Pro Gln Gly Gly Trp Ala Ala Glu Ser Ala
        280                 285                 290

Leu Asn Asp Lys Thr Leu Glu Ile Leu Thr Asn Asn Gly Trp Lys Trp
    295                 300                 305

Val Met Thr Asp Gln Met Val Leu Asp Ile Leu Gly Ile Pro Asn Thr
310                 315                 320                 325

Val Glu Asn Tyr Tyr Lys Pro Trp Val Ala Glu Phe Asn Gly Lys Lys
                330                 335                 340
```

Ile Tyr Leu Phe Pro Arg Asn His Asp Leu Ser Asp Arg Val Gly Phe
345 350 355

Arg Tyr Ser Gly Met Asn Gln Tyr Gln Ala Val Glu Asp Phe Val Asn
360 365 370

Glu Leu Leu Lys Val Gln Lys Glu Asn Tyr Asp Gly Ser Leu Val Tyr
375 380 385

Val Val Thr Leu Asp Gly Glu Asn Pro Trp Glu His Tyr Pro Phe Asp
390 395 400 405

Gly Lys Ile Phe Leu Glu Glu Leu Tyr Lys Lys Leu Thr Glu Leu Gln
410 415 420

Lys Gln Gly Leu Ile Arg Thr Val Thr Pro Ser Glu Tyr Ile Gln Met
425 430 435

Tyr Gly Asp Lys Ala Asn Lys Leu Thr Pro Arg Met Met Glu Arg Leu
440 445 450

Asp Leu Thr Gly Asp Asn Val Asn Ala Leu Leu Lys Ala Gln Ser Leu
455 460 465

Gly Glu Leu Tyr Asp Met Thr Gly Val Lys Glu Glu Met Gln Trp Pro
470 475 480 485

Glu Ser Ser Trp Ile Asp Gly Thr Leu Ser Thr Trp Ile Gly Glu Pro
490 495 500

Gln Glu Asn Tyr Gly Trp Tyr Trp Leu Tyr Met Ala Arg Lys Ala Leu
505 510 515

Met Glu Asn Lys Asp Lys Met Ser Gln Ala Asp Trp Glu Lys Ala Tyr
520 525 530

Glu Tyr Leu Leu Arg Ala Glu Ala Ser Asp Trp Phe Trp Trp Tyr Gly
535 540 545

Ser Asp Gln Asp Ser Gly Gln Asp Tyr Thr Phe Asp Arg Tyr Leu Lys
550 555 560 565

Thr Tyr Leu Tyr Glu Met Tyr Lys Leu Ala Gly Val Glu Pro Pro Ser
570 575 580

Tyr Leu Phe Gly Asn Tyr Phe Pro Asp Gly Glu Pro Tyr Thr Thr Arg

```
            585                 590                 595

Gly Leu Val Gly Leu Lys Asp Gly Glu Met Lys Asn Phe Ser Ser Met
        600                 605                 610

Ser Pro Leu Ala Lys Gly Val Ser Val Tyr Phe Asp Gly Glu Gly Ile
    615                 620                 625

His Phe Ile Val Lys Gly Asn Leu Asp Arg Phe Glu Val Ser Ile Trp
630                 635                 640                 645

Glu Lys Asp Glu Arg Val Gly Asn Thr Phe Thr Arg Leu Gln Glu Lys
                650                 655                 660

Pro Asp Glu Leu Ser Tyr Phe Met Phe Pro Phe Ser Arg Asp Ser Val
            665                 670                 675

Gly Leu Leu Ile Thr Lys His Val Val Tyr Glu Asn Gly Lys Ala Glu
        680                 685                 690

Ile Tyr Gly Ala Thr Asp Tyr Glu Lys Ser Glu Lys Leu Gly Glu Ala
    695                 700                 705

Thr Val Lys Asn Thr Ser Glu Gly Ile Glu Val Val Leu Pro Phe Asp
710                 715                 720                 725

Tyr Ile Glu Asn Pro Ser Asp Phe Tyr Phe Ala Val Ser Thr Val Lys
                730                 735                 740

Asp Gly Asp Leu Glu Val Ile Ser Thr Pro Val Glu Leu Lys Leu Pro
            745                 750                 755

Thr Glu Val Lys Gly Val Val Ile Ala Asp Ile Thr Asp Pro Glu Gly
        760                 765                 770

Asp Asp His Gly Pro Gly Asn Tyr Thr
    775                 780
```

<210> 13
<211> 412
<212> PRT
<213> Pyrococcus furiosus

<220>
<221> mat_peptide
<222> (1)..(412)
<223> Pyrococcus furiosus protease (Pfu)

<400> 13

```
Ala Glu Leu Glu Gly Leu Asp Glu Ser Ala Ala Gln Val Met Ala Thr
1               5                   10                  15

Tyr Val Trp Asn Leu Gly Tyr Asp Gly Ser Gly Ile Thr Ile Gly Ile
            20                  25                  30

Ile Asp Thr Gly Ile Asp Ala Ser His Pro Asp Leu Gln Gly Lys Val
        35                  40                  45

Ile Gly Trp Val Asp Phe Val Asn Gly Arg Ser Tyr Pro Tyr Asp Asp
    50                  55                  60

His Gly His Gly Thr His Val Ala Ser Ile Ala Ala Gly Thr Gly Ala
65                  70                  75                  80

Ala Ser Asn Gly Lys Tyr Lys Gly Met Ala Pro Gly Ala Lys Leu Ala
                85                  90                  95

Gly Ile Lys Val Leu Gly Ala Asp Gly Ser Gly Ser Ile Ser Thr Ile
            100                 105                 110

Ile Lys Gly Val Glu Trp Ala Val Asp Asn Lys Asp Lys Tyr Gly Ile
        115                 120                 125

Lys Val Ile Asn Leu Ser Leu Gly Ser Ser Gln Ser Ser Asp Gly Thr
    130                 135                 140

Asp Ala Leu Ser Gln Ala Val Asn Ala Ala Trp Asp Ala Gly Leu Val
145                 150                 155                 160

Val Val Val Ala Ala Gly Asn Ser Gly Pro Asn Lys Tyr Thr Ile Gly
                165                 170                 175

Ser Pro Ala Ala Ala Ser Lys Val Ile Thr Val Gly Ala Val Asp Lys
            180                 185                 190

Tyr Asp Val Ile Thr Ser Phe Ser Ser Arg Gly Pro Thr Ala Asp Gly
        195                 200                 205

Arg Leu Lys Pro Glu Val Val Ala Pro Gly Asn Trp Ile Ile Ala Ala
    210                 215                 220

Arg Ala Ser Gly Thr Ser Met Gly Gln Pro Ile Asn Asp Tyr Tyr Thr
225                 230                 235                 240

Ala Ala Pro Gly Thr Ser Met Ala Thr Pro His Val Ala Gly Ile Ala
                245                 250                 255
```

```
Ala Leu Leu Leu Gln Ala His Pro Ser Trp Thr Pro Asp Lys Val Lys
            260                 265                 270

Thr Ala Leu Ile Glu Thr Ala Asp Ile Val Lys Pro Asp Glu Ile Ala
        275                 280                 285

Asp Ile Ala Tyr Gly Ala Gly Arg Val Asn Ala Tyr Lys Ala Ile Asn
    290                 295                 300

Tyr Asp Asn Tyr Ala Lys Leu Val Phe Thr Gly Tyr Val Ala Asn Lys
305                 310                 315                 320

Gly Ser Gln Thr His Gln Phe Val Ile Ser Gly Ala Ser Phe Val Thr
                325                 330                 335

Ala Thr Leu Tyr Trp Asp Asn Ala Asn Ser Asp Leu Asp Leu Tyr Leu
            340                 345                 350

Tyr Asp Pro Asn Gly Asn Gln Val Asp Tyr Ser Tyr Thr Ala Tyr Tyr
        355                 360                 365

Gly Phe Glu Lys Val Gly Tyr Tyr Asn Pro Thr Asp Gly Thr Trp Thr
    370                 375                 380

Ile Lys Val Val Ser Tyr Ser Gly Ser Ala Asn Tyr Gln Val Asp Val
385                 390                 395                 400

Val Ser Asp Gly Ser Leu Ser Gln Pro Gly Ser Ser
                405                 410
```

<210> 14
<211> 595
<212> PRT
<213> Penicillium oxalicum

<220>
<221> mat_peptide
<222> (1)..(595)
<223> mature Penicillium oxalicum glucoamylase sequence

<400> 14

```
Arg Pro Asp Pro Lys Gly Gly Asn Leu Thr Pro Phe Ile His Lys Glu
1               5                   10                  15

Gly Glu Arg Ser Leu Gln Gly Ile Leu Asp Asn Leu Gly Gly Arg Gly
            20                  25                  30
```

```
Lys Lys Thr Pro Gly Thr Ala Ala Gly Leu Phe Ile Ala Ser Pro Asn
        35                  40                  45

Thr Glu Asn Pro Asn Tyr Tyr Tyr Thr Trp Thr Arg Asp Ser Ala Leu
    50                  55                  60

Thr Ala Lys Cys Leu Ile Asp Leu Phe Glu Asp Ser Arg Ala Lys Phe
65                  70                  75                  80

Pro Ile Asp Arg Lys Tyr Leu Glu Thr Gly Ile Arg Asp Tyr Lys Ser
                85                  90                  95

Ser Gln Ala Ile Leu Gln Ser Val Ser Asn Pro Ser Gly Thr Leu Lys
            100                 105                 110

Asp Gly Ser Gly Leu Gly Glu Pro Lys Phe Glu Ile Asp Leu Asn Pro
        115                 120                 125

Phe Ser Gly Ala Trp Gly Arg Pro Gln Arg Asp Gly Pro Ala Leu Arg
    130                 135                 140

Ala Thr Ala Met Ile Thr Tyr Ala Asn Tyr Leu Ile Ser His Gly Gln
145                 150                 155                 160

Lys Ser Asp Val Ser Gln Val Met Trp Pro Ile Ile Ala Asn Asp Leu
                165                 170                 175

Ala Tyr Val Gly Gln Tyr Trp Asn Asn Thr Gly Phe Asp Leu Trp Glu
            180                 185                 190

Glu Val Asp Gly Ser Ser Phe Phe Thr Ile Ala Val Gln His Arg Ala
        195                 200                 205

Leu Val Glu Gly Ser Gln Leu Ala Lys Lys Leu Gly Lys Ser Cys Asp
    210                 215                 220

Ala Cys Asp Ser Gln Pro Pro Gln Ile Leu Cys Phe Leu Gln Ser Phe
225                 230                 235                 240

Trp Asn Gly Lys Tyr Ile Thr Ser Asn Ile Asn Thr Gln Ala Ser Arg
                245                 250                 255

Ser Gly Ile Asp Leu Asp Ser Val Leu Gly Ser Ile His Thr Phe Asp
            260                 265                 270

Pro Glu Ala Ala Cys Asp Asp Ala Thr Phe Gln Pro Cys Ser Ala Arg
        275                 280                 285
```

```
Ala Leu Ala Asn His Lys Val Tyr Val Asp Ser Phe Arg Ser Ile Tyr
    290                 295                 300

Lys Ile Asn Ala Gly Leu Ala Glu Gly Ser Ala Ala Asn Val Gly Arg
305                 310                 315                 320

Tyr Pro Glu Asp Val Tyr Gln Gly Gly Asn Pro Trp Tyr Leu Ala Thr
                325                 330                 335

Leu Gly Ala Ser Glu Leu Leu Tyr Asp Ala Leu Tyr Gln Trp Asp Arg
            340                 345                 350

Leu Gly Lys Leu Glu Val Ser Glu Thr Ser Leu Ser Phe Phe Lys Asp
        355                 360                 365

Phe Asp Ala Thr Val Lys Ile Gly Ser Tyr Ser Arg Asn Ser Lys Thr
    370                 375                 380

Tyr Lys Lys Leu Thr Gln Ser Ile Lys Ser Tyr Ala Asp Gly Phe Ile
385                 390                 395                 400

Gln Leu Val Gln Gln Tyr Thr Pro Ser Asn Gly Ser Leu Ala Glu Gln
                405                 410                 415

Tyr Asp Arg Asn Thr Ala Ala Pro Leu Ser Ala Asn Asp Leu Thr Trp
            420                 425                 430

Ser Phe Ala Ser Phe Leu Thr Ala Thr Gln Arg Arg Asp Ala Val Val
        435                 440                 445

Pro Pro Ser Trp Gly Ala Lys Ser Ala Asn Lys Val Pro Thr Thr Cys
    450                 455                 460

Ser Ala Ser Pro Val Val Gly Thr Tyr Lys Ala Pro Thr Ala Thr Phe
465                 470                 475                 480

Ser Ser Lys Thr Lys Cys Val Pro Ala Lys Asp Ile Val Pro Ile Thr
                485                 490                 495

Phe Tyr Leu Ile Glu Asn Thr Tyr Tyr Gly Glu Asn Val Phe Met Ser
            500                 505                 510

Gly Asn Ile Thr Ala Leu Gly Asn Trp Asp Ala Lys Lys Gly Phe Pro
        515                 520                 525

Leu Thr Ala Asn Leu Tyr Thr Gln Asp Gln Asn Leu Trp Phe Ala Ser
    530                 535                 540
```

```
Val Glu Phe Ile Pro Ala Gly Thr Pro Phe Glu Tyr Lys Tyr Tyr Lys
545                 550                 555                 560

Val Glu Pro Asn Gly Asp Ile Thr Trp Glu Lys Gly Pro Asn Arg Val
                565                 570                 575

Phe Val Ala Pro Thr Gly Cys Pro Val Gln Pro His Ser Asn Asp Val
            580                 585                 590

Trp Gln Phe
        595
```

<210> 15
<211> 25
<212> DNA
<213> Artificial Sequence

<220>
<223> Sense Primer

<400> 15
atgcgtctca ctctattatc aggtg 25

<210> 16
<211> 39
<212> DNA
<213> Artificial Sequence

<220>
<223> Primer F

<400> 16
acacaactgg ggatccacca tgcgtctcac tctattatc 39

<210> 17
<211> 37
<212> DNA
<213> Artificial Sequence

<220>
<223> Primer R

<400> 17
agatctcgag aagcttaaaa ctgccacacg tcgttgg 37

<210> 18
<211> 18
<212> DNA
<213> Artificial Sequence

<220>
<223> Primer K79V F 18mer

<400> 18
gcagtctttc caattgac 18

<210> 19
<211> 18
<212> DNA
<213> Artificial Sequence

<220>
<223> Primer K79V R 18mer

<400> 19
aattggaaag actgcccg 18

<210> 20
<211> 39
<212> DNA
<213> Artificial Sequence

<220>
<223> Primer F-NP003940

<400> 20
acacaactgg ggatccacca tgcgtctcac tctattatc 39

<210> 21
<211> 37
<212> DNA
<213> Artificial Sequence

<220>
<223> Primer F-NP003940

<400> 21
agatctcgag aagcttaaaa ctgccacacg tcgttgg 37

## Description

### FIELD OF THE INVENTION

The present invention relates to processes for producing fermentation products from starch-containing material. The invention also relates to a composition suitable for use in a process of the invention. The processes and the compositions are characterised by a particular combination of alpha-amylases and proteases.

### SEQUENCE LISTING

This patent contains a Sequence Listing.

### BACKGROUND OF THE INVENTION

Production of fermentation products, such as ethanol, from starch-containing material is well-known in the art. Industrially two different kinds of processes are used today. The most commonly used process, often referred to as a "conventional process", includes liquefying gelatinized starch at high temperature using typically a bacterial alpha-amylase, followed by simultaneous saccharification and fermentation carried out in the presence of a glucoamylase and a fermentation organism. Another well-known process, often referred to as a "raw starch hydrolysis"-process (RSH process), includes simultaneously saccharifying and fermenting granular starch below the initial gelatization temperature typically in the presence of at least a glucoamylase.

Despite significant improvement of fermentation product production processes over the past decade a significant amount of residual starch material is not converted into the desired fermentation product, such as ethanol. At least some of the unconverted residual starch material, e.g., sugars and dextrins, is in the form of non-fermentable Maillard products.

WO 2011/072191 (Novozymes) concerns protease variants with improved thermostability derived from *Thermoascus aurantiacus.*

WO 2006/086792 (Novozymes) concerns processes of producing fermentation products from starch-containing material comprising a liquefaction step where an alpha-amylase and a protease is present during liquefaction.

WO 2012/088303 (Novozymes, published 28 June 2012) concerns processes of producing fermentation products from starch-containing material, where liquefaction is carried out at a pH in the range from 4.5-5.0 in the presence of a thermostable alpha-amylase and a thermostable protease.

Therefore, there is still a desire and need for providing processes for producing fermentation products, such as ethanol, from starch-containing material that can provide a higher fermentation product yield, or other advantages, compared to a conventional process.

### SUMMARY OF THE INVENTION

The present invention relates to processes of producing fermentation products, such as ethanol, from starch-containing material comprising the steps of:

i) liquefying the starch-containing material at a pH in the range between from above 5.0 to 7.0 at a temperature above the initial gelatinization temperature using:

- an alpha-amylase having at least 80% sequence identity to SEQ ID NO: 1;
- a protease having at least 80% identity to SEQ ID NO: 13 and having a thermostability value of more than 20% determined as Relative Activity at 80°C/70°C; and

ii) saccharifying using a carbohydrate-source generating enzyme;
iii) fermenting using a fermenting organism.

In a preferred embodiment liquefaction is carried out at a temperature between 80-90°C, such as around 85°C. In a preferred embodiment liquefaction is carried out at a pH in the range pH above 5.0 to 6.0.

In a second aspect the invention relates to an enzyme composition comprising:

i) an alpha-amylase having at least 80% sequence identity to SEQ ID NO: 1;
ii) a protease having at least 80% identity to SEQ ID NO: 13 and having a thermostability value of more than 20% determined as Relative Activity at 80°C/70°C.

## BRIEF DESCRIPTION OF THE FIGURES

Fig. 1 shows a comparison of the 54 hour ethanol fermentation yield (%) for Alpha-Amylase 1407 with and without Protease Pfu and/or Glucoamylase PE001 added during liquefaction at pH 5.4 and 5.8, respectively, at 85°C for 2 hours.

## DETAILED DESCRIPTION OF THE INVENTION

Disclosed are processes of producing fermentation products, such as ethanol from starch-containing material using a fermenting organism.

The inventors have found that an increased ethanol yield is obtained when liquefying starch-containing material with a mature *Bacillus stearothermophilus* alpha-amylase disclosed in SEQ ID NO: 1 herein having a double deletion (I181* + G182*) and substitution N193F together with *Pyrococcus furiosus* protease (pfu S) at 85°C, at pH 5.4 or 5.8 for 2 hours.

In the first aspect the invention relates to processes for producing fermentation products, preferably ethanol, comprising the steps of:

i) liquefying the starch-containing material at a pH in the range between from above 5.0 to 7.0 at a temperature above the initial gelatinization temperature using:

- an alpha-amylase having at least 80% sequence identity to SEQ ID NO: 1;
- a protease having at least 80% identity to SEQ ID NO: 13 and having a thermostability value of more than 20% determined as Relative Activity at 80°C/70°C; and

ii) saccharifying using a carbohydrate-source generating enzyme;
iii) fermenting using a fermenting organism.

Steps ii) and iii) are carried out either sequentially or simultaneously. In a preferred embodiment steps ii) and iii) are carried out simultaneously. The alpha-amylase, thermostable protease and optionally a carbohydrate-source generating enzyme, preferably glucoamylase, and/or optionally a pullulanase may be added before and/or during liquefaction step i). A composition of the invention may suitably be used in a process of the invention. However, the enzymes may also be added separately. Examples of alpha-amylases can be found in the "Alpha-Amylase Present and/or Added During Liquefaction"-section below. Examples of thermostable proteases can be found in the "Protease Present and/or Added During Liquefaction"-section below. Examples of suitable optional carbohydrate-source generating enzymes, preferably thermostable carbohydrate-source generating enzymes, in particular a thermostable glucoamylase, can be found in the "Carbohydrate-Source Generating Enzymes Present and/or Added During Liquefaction"-section below. A suitable optional pullulanase can be found in the "Pullulanase Present and/or Added During Liquefaction"-section below.

The pH during liquefaction is above 5.0 to 7.0. such as between above 5.0-6.5, such as between 5.2-6.2, such as between pH 5.0-6.0, such as around 5.2, such as around 5.4, such as around 5.6, such as around 5.8. In an embodiment the pH is between 5.0 and 5.5.

According to the invention the temperature is above the initial gelatinization temperature. The term "initial gelatinization temperature" refers to the lowest temperature at which solubilization of starch, typically by heating, begins. The temperature can vary for different starches.

In an embodiment the temperature during liquefaction step i) is in the range from 70-100°C, such as between 75-95°C, such as between 75-90°C, preferably between 80-90°C, such as around 85°C.

In an embodiment, the process of the invention further comprises, prior to the step i), the steps of:

a) reducing the particle size of the starch-containing material, preferably by dry milling;
b) forming a slurry comprising the starch-containing material and water.

The starch-containing starting material, such as whole grains, may be reduced in particle size, e.g., by milling, in order to open up the structure, to increase surface area and allowing for further processing. Generally there are two types of processes: wet and dry milling. In dry milling whole kernels are milled and used. Wet milling gives a good separation of germ and meal (starch granules and protein). Wet milling is often applied at locations where the starch hydrolysate is used in production of, e.g., syrups. Both dry and wet millings are well known in the art of starch processing. According to the present invention dry milling is preferred. In an embodiment the particle size is reduced to between 0.05 to 3.0 mm, preferably 0.1-0.5 mm, or so that at least 30%, preferably at least 50%, more preferably at least 70%, even more preferably at least 90% of the starch-containing material fit through a sieve with a 0.05 to 3.0 mm screen, preferably 0.1-0.5 mm screen. In another embodiment at least 50%, preferably at least 70%, more preferably at least 80%, especially at least 90% of the starch-containing material fit through a sieve with # 6 screen.

The aqueous slurry may contain from 10-55 w/w-% dry solids (DS), preferably 25-45 w/w-% dry solids (DS), more preferably 30-40 w/w-% dry solids (DS) of starch-containing material.

The slurry may be heated to above the initial gelatinization temperature, preferably to between 80-90°C, between pH 5.0-7.0, preferably between 5.0 and 6.0, for 30 minutes to 5 hours, such as around 2 hours.

The alpha-amylase, thermostable protease and optional carbohydrate-source generating enzyme, in particular thermostable glucoamylase, and/or optional pullulanase may initially be added to the aqueous slurry to initiate liquefaction (thinning). In an embodiment only a portion of the enzymes is added to the aqueous slurry, while the rest of the enzymes are added during liquefaction step i).

Liquefaction step i) is according to the invention carried out for 0.5-5 hours, such as 1-3 hours, such as typically around 2 hours.

The aqueous slurry may in an embodiment be jet-cooked to further gelatinize the slurry before being subjected to liquefaction in step i). The jet-cooking may be carried out at a temperature between 110-145°C, preferably 120-140°C, such as 125-135°C, preferably around 130°C for about 1-15 minutes, preferably for about 3-10 minutes, especially around about 5 minutes.

Saccharification and Fermentation

One or more carbohydrate-source generating enzymes, in particular glucoamylase, may be present and/or added during saccharification step ii) and/or fermentation step iii). The carbohydrate-source generating enzyme may preferably be a glucoamylase, but may also be an enzyme selected from the group consisting of: beta-amylase, maltogenic amylase and alpha-glucosidase. The carbohydrate-source generating enzyme added during saccharification step ii) and/or fermentation step iii) is typically different from the optional carbohydrate-source generating enzyme, in particular thermostable glucoamylase, optionally added during liquefaction step i). In an embodiment the carbohydrate-source generating enzymes, in particular glucoamylase, is added together with a fungal alpha-amylase.

Examples of carbohydrate-source generating enzymes, including glucoamylases, can be found in the "Carbohydrate-Source Generating Enzyme Present and/or Added During Saccharification and/or Fermentation"-section below.

When doing sequential saccharification and fermentation, saccharification step ii) may be carried out at conditions well-known in the art. For instance, the saccharification step ii) may last up to from about 24 to about 72 hours. In an embodiment pre-saccharification is done. Pre-saccharification is typically done for 40-90 minutes at a temperature between 30-65°C, typically about 60°C. Pre-saccharification is followed by saccharification during fermentation in simultaneous saccharification and fermentation ("SSF). Saccharification is typically carried out at temperatures from 20-75°C, preferably from 40-70°C, typically around 60°C, and at a pH between 4 and 5, normally at about pH 4.5.

Simultaneous saccharification and fermentation ("SSF") is widely used in industrial scale fermentation product production processes, especially ethanol production processes. When doing SSF the saccharification step ii) and the fermentation step iii) are carried out simultaneously. There is no holding stage for the saccharification, meaning that a fermenting organism, such as yeast, and enzyme(s), may be added together. However, it is also contemplated to add the fermenting organism and enzyme(s) separately. SSF is according to the invention typically carried out at a temperature from 25°C to 40°C, such as from 28°C to 35°C, such as from 30°C to 34°C, preferably around about 32°C. In an embodiment fermentation is ongoing for 6 to 120 hours, in particular 24 to 96 hours. In an embodiment the pH is between 3.5-5, in particular between 3.8 and 4.3.

Fermentation Medium

"Fermentation media" or "fermentation medium" refers to the environment in which fermentation is carried out. The fermentation medium includes the fermentation substrate, that is, the carbohydrate source that is metabolized by the fermenting organism. According to the invention the fermentation medium may comprise nutrients and growth stimulator(s) for the fermenting organism(s). Nutrient and growth stimulators are widely used in the art of fermentation and include nitrogen sources, such as ammonia; urea, vitamins and minerals, or combinations thereof.

Fermenting Organisms

The term "Fermenting organism" refers to any organism, including bacterial and fungal organisms, especially yeast, suitable for use in a fermentation process and capable of producing the desired fermentation product. Especially suitable fermenting organisms are able to ferment, i.e., convert, sugars, such as glucose or maltose, directly or indirectly into the desired fermentation product, such as ethanol. Examples of fermenting organisms include fungal organisms, such as yeast. Preferred yeast includes strains of *Saccharomyces* spp., in particular, *Saccharomyces cerevisiae.*

Suitable concentrations of the viable fermenting organism during fermentation, such as SSF, are well known in the art or can easily be determined by the skilled person in the art. In one embodiment the fermenting organism, such as ethanol fermenting yeast, (e.g., *Saccharomyces cerevisiae*) is added to the fermentation medium so that the viable fermenting organism, such as yeast, count per mL of fermentation medium is in the range from $10^5$ to $10^{12}$, preferably from $10^7$ to $10^{10}$, especially about $5x10^7$.

Examples of commercially available yeast includes, e.g., RED STAR™ and ETHANOL RED™ yeast (available from Fermentis/Lesaffre, USA), FALI (available from Fleischmann's Yeast, USA), SUPERSTART and THERMOSACC™ fresh yeast (available from Ethanol Technology, WI, USA), BIOFERM AFT and XR (available from NABC - North American Bioproducts Corporation, GA, USA), GERT STRAND (available from Gert Strand AB, Sweden), and FERMIOL (available from DSM Specialties).

Starch-Containing Materials

Any suitable starch-containing material may be used according to the present invention. The starting material is generally selected based on the desired fermentation product. Examples of starch-containing materials, suitable for use in a process of the invention, include whole grains, corn, wheat, barley, rye, milo, sago, cassava, tapioca, sorghum, rice, peas, beans, or sweet potatoes, or mixtures thereof or starches derived therefrom, or cereals. Contemplated are also waxy and non-waxy types of corn and barley. In a preferred embodiment the starch-containing material, used for ethanol production according to the invention, is corn or wheat.

Fermentation Products

The term "fermentation product" means a product produced by a process including a fermentation step using a fermenting organism. Fermentation products contemplated according to the invention include alcohols (e.g., ethanol, methanol, butanol; polyols such as glycerol, sorbitol and inositol); organic acids (e.g., citric acid, acetic acid, itaconic acid, lactic acid, succinic acid, gluconic acid); ketones (e.g., acetone); amino acids (e.g., glutamic acid); gases (e.g., $H_2$ and $CO_2$); antibiotics (e.g., penicillin and tetracycline); enzymes; vitamins (e.g., riboflavin, $B_{12}$, beta-carotene); and hormones. In a preferred embodiment the fermentation product is ethanol, e.g., fuel ethanol; drinking ethanol, i.e., potable neutral spirits; or industrial ethanol or products used in the consumable alcohol industry (e.g., beer and wine), dairy industry (e.g., fermented dairy products), leather industry and tobacco industry. Preferred beer types comprise ales, stouts, porters, lagers, bitters, malt liquors, happoushu, high-alcohol beer, low-alcohol beer, low-calorie beer or light beer. Preferably processes of the invention are used for producing an alcohol, such as ethanol. The fermentation product, such as ethanol, obtained according to the invention, may be used as fuel, which is typically blended with gasoline. However, in the case of ethanol it may also be used as potable ethanol.

Recovery

Subsequent to fermentation, or SSF, the fermentation product may be separated from the fermentation medium. The slurry may be distilled to extract the desired fermentation product (e.g., ethanol). Alternatively the desired fermentation product may be extracted from the fermentation medium by micro or membrane filtration techniques. The fermentation product may also be recovered by stripping or other method well known in the art.

Alpha-Amylase Present and/or Added During Liquefaction

According to the invention an alpha-amylase having at least 80% sequence identity to SEQ ID NO: 1 is present and/or added during liquefaction together with a thermostable protease having at least 80% identity to SEQ ID NO: 13 and having a thermostability value of more than 20% determined as Relative Activity at 80°C/70°C, and optionally a carbohydrate-source generating enzyme, in particular a thermostable glucoamylase, and/or optionally a pullulanase.

Bacterial alpha-amylase

The alpha-amylase used according to the invention, as defined in claims 1 and 8, is classified under EC 3.2.1.1. A bacterial alpha-amylase used according to the invention may, e.g., be derived from a strain of the genus *Bacillus,* which is sometimes also referred to as the genus *Geobacillus.* In an embodiment the *Bacillus* alpha-amylase is derived from a strain of *Bacillus amyloliquefaciens, Bacillus licheniformis, Bacillus stearothermophilus*, or *Bacillus subtilis,* but may also be derived from other *Bacillus* sp.

The alpha-amylase for the purpose of the invention has at least 80% sequence identity to SEQ ID NOS: 1 herein, and may be an enzyme having a degree of identity of at least 90%, at least 91%, at least 92%, at least 93%, at least 94%, at least 95%, at least 96%, at least 97%, at least 98% or at least 99% to SEQ ID NO: 1 herein.

The alpha-amylase may be derived from *Bacillus stearothermophilus.* The *Bacillus stearothermophilus* alpha-amylase may be a mature wild-type or a mature variant thereof. The mature *Bacillus stearothermophilus* alpha-amylases, or variant thereof, may be naturally truncated during recombinant production. For instance, the *Bacillus stearothermophilus* alpha-amylase may be a truncated so it has around 491 amino acids (compared to SEQ ID NO: 3 in WO 99/19467), such as from 480-495 amino acids.

The *Bacillus* alpha-amylase may also be a variant and/or hybrid, as long as it has at least 80% identity to SEQ ID NO: 1. Examples of such a variant can be found in any of WO 96/23873, WO 96/23874, WO 97/41213, WO 99/19467, WO 00/60059, and WO 02/10355. Specific alpha-amylase variants are disclosed in U.S. Patent Nos. 6,093,562, 6,187,576, 6,297,038, and 7,713,723 and include *Bacillus stearothermophilus* alpha-amylase (often referred to as BSG alpha-amylase) variants having a deletion of one or two amino acids at positions R179, G180, I181 and/or G182, preferably a double deletion disclosed in WO 96/23873 - see, e.g., page 20, lines 1-10, preferably corresponding to deletion of positions I181 and G182 compared to the amino acid sequence of *Bacillus stearothermophilus* alpha-amylase set forth in SEQ ID NO: 1 herein or the deletion of amino acids R179 and G180 using SEQ ID NO: 1 herein for numbering. Even more preferred are *Bacillus* alpha-amylases, especially *Bacillus stearothermophilus* alpha-amylases, which have a double deletion corresponding to a deletion of positions 181 and 182 and further comprise a N193F substitution (also denoted I181* + G182* + N193F) compared to the wild-type BSG alpha-amylase amino acid sequence set forth in SEQ ID NO: 3 disclosed in WO 99/19467 or SEQ ID NO: 1 herein. The bacterial alpha-amylase may also have a substitution in a position corresponding to S239 in the *Bacillus licheniformis* alpha-amylase shown in SEQ ID NO: 4 in WO 99/19467, or a S242 variant of the *Bacillus stearothermophilus* alpha-amylase of SEQ ID NO: 3 in WO 99/19467 or SEQ ID NO: 1 herein.

The variant may be a S242A, E or Q variant, preferably a S242Q variant, of the *Bacillus stearothermophilus* alpha-amylase (using SEQ ID NO: 1 herein for numbering).

The variant may be a position E188 variant, preferably E188P variant of the *Bacillus stearothermophilus* alpha-amylase (using SEQ ID NO: 1 herein for numbering).

The bacterial alpha-amylase may be a truncated alpha-amylase. Especially the truncation is so that the *Bacillus stearothermophilus* alpha-amylase shown in SEQ ID NO: 3 in WO 99/19467 or SEQ ID NO: 1 herein, is around 491 amino acids long, such as from 480-495 amino acids long.

## Thermostable Alpha-Amylase

The alpha-amylase used in a process or composition of the invention has at least 80% sequence identity to SEQ ID NO: 1 and may be a thermostable alpha-amylase, such as a thermostable bacterial alpha-amylase, preferably from *Bacillus stearothermophilus.* The alpha-amylase used mayhave a T½ (min) at pH 4.5, 85°C, 0.12 mM $CaCl_2$ of at least 10.

The thermostable alpha-amylase may have a T½ (min) at pH 4.5, 85°C, 0.12 mM $CaCl_2$, of at least 15.

The thermostable alpha-amylase may have a T½ (min) at pH 4.5, 85°C, 0.12 mM $CaCl_2$, of at least 20.

The thermostable alpha-amylase may have a T½ (min) at pH 4.5, 85°C, 0.12 mM $CaCl_2$, of at least 25.

The thermostable alpha-amylase may have a T½ (min) at pH 4.5, 85°C, 0.12 mM $CaCl_2$, of at least 30.

The thermostable alpha-amylase may have a T½ (min) at pH 4.5, 85°C, 0.12 mM $CaCl_2$, of at least 40.

The thermostable alpha-amylase may have a T½ (min) at pH 4.5, 85°C, 0.12 mM $CaCl_2$, of at least 50.

The thermostable alpha-amylase may have a T½ (min) at pH 4.5, 85°C, 0.12 mM $CaCl_2$, of at least 60.

The thermostable alpha-amylase may have a T½ (min) at pH 4.5, 85°C, 0.12 mM $CaCl_2$, between 10-70.

The thermostable alpha-amylase may have a T½ (min) at pH 4.5, 85°C, 0.12 mM $CaCl_2$, between 15-70.

The thermostable alpha-amylase may have a T½ (min) at pH 4.5, 85°C, 0.12 mM $CaCl_2$, between 20-70.

The thermostable alpha-amylase may have a T½ (min) at pH 4.5, 85°C, 0.12 mM $CaCl_2$, between 25-70.

The thermostable alpha-amylase may have a T½ (min) at pH 4.5, 85°C, 0.12 mM $CaCl_2$, between 30-70.

The thermostable alpha-amylase may have a T½ (min) at pH 4.5, 85°C, 0.12 mM $CaCl_2$, between 40-70.

The thermostable alpha-amylase may have a T½ (min) at pH 4.5, 85°C, 0.12 mM $CaCl_2$, between 50-70.

The thermostable alpha-amylase may have a T½ (min) at pH 4.5, 85°C, 0.12 mM $CaCl_2$, between 60-70.

The alpha-amylase having at least 80% sequence identity to SEQ ID NO: 1 used according to the invention is a bacterial alpha-amylase, preferably derived from the genus *Bacillus,* especially a strain of *Bacillus stearothermophilus,* in particular the *Bacillus stearothermophilus* as disclosed in WO 99/19467 as SEQ ID NO: 3 (SEQ ID NO: 1 herein) with one or two amino acids deleted at positions R179, G180, I181 and/or G182, in particular with R179 and G180 deleted, or with I181 and G182 deleted, with mutations in below list of mutations.

The alpha-amylase having at least 80% sequence identity to SEQ ID NO: 1 may have double deletion I181 + G182, and optional substitution N193F, further comprising mutations selected from below list:

| |
|---|
| - V59A+Q89R+G112D+E129V+K177L+R179E+K220P+N224L+Q254S; |
| - V59A+Q89R+E129V+K177L+R179E+H208Y+K220P+N224L+Q254S; |
| - V59A+Q89R+E129V+K177L+R179E+K220P+N224L+Q254S+D269E+D281N; |
| - V59A+Q89R+E129V+K177L+R179E+K220P+N224L+Q254S+1270L; |
| - V59A+Q89R+E129V+K177L+R179E+K220P+N224L+Q254S+H274K; |
| - V59A+Q89R+E129V+K177L+R179E+K220P+N224L+Q254S+Y276F; |
| - V59A+E129V+R157Y+K177L+R179E+K220P+N224L+S242Q+Q254S; |
| - V59A+E129V+K177L+R179E+H208Y+K220P+N224L+S242Q+Q254S; |
| - V59A+E129V+K177L+R179E+K220P+N224L+S242Q+Q254S; |
| - V59A+E129V+K177L+R179E+K220P+N224L+S242Q+Q254S+H274K; |
| - V59A+E129V+K177L+R179E+K220P+N224L+S242Q+Q254S+Y276F; |
| - V59A+E129V+K177L+R179E+K220P+N224L+S242Q+Q254S+D281N; |
| - V59A+E129V+K177L+R179E+K220P+N224L+S242Q+Q254S+M284T; |
| - V59A+E129V+K177L+R179E+K220P+N224L+S242Q+Q254S+G416V; |
| - V59A+E129V+K177L+R179E+K220P+N224L+Q254S; |
| - V59A+E129V+K177L+R179E+K220P+N224L+Q254S+M284T; |
| - A91L+M961+E129V+Kl77L+Rl79E+K220P+N224L+S242Q+Q254S; |
| - E129V+K177L+R179E; |
| - E129V+K177L+R179E+K220P+N224L+S242Q+Q254S; |
| - E129V+K177L+R179E+K220P+N224L+S242Q+Q254S+Y276F+L427M; |
| - E129V+K177L+R179E+K220P+N224L+S242Q+Q254S+M284T; |
| - E129V+K177L+R179E+K220P+N224L+S242Q+Q254S+N376*+1377*; |
| - E129V+K177L+R179E+K220P+N224L+Q254S; |
| - E129V+K177L+R179E+K220P+N224L+Q254S+M284T; |
| - E129V+K177L+R179E+S242Q; |
| - E129V+K177L+R179V+K220P+N224L+S242Q+Q254S; |
| - K220P+N224L+S242Q+Q254S; |
| - M284V; |
| - V59A+Q89R+ E129V+ K177L+ R179E+ Q254S+ M284V. |

The alpha-amylase having at least 80% sequence identity to SEQ ID NO: 1 used in accordance with the invention may be selected from the group of *Bacillus stearothermphilus* alpha-amylase variants:

- I181*+G182*+N193F+E129V+K177L+R179E;
- I181*+G182*+N193F+V59A+Q89R+E129V+K177L+R179E+H208Y+K220P+N224L+Q254S;
- I181*+G182*+N193F+V59A+Q89R+E129V+K177L+R179E+Q254S+M284V; and
- I181*+G182*+N193F+E129V+K177L+R179E+K220P+N224L+S242Q+Q254S (using SEQ ID NO: 1 herein for numbering).

It should be understood that when referring to *Bacillus stearothermophilus* alpha-amylase and variants thereof they are normally produced in truncated form. In particular, the truncation may be so that the *Bacillus stearothermophilus* alpha-amylase shown in SEQ ID NO: 3 in WO 99/19467 or SEQ ID NO: 1 herein, or variants thereof, are truncated in the C-terminal and are typically around 491 amino acids long, such as from 480-495 amino acids long.

The alpha-amylase variant may be an enzyme having a degree of identity of at least 80%, at least 90%, at least 95%, at least 91%, at least 92%, at least 93%, at least 94%, at least 95%, at least 96%, at least 97%, at least 98% or at least 99%, but less than 100% to the sequence shown in SEQ ID NO: 3 in WO 99/19467 or SEQ ID NO: 1 herein.

Protease Present and/or Added During Liquefaction

A thermostable protease having at least 80% identity to SEQ ID NO: 13 and having a thermostability value of more than 20% determined as Relative Activity at 80°C/70°C is present and/or added during liquefaction together with an alpha-amylase having at least 80% sequence identity to SEQ ID NO: 1, such as a thermostable alpha-amylase, and optionally a carbohydrate-source generating enzyme, in particular a thermostable glucoamylase, and/or optionally a pullulanase.

Proteases are classified on the basis of their catalytic mechanism into the following groups: Serine proteases (S), Cysteine proteases (C), Aspartic proteases (A), Metallo proteases (M), and Unknown, or as yet unclassified, proteases (U), see Handbook of Proteolytic Enzymes, A.J.Barrett, N.D.Rawlings, J.F.Woessner (eds), Academic Press (1998), in particular the general introduction part.

Protease activity can be measured using any suitable assay, in which a substrate is employed, that includes peptide bonds relevant for the specificity of the protease in question. Assay-pH and assay-temperature are likewise to be adapted to the protease in question. Examples of assay-pH-values are pH 6, 7, 8, 9, 10, or 11. Examples of assay-temperatures are 30, 35, 37, 40, 45, 50, 55, 60, 65, 70 or 80°C.

Examples of protease substrates are casein, such as Azurine-Crosslinked Casein (AZCL-casein). Two protease assays are described below in the "Materials & Methods"-section, of which the so-called "AZCL-Casein Assay" is the preferred assay.

The thermostable protease has at least 20%, such as at least 30%, such as at least 40%, such as at least 50%, such as at least 60%, such as at least 70%, such as at least 80%, such as at least 90%, such as at least 95%, such as at least 100% of the protease activity of Protease Pfu determined by the AZCL-casein assay described in the "Materials & Methods" section.

There are no limitations on the origin of the protease used in a process of the invention as long as it has at least 80% identity to SEQ ID NO: 13 and further fulfills the thermostability properties defined below.

The thermostable protease may be derived from any bacterium as long as the protease has at least 80% identity to SEQ ID NO: 13 and further has the thermostability properties defined according to the invention.

The thermostable protease may be derived from a strain of the bacterium *Pyrococcus,* such as a strain of *Pyrococcus furiosus* (pfu protease).

The protease may be the one shown as SEQ ID NO: 1 in US patent No. 6,358,726-B1 (Takara Shuzo Company) and SEQ ID NO: 13 herein.

The thermostable protease may be the one disclosed as SEQ ID NO: 13 herein or a protease having at least 80% identity, such as at least 85%, such as at least 90%, such as at least 95%, such as at least 96%, such as at least 97%, such as at least 98%, such as at least 99% identity to SEQ ID NO: 13 herein. The *Pyroccus furiosus* protease can be purchased from Takara Bio, Japan.

The *Pyrococcus furiosus* protease is a thermostable protease according to the invention. The commercial product *Pyrococcus furiosus* protease (Pfu S) was found to have a thermostability of 110% (80°C/70°C) and 103% (90°C/70°C) at pH 4.5 determined as described in Example 2 herein.

A thermostable protease used in a process of the invention has a thermostability value of more than 20% determined as Relative Activity at 80°C/70°C determined as described in Example 2.

The protease may have a thermostability of more than 30%, more than 40%, more than 50%, more than 60%, more than 70%, more than 80%, more than 90%, more than 100%, such as more than 105%, such as more than 110%, such as more than 115%, such as more than 120% determined as Relative Activity at 80°C/70°C.

The protease may have a thermostability of between 20 and 50%, such as between 20 and 40%, such as 20 and 30% determined as Relative Activity at 80°C/70°C.

The protease may have a thermostability between 50 and 115%, such as between 50 and 70%, such as between 50 and 60%, such as between 100 and 120%, such as between 105 and 115% determined as Relative Activity at 80°C/70°C.

The protease may have a thermostability value of more than 10% determined as Relative Activity at 85°C/70°C determined as described in Example 2.

The protease may have a thermostability of more than 10%, such as more than 12%, more than 14%, more than 16%, more than 18%, more than 20%, more than 30%, more than 40%, more that 50%, more than 60%, more than 70%, more than 80%, more than 90%, more than 100%, more than 110% determined as Relative Activity at 85°C/70°C.

The protease may have a thermostability of between 10 and 50%, such as between 10 and 30%, such as between 10 and 25% determined as Relative Activity at 85°C/70°C.

The protease may have more than 20%, more than 30%, more than 40%, more than 50%, more than 60%, more than 70%, more than 80%, more than 90% determined as Remaining Activity at 80°C.

The protease may have more than 20%, more than 30%, more than 40%, more than 50%, more than 60%, more than 70%, more than 80%, more than 90% determined as Remaining Activity at 84°C.

Determination of "Relative Activity" and "Remaining Activity" is done as described in Example 2.

The protease may have a themostability for above 90, such as above 100 at 85°C as determined using the Zein-BCA assay as disclosed in Example 3.

The protease may have a themostability above 60%, such as above 90%, such as above 100%, such as above 110% at 85°C as determined using the Zein-BCA assay.

The protease may have a themostability between 60-120, such as between 70-120%, such as between 80-120%, such as between 90-120%, such as between 100-120%, such as 110-120% at 85°C as determined using the Zein-BCA assay.

The thermostable protease may have at least 20%, such as at least 30%, such as at least 40%, such as at least 50%, such as at least 60%, such as at least 70%, such as at least 80%, such as at least 90%, such as at least 95%, such as at least 100% of the activity of Protease Pfu shown as SEQ ID NO: 13 determined by the AZCL-casein assay.

Carbohydrate-Source Generating Enzyme Present and/or Added During Liquefaction

According to the invention a carbohydrate-source generating enzyme, in particular a glucoamylase, preferably a thermostable glucoamylase, may be present and/or added during liquefaction together with an alpha-amylase having at least 80% sequence identity to SEQ ID NO: 1 and a thermostable protease having at least 80% sequence identity to SEQ ID NO: 13 and having a thermostability value of more than 20% determined as Relative Activity at 80°C/70°C. As mentioned above, a pullulanase may also be present and/or added during liquefaction step i).

The term "carbohydrate-source generating enzyme" includes any enzymes generating fermentable sugars. A carbohydrate-source generating enzyme is capable of producing a carbohydrate that can be used as an energy-source by the fermenting organism(s) in question, for instance, when used in a process of the invention for producing a fermentation product, such as ethanol. The generated carbohydrates may be converted directly or indirectly to the desired fermentation product, preferably ethanol. According to the invention a mixture of carbohydrate-source generating enzymes may be used. Specific examples include glucoamylase (being glucose generators), beta-amylase and maltogenic amylase (being maltose generators).

The carbohydrate-source generating enzyme may be thermostable. The carbohydrate-source generating enzyme, in particular thermostable glucoamylase, may be added together with or separately from the alpha-amylase and the thermostable protease.

The carbohydrate-source generating enzyme, preferably a thermostable glucoamylase, may have a Relative Activity heat stability at 85°C of at least 20%, at least 30%, preferably at least 35% determined as described in Example 4 (heat stability).

The carbohydrate-source generating enzyme may be a glucoamylase having a relative activity pH optimum at pH 5.0 of at least 90%, preferably at least 95%, preferably at least 97%, such as 100% determined as described in Example 4 (pH optimum).

The carbohydrate-source generating enzyme may be a glucoamylase having a pH stability at pH 5.0 of at least at least 80%, at least 85%, at least 90% determined as described in Example 4 (pH stability).

The carbohydrate-source generating enzyme may be a thermostable glucoamylase, preferably of fungal origin, preferably a filamentous fungi, such as from a strain of the genus *Penicillium,* especially a strain of *Penicillium oxalicum,* in particular the *Penicillium oxalicum* glucoamylasedisclosed as SEQ ID NO: 2 in PCT/CN10/071753 published as WO 2011/127802 and shown in SEQ ID NO: 9 or 14 herein.

The thermostable glucoamylase may have at least 80%, more preferably at least 85%, more preferably at least 90%, more preferably at least 91%, more preferably at least 92%, even more preferably at least 93%, most preferably at least 94%, and even most preferably at least 95%, such as even at least 96%, at least 97%, at least 98%, at least 99% or 100% identity to the mature polypeptide shown in SEQ ID NO: 2 in WO 2011/127802 or SEQ ID NOs: 9 or 14 herein.

The carbohydrate-source generating enzyme, in particular thermostable glucoamylase, may be the *Penicillium oxalicum* glucoamylase.

The carbohydrate-source generating enzyme may be a variant of the *Penicillium oxalicum* glucoamylase disclosed as SEQ ID NO: 2 in WO 2011/127802 and shown in SEQ ID NO: 9 and 14 herein, having a K79V substitution (referred to as PE001) (using the mature sequence shown in SEQ ID NO: 14 for numbering). The K79V glucoamylase variant has reduced sensitivity to protease degradation relative to the parent. The thermostable glucoamylase may be a variant of the *Penicillium oxalicum* glucoamylase disclosed as SEQ ID NO: 2 in WO 2011/127802 and shown in SEQ ID NO: 9 and 14 herein. The *Penicillium oxalicum* glucoamylase may be the one disclosed as SEQ ID NO: 2 in WO 2011/127802 and shown in SEQ ID NO: 9 and 14 herein having Val (V) in position 79 (using SEQ ID NO: 14 for numbering).

These variants have reduced sensitivity to protease degradation.
These variants have improved thermostability compared to the parent.

More specifically, the glucoamylase may have a K79V substitution (using SEQ ID NO: 14 for numbering), corresponding to the PE001 variant, and further comprises at least one of the following substitutions or combination of substitutions:

T65A; or
Q327F; or
E501V; or
Y504T; or
Y504*; or
T65A + Q327F; or
T65A + E501V; or
T65A + Y504T; or
T65A + Y504*; or
Q327F + E501V; or
Q327F + Y504T; or
Q327F + Y504*; or
E501V + Y504T; or
E501V + Y504*; or
T65A + Q327F + E501V; or
T65A + Q327F + Y504T; or
T65A + E501V + Y504T; or
Q327F + E501V + Y504T; or
T65A + Q327F + Y504*; or
T65A + E501V + Y504*; or
Q327F + E501V + Y504*; or
T65A + Q327F + E501V + Y504T; or
T65A + Q327F + E501V + Y504*;
E501V + Y504T; or
T65A + K161S; or
T65A + Q405T; or
T65A + Q327W; or
T65A + Q327F; or
T65A + Q327Y; or
P11 F + T65A + Q327F; or
R1K + D3W + K5Q + G7V + N8S + T10K + P11S + T65A + Q327F; or
P2N + P4S + P11 F + T65A + Q327F; or
P11 F + D26C + K33C + T65A + Q327F; or
P2N + P4S + P11 F + T65A + Q327W + E501V + Y504T; or
R1E + D3N + P4G + G6R + G7A + N8A + T10D+ P11 D + T65A + Q327F; or
P11 F + T65A + Q327W; or
P2N + P4S + P11 F + T65A + Q327F + E501V + Y504T; or
P11 F + T65A + Q327W + E501V + Y504T; or
T65A + Q327F + E501V + Y504T; or
T65A + S105P + Q327W; or
T65A + S105P + Q327F; or
T65A + Q327W + S364P; or
T65A + Q327F + S364P; or
T65A + S103N + Q327F; or
P2N + P4S + P11 F + K34Y + T65A + Q327F; or
P2N + P4S + P11 F + T65A + Q327F + D445N + V447S; or
P2N + P4S + P11F + T65A + 1172V + Q327F; or
P2N + P4S + P11 F + T65A + Q327F + N502*; or
P2N + P4S + P11 F + T65A + Q327F + N502T + P563S + K571 E; or
P2N + P4S + P11F + R31S + K33V + T65A + Q327F + N564D + K571S; or
P2N + P4S + P11 F + T65A + Q327F + S377T; or
P2N + P4S + P11 F + T65A + V325T+ Q327W; or

P2N + P4S + P11 F + T65A + Q327F + D445N + V447S + E501V + Y504T; or
P2N + P4S + P11F + T65A + 1172V + Q327F + E501V + Y504T; or
P2N + P4S + P11 F + T65A + Q327F + S377T + E501V + Y504T; or
P2N + P4S + P11 F + D26N + K34Y + T65A + Q327F; or
P2N + P4S + P11 F + T65A + Q327F + I375A + E501V + Y504T; or
P2N + P4S + P11 F + T65A + K218A + K221 D + Q327F + E501 V + Y504T; or
P2N + P4S + P11 F + T65A + S103N + Q327F + E501V + Y504T; or
P2N + P4S + T10D + T65A + Q327F + E501V + Y504T; or
P2N + P4S + F12Y + T65A + Q327F + E501V + Y504T; or
K5A + P11 F + T65A + Q327F + E501V + Y504T; or
P2N + P4S + T10E + E18N + T65A + Q327F + E501V + Y504T; or
P2N + T10E + E18N + T65A + Q327F + E501V + Y504T; or
P2N + P4S + P11 F + T65A + Q327F + E501V + Y504T + T568N; or
P2N + P4S + P11 F + T65A + Q327F + E501V + Y504T + K524T + G526A; or
P2N + P4S + P11 F + K34Y + T65A + Q327F + D445N + V447S + E501V + Y504T; or
P2N + P4S + P11 F + R31S + K33V + T65A + Q327F + D445N + V447S + E501V + Y504T; or
P2N + P4S + P11 F + D26N + K34Y + T65A + Q327F + E501V + Y504T; or
P2N + P4S + P11 F + T65A + F80* + Q327F + E501V + Y504T; or
P2N + P4S + P11 F + T65A + K112S + Q327F + E501V + Y504T; or
P2N + P4S + P11 F + T65A + Q327F + E501V + Y504T + T516P + K524T + G526A; or
P2N + P4S + P11 F + T65A + Q327F + E501V + N502T + Y504*; or
P2N + P4S + P11 F + T65A + Q327F + E501V + Y504T; or
P2N + P4S + P11 F + T65A + S103N + Q327F + E501V + Y504T; or
K5A + P11 F + T65A + Q327F + E501V + Y504T; or
P2N + P4S + P11 F + T65A + Q327F + E501V + Y504T + T516P + K524T + G526A; or
P2N + P4S + P11 F + T65A + V79A + Q327F + E501V + Y504T; or
P2N + P4S + P11 F + T65A + V79G + Q327F + E501V + Y504T; or
P2N + P4S + P11 F + T65A + V79I + Q327F + E501V + Y504T; or
P2N + P4S + P11 F + T65A + V79L + Q327F + E501V + Y504T; or
P2N + P4S + P11 F + T65A + V79S + Q327F + E501V + Y504T; or
P2N + P4S + P11 F + T65A + L72V + Q327F + E501V + Y504T; or
S255N + Q327F + E501V + Y504T; or
P2N + P4S + P11F + T65A + E74N +Q327F + E501V + Y504T; or
P2N + P4S + P11 F + T65A + G220N + Q327F + E501V + Y504T; or
P2N + P4S + P11 F + T65A + Y245N + Q327F + E501V + Y504T; or
P2N + P4S + P11 F + T65A + Q253N + Q327F + E501V + Y504T; or
P2N + P4S + P11 F + T65A + D279N + Q327F + E501V + Y504T; or
P2N + P4S + P11 F + T65A + Q327F + S359N + E501V + Y504T; or
P2N + P4S + P11 F + T65A + Q327F + D370N + E501V + Y504T; or
P2N + P4S + P11 F + T65A + Q327F + V460S + E501V + Y504T; or
P2N + P4S + P11 F + T65A + Q327F + V460T + P468T + E501V + Y504T; or
P2N + P4S + P11 F + T65A + Q327F + T463N + E501V + Y504T; or
P2N + P4S + P11 F + T65A + Q327F + S465N + E501V + Y504T; or
P2N + P4S + P11 F + T65A + Q327F + T477N + E501V + Y504T.

The *Penicillium oxalicum* glucoamylase variant may have a K79V substitution (using SEQ ID NO: 14 for numbering), corresponding to the PE001 variant, and further comprises one of the following mutations:

P11 F + T65A + Q327F; or
P2N + P4S + P11 F + T65A + Q327F; or
P11 F + D26C + K33C + T65A + Q327F; or
P2N + P4S + P11 F + T65A + Q327W + E501V + Y504T; or
P2N + P4S + P11 F + T65A + Q327F + E501V + Y504T; or
P11 F + T65A + Q327W + E501V + Y504T.

The carbohydrate-source generating enzyme, in particular, may be added in amounts from 0.1- 100 micrograms EP/g, such as 0.5-50 micrograms EP/g, such as 1-25 micrograms EP/g, such as 2-12 micrograms EP/g DS.

Pullulanase Present and/or Added During Liquefaction

Optionally a pullulanase may be present and/or added during liquefaction step i) together with an alpha-amylase having at least 80% sequence identity to SEQ ID NO: 1 and a thermostable protease having at least 80% identity to SEQ ID NO: 13 and further having a thermostability value of more than 20% determined as Relative Activity at 80°C/70°C. As mentioned above a carbohydrate-source generating enzyme, preferably a thermostable glucoamylase, may also be present and/or added during liquefaction step i).

The pullulanase may be present and/or added during liquefaction step i) and/or saccharification step ii) or simultaneous saccharification and fermentation.

Pullulanases (E.C. 3.2.1.41, pullulan 6-glucano-hydrolase), are debranching enzymes characterized by their ability to hydrolyze the alpha-1,6-glycosidic bonds in, for example, amylopectin and pullulan.

Contemplated pullulanases include the pullulanases from *Bacillus amyloderamificans* disclosed in U.S. Patent No. 4,560,651, the pullulanase disclosed as SEQ ID NO: 2 in WO 01/151620, the *Bacillus deramificans* disclosed as SEQ ID NO: 4 in WO 01/151620, and the pullulanase from *Bacillus acidopullulyticus* disclosed as SEQ ID NO: 6 in WO 01/151620 and also described in FEMS Mic. Let. (1994) 115, 97-106.

Additional pullulanases contemplated according to the present invention included the pullulanases from *Pyrococcus woesei,* specifically from *Pyrococcus woesei* DSM No. 3773 disclosed in WO 92/02614.

The pullulanase may be a family GH57 pullulanase. The pullulanase may include an X47 domain as disclosed in US 61/289,040 published as WO 2011/087836. More specifically the pullulanase may be derived from a strain of the genus *Thermococcus,* including *Thermococcus litoralis* and *Thermococcus hydrothermalis,* such as the *Thermococcus hydrothermalis* pullulanase shown in SEQ ID NO: 11 truncated at site X4 right after the X47 domain (i.e., amino acids 1-782 in SEQ ID NOS: 11 and 12 herein). The pullulanase may also be a hybrid of the *Thermococcus litoralis* and *Thermococcus hydrothermalis* pullulanases or a T. *hydrothermalis*/*T. litoralis* hybrid enzyme with truncation site X4 disclosed in US 61/289,040 published as WO 2011/087836 and disclosed in SEQ ID NO: 12 herein.

The pullulanase may be one comprising an X46 domain disclosed in WO 2011/076123 (Novozymes).

The pullulanase may be added in an effective amount which include the preferred amount of about 0.0001-10 mg enzyme protein per gram DS, preferably 0.0001-0.10 mg enzyme protein per gram DS, more preferably 0.0001-0.010 mg enzyme protein per gram DS. Pullulanase activity may be determined as NPUN. An Assay for determination of NPUN is described in the "Materials & Methods"-section below.

Suitable commercially available pullulanase products include PROMOZYME D, PROMOZYME™ D2 (Novozymes A/S, Denmark), OPTIMAX L-300 (Genencor Int., USA), and AMANO 8 (Amano, Japan).

Carbohydrate-Source Generating Enzyme present and/or added during Saccharification and/or Fermentation

According to the invention a carbohydrate-source generating enzyme, preferably a glucoamylase, is present and/or added during saccharification and/or fermentation.

In a preferred embodiment the carbohydrate-source generating enzyme is a glucoamylase, of fungal origin, preferably from a stain of *Aspergillus,* preferably *A. niger, A. awamori*, or *A. oryzae*; or a strain of *Trichoderma,* preferably *T. reesei*; or a strain of *Talaromyces,* preferably *T. emersonii,* Glucoamylase

According to the invention the glucoamylase present and/or added during saccharification and/or fermentation may be derived from any suitable source, e.g., derived from a microorganism or a plant. Preferred glucoamylases are of fungal or bacterial origin, selected from the group consisting of *Aspergillus* glucoamylases, in particular *Aspergillus niger* G1 or G2 glucoamylase (Boel et al. (1984), EMBO J. 3 (5), p. 1097-1102), or variants thereof, such as those disclosed in WO 92/00381, WO 00/04136 and WO 01/04273 (from Novozymes, Denmark); the *A. awamori* glucoamylase disclosed in WO 84/02921, *Aspergillus oryzae* glucoamylase (Agric. Biol. Chem. (1991), 55 (4), p. 941-949), or variants or fragments thereof. Other *Aspergillus* glucoamylase variants include variants with enhanced thermal stability: G137A and G139A (Chen et al. (1996), Prot. Eng. 9, 499-505); D257E and D293E/Q (Chen et al. (1995), Prot. Eng. 8, 575-582); N182 (Chen et al. (1994), Biochem. J. 301, 275-281); disulphide bonds, A246C (Fierobe et al. (1996), Biochemistry, 35, 8698-8704; and introduction of Pro residues in position A435 and S436 (Li et al. (1997), Protein Eng. 10, 1199-1204.

Other glucoamylases include *Athelia rolfsii* (previously denoted *Corticium rolfsii*) glucoamylase (see US patent no. 4,727,026 and (Nagasaka et al. (1998) "Purification and properties of the raw-starch-degrading glucoamylases from Corticium rolfsii, Appl Microbiol Biotechnol 50:323-330), *Talaromyces* glucoamylases, in particular derived from *Talaromyces emersonii* (WO 99/28448), *Talaromyces leycettanus* (US patent no. Re. 32,153), *Talaromyces duponti, Talaromyces thermophilus* (US patent no. 4,587,215). In a preferred embodiment the glucoamylase used during saccharification and/or fermentation is the *Talaromyces emersonii* glucoamylase disclosed in WO 99/28448.

Bacterial glucoamylases contemplated include glucoamylases from the genus *Clostridium,* in particular C. *thermoamylolyticum* (EP 135,138), and C. *thermohydrosulfuricum* (WO 86/01831).

Contemplated fungal glucoamylases include *Trametes cingulata, Pachykytospora papyracea*; and *Leucopax-*

*illus giganteus* all disclosed in WO 2006/069289; and *Peniophora rufomarginata* disclosed in WO2007/124285; or a mixture thereof. Also hybrid glucoamylase are contemplated according to the invention. Examples include the hybrid glucoamylases disclosed in WO 2005/045018. Specific examples include the hybrid glucoamylase disclosed in Table 1 and 4 of Example 1.

The glucoamylase may be derived from a strain of the genus *Pycnoporus,* in particular a strain of *Pycnoporus* as described in US 61/264,977 published as WO 2011/066576 (SEQ ID NOs 2, 4 or 6), or from a strain of the genus *Gloephyllum,* in particular a strain of *Gloephyllum* as described in US 61/406,741 published as WO 2011/068803 (SEQ ID NO: 2, 4, 6, 8, 10, 12, 14 or 16) or a strain of the genus *Nigrofomes,* in particular a strain of *Nigrofomes sp.* disclosed in WO 2012/064351 (SEQ ID NO: 2). Contemplated are also glucoamylases which exhibit a high identity to any of the above-mentioned glucoamylases, i.e., at least 70%, at least 75%, at least 80%, at least 85%, at least 90%, at least 95%, at least 96%, at least 97%, at least 98%, at least 99% or even 100% identity to any one of the mature parts of the enzyme sequences mentioned above.

Glucoamylases may in an embodiment be added to the saccharification and/or fermentation in an amount of 0.0001-20 AGU/g DS, preferably 0.001-10 AGU/g DS, especially between 0.01-5 AGU/g DS, such as 0.1-2 AGU/g DS.

Commercially available compositions comprising glucoamylase include AMG 200L; AMG 300 L; SAN™ SUPER, SAN™ EXTRA L, SPIRIZYME™ PLUS, SPIRIZYME™ FUEL, SPIRIZYME™ B4U, SPIRIZYME™ ULTRA, SPIRIZYME™ EXCEL and AMG™ E (from Novozymes A/S); OPTIDEX™ 300, GC480, GC417 (from Genencor Int.); AMIGASE™ and AMIGASE™ PLUS (from DSM); G-ZYME™ G900, G-ZYME™ and G990 ZR (from Genencor Int.).

Maltogenic Amylase

The carbohydrate-source generating enzyme present and/or added during saccharification and/or fermentation may also be a maltogenic alpha-amylase. A "maltogenic alpha-amylase" (glucan 1,4-alpha-maltohydrolase, E.C. 3.2.1.133) is able to hydrolyze amylose and amylopectin to maltose in the alpha-configuration. A maltogenic amylase from *Bacillus stearothermophilus* strain NCIB 11837 is commercially available from Novozymes A/S. Maltogenic alpha-amylases are described in US Patent nos. 4,598,048, 4,604,355 and 6,162,628. The maltogenic amylase may in a preferred embodiment be added in an amount of 0.05-5 mg total protein/gram DS or 0.05-5 MANU/g DS.

Examples of Preferred Processes of the Invention

In an embodiment the invention relates to a process for producing fermentation products from starch-containing material comprising the steps of:

i) liquefying the starch-containing material at a pH in the range between from above 5.0-7.0 at a temperature above the initial gelatinization temperature using:

- an alpha-amylase having at least 80% sequence identity to SEQ ID NO: 1, preferably derived from *Bacillus stearothermophilus,* having a T½ (min) at pH 4.5, 85°C, 0.12 mM $CaCl_2$ of at least 10, at least 15, at least 20, at least 25, at least 30, at least 40, at least 50, at least 60, at least 70, such as between 10-70; such as between 15-70, such as between 20-70; such as between 25-70; such as between 30-70; such as between 40-70; such as between 50-70; such as between 60-70;
- a protease having at least 80% sequence identity to SEQ ID NO: 13, preferably derived from *Pyrococcus furiosus* having a thermostability value of more than 20%, more than 30%, more than 40%, more than 50%, more than 60%, more than 70%, more than 80%, more than 90%, more than 100%, more than 105%, more than 110%, more than 115%, more than 120%; such as between 20 and 50%, between 20 and 40%, 20 and 30%, between 50 and 115%, between 50 and 70%, between 50 and 60%, between 100 and 120%, between 105 and 115% determined as Relative Activity at 80°C/70°C determined as Relative Activity at 80°C/70°C;
- optionally a *Penicillium oxalicum* glucoamylase

ii) saccharifying using a glucoamylase enzyme;
iii) fermenting using a fermenting organism.

In another preferred embodiment the invention relates to a process for producing fermentation products from starch-containing material comprising the steps of:

i) liquefying the starch-containing material at a pH in the range between from above 5.0-6.0 at a temperature between 80-90°C using:

- an alpha-amylase having at least 80% sequence identity to SEQ ID NO: 1, preferably derived from *Bacillus stearothermophilus,* having a T½ (min) at pH 4.5, 85°C, 0.12 mM $CaCl_2$ of at least 10, at least 15, at least 20, at least 25, at least 30, at least 40, at least 50, at least 60, at least 70, such as between 10-70; such as between 15-70, such as between 20-70; such as between 25-70; such as between 30-70; such as between 40-70; such as between 50-70; such as between 60-70;
- a protease having at least 80% sequence identity to SEQ ID NO: 13, preferably derived from *Pyrococcus furiosus* having a thermostability value of more than 20%, more than 30%, more than 40%, more than 50%, more than 60%, more than 70%, more than 80%, more than 90%, more than 100%, more than 105%, more than 110%, more than 115%, more than 120%; such as between 20 and 50%, between 20 and 40%, 20 and 30%, between 50 and 115%, between 50 and 70%, between 50 and 60%, between 100 and 120%, between 105 and 115% determined as Relative Activity at 80°C/70°C determined as Relative Activity at 80°C/70°C;
- optionally a *Penicillium oxalicum* glucoamylase

ii) saccharifying using a glucoamylase enzyme;
iii) fermenting using a fermenting organism.

In another preferred embodiment the invention relates to a process for producing fermentation products from starch-containing material comprising the steps of:

i) liquefying the starch-containing material at a pH in the range between from above 5.0-7.0 at a temperature above the initial gelatinization temperature using:

- an alpha-amylase having at least 80% sequence identity to SEQ ID NO: 1 and derived from *Bacillus stearothermophilus* having a double deletion I181 + G182 and optionally substitution N193F; and optionally further one of the following set of substitutions:
- E129V+K177L+R179E;
- V59A+Q89R+E129V+K177L+R179E+H208Y+K220P+N224L+Q254S;
- E129V+K177L+R179E+K220P+N224L+S242Q+Q254S (using SEQ ID NO: 1 herein for numbering).
- a protease having at least 80% identity to SEQ ID NO: 13, preferably derived from *Pyrococcus furiosus,* having a thermostability value of more than 20% determined as Relative Activity at 80°C/70°C; and
- optionally *Penicillium oxalicum* glucoamylase in SEQ ID NO: 14 having substitutions selected from the group of:
- K79V;
- K79V+ P11 F + T65A + Q327F; or
- K79V+P2N + P4S + P11 F + T65A + Q327F; or
- K79V +P11 F + D26C + K33C + T65A + Q327F; or
- K79V +P2N + P4S + P11 F + T65A + Q327W + E501V + Y504T; or
- K79V +P2N + P4S + P11 F + T65A + Q327F + E501V + Y504T; or
- K79V +P11F + T65A + Q327W + E501V + Y504T (using SEQ ID NO: 14 for numbering);

ii) saccharifying using a glucoamylase enzyme;
iii) fermenting using a fermenting organism.

In another preferred embodiment the process for producing fermentation products from starch-containing material comprises the steps of:

i) liquefying the starch-containing material at a pH in the range between from above 5.0-6.0 at a temperature between 80-90°C using:

- an alpha-amylase having at least 80% sequence identity to SEQ ID NO: 1 and derived from *Bacillus stearothermophilus* having a double deletion I181 + G182 and optional substitution N193F; and optionally further one of the following set of substitutions:
- E129V+K177L+R179E;
- V59A+Q89R+E129V+K177L+R179E+H208Y+K220P+N224L+Q254S;
- E129V+K177L+R179E+K220P+N224L+S242Q+Q254S (using SEQ ID NO: 1 herein for numbering).
- a protease having at least 80% sequence identity to SEQ ID NO: 13, preferably derived from *Pyrococcus furiosus* and/or *Thermoascus aurantiacus,* having a thermostability value of more than 20% determined as Relative Activity at 80°C/70°C; and
- optionally a *Penicillium oxalicum* glucoamylase in SEQ ID NO: 14 having substitutions selected from the group of:

- K79V;
- K79V+ P11F+T65A+Q327F; or
- K79V+P2N+P4S + P11F+T65A+Q327F; or
- K79V +P11 F+D26C + K33C+T65A+Q327F; or
- K79V +P2N+P4S+P11F+T65A+Q327W+E501V+Y504T; or
- K79V +P2N+P4S+P11F+T65A+Q327F+E501V+Y504T; or
- K79V +P11F+T65A+Q327W+E501V+Y504T (using SEQ ID NO: 14 for numbering);

ii) saccharifying using a glucoamylase enzyme;
iii) fermenting using a fermenting organism.

The alpha-amylase mentioned above derived from *Bacillus stearothermophilus* (SEQ ID NO: 1 herein), or a variant thereof, is the mature alpha-amylase or corresponding mature alpha-amylases having at least 80% identity, at least 90% identity, at least 95% identity at least 96% identity at least 97% identity at least 99% identity to the SEQ ID NO: 1.

The protease mentioned above, derived from *Pyrococcus furiosus* (SEQ ID NO: 13) is the mature protease or corresponding mature proteases having at least 80% identity, at least 90% identity, at least 95% identity at least 96% identity at least 97% identity at least 99% identity to the SEQ ID NO: 13.

The glucoamylase mentioned above derived from *Penicillium oxalicum* (SEQ ID NO: 14 herein), or a variant thereof, is the mature glucoamylase or corresponding mature glucoamylase having at least 80% identity, at least 90% identity, at least 95% identity at least 96% identity at least 97% identity at least 99% identity to the SEQ ID NO: 14 herein.

A Composition Comprising Alpha-Amylase and Thermostable Protease

A composition of the invention comprises an alpha-amylase having at least 80% sequence identity to SEQ ID NO: 1, such as a thermostable alpha-amylase, and a thermostable protease a protease having at least 80% sequence identity to SEQ ID NO: 13 and having a thermostability value of more than 20% determined as Relative Activity at 80°C/70°C. The composition may also further comprise a thermostable carbohydrate-source generating enzyme, in particular a glucoamylase, and/or optionally a pullulanase too.

Therefore, in this aspect the invention relates to composition comprising:

i) an alpha-amylase having at least 80% sequence identity to SEQ ID NO: 1;
ii) a protease having at least 80% identity to SEQ ID NO: 13, preferably derived from *Pyrococcus furiosus* having a thermostability value of more than 20% determined as Relative Activity at 80°C/70°C; and
iii) optionally a carbohydrate-source generating enzyme.

Alpha-amylase: The alpha-amylase may be any alpha-amylase having at least 80% sequence identity to SEQ ID NO: 1, such as bacterial alpha-amylases, such as alpha-amylases derived from the genus *Bacillus,* such as *Bacillus stearomthermphilus.*

The alpha-amylase having at least 80% sequence identity to SEQ ID NO: 1 may be a thermostable alpha-amylase. The thermostable alpha-amylase may have a T½ (min) at pH 4.5, 85°C, 0.12 mM $CaCl_2$) of at least 10, such as at least 15, such as at least 20, such as at least 25, such as at least 30, such as at least 40, such as at least 50, such as at least 60, such as between 10-70, such as between 15-70, such as between 20-70, such as between 25-70, such as between 30-70, such as between 40-70, such as between 50-70, such as between 60-70.

The alpha-amylase having at least 80% sequence identity to SEQ ID NO: 1 may be selected from the group of *Bacillus stearomthermphilus* alpha-amylase variants, in particular truncated to be 491 amino acids long, such as from 480 to 495 amino acids long, with mutations selected from the group of:

- I181*+G182*+N193F+E129V+K177L+R179E;
- I181*+G182*+N193F+V59A+Q89R+E129V+K177L+R179E+H208Y+K220P+N224L+Q254S;
- I181*+G182*+N193F +V59A Q89R+ E129V+ K177L+ R179E+ Q254S+ M284V; and
- I181*+G182*+N193F+E129V+K177L+R179E+K220P+N224L+S242Q+Q254S (using SEQ ID NO: 1 herein for numbering).

It should be understood that these alpha-amylases are only specific examples. Any alpha-amylase disclosed above in the "Alpha-Amylase Present and/or Added During Liquefaction"-section above may be used as the alpha-amylase component in a composition of the invention, as long as it has at least 80% sequence identity to SEQ ID NO: 1 Protease: A composition of the invention comprises a thermostable protease having at least 80% identity to SEQ ID NO: 13 and further having a thermostability value of more than 20% determined as Relative Activity at 80°C/70°C.

There is no limitation on the origin of the protease component as long as it fulfills the sequence and thermostability properties defined herein.

The protease may be derived from a strain of *Pyrococcus furiosus,* such as the one shown in SEQ ID NO: 1 in US 6,358,726 or SEQ ID NO: 13 herein.

It should be understood that these proteases are only examples. Any protease disclosed above in the "Protease Present and/or Added During Liquefaction" section above may be used as the protease component in a composition of the invention, as long as it has at least 80% sequence identity to DEQ ID NO: 13 and the required thermostability value. Carbohydrate-source generating enzymes: A composition of the invention may further comprise a carbohydrate-source generating enzyme, in particular a glucoamylase, which has a heat stability at 85°C, pH 5.3, of at least 30%, preferably at least 35%.

Said carbohydrate-source generating enzyme may be a thermostable glucoamylase having a Relative Activity heat stability at 85°C of at least 20%, at least 30%, preferably at least 35% determined as described in Example 4 (Heat stability).

The carbohydrate-source generating enzyme may be a glucoamylase having a relative activity pH optimum at pH 5.0 of at least 90%, preferably at least 95%, preferably at least 97%, such as 100% determined as described in Example 4 (pH optimum).

The carbohydrate-source generating enzyme may be a glucoamylase having a pH stability at pH 5.0 of at least at least 80%, at least 85%, at least 90% determined as described in Example 4 (pH stability).

The carbohydrate-source generating enzyme may be a thermostable glucoamylase, preferably of fungal origin, preferably a filamentous fungi, such as from a strain of the genus *Penicillium,* especially a strain of *Penicillium oxalicum* disclosed as SEQ ID NO: 2 in PCT/CN10/071753 published as WO 2011/127802, or a variant thereof, and shown in SEQ ID NO: 9 or 14 herein.

The glucoamylase, or a variant thereof, may have at least 80%, more preferably at least 85%, more preferably at least 90%, more preferably at least 91%, more preferably at least 92%, even more preferably at least 93%, most preferably at least 94%, and even most preferably at least 95%, such as even at least 96%, at least 97%, at least 98%, at least 99% or 100% identity to the mature polypeptide shown in SEQ ID NO: 2 in WO 2011/127802 or SEQ ID NO: 9 or 14 herein.

The carbohydrate-source generating enzyme may be a variant of the *Penicillium oxalicum* glucoamylase disclosed as SEQ ID NO: 2 in WO 2011/127802 and shown in SEQ ID NO: 9 and 14 herein, having a K79V substitution (using the mature sequence shown in SEQ ID NO: 14 for numbering). The K79V glucoamylase variant has reduced sensitivity to protease degradation relative to the parent. Examples of suitable thermostable *Penicillium oxalicum* glucoamylase variants are listed above and in Examples 17 and 18 below.

The carbohydrate-source generating enzyme may have pullulanase side activity.

It should be understood that these carbohydrate-source generating enzymes, in particular glucoamylases, are only examples. Any carbohydrate-source generating enzyme disclosed above in the "Carbohydrate-source generating enzyme Present and/or Added During Liquefaction" section above may be used as component in a composition of the invention. Pullulanase: A composition of the invention may further comprise a pullulanase. The pullulanase may be a family GH57 pullulanase. The pullulanase may include an X47 domain as disclosed in US 61/289,040 published as WO 2011/087836.

Specifically the pullulanase may be derived from a strain from the genus *Thermococcus,* including *Thermococcus litoralis* and *Thermococcus hydrothermalis* or a hybrid thereof.

The pullulanase may be *Thermococcus hydrothermalis* pullulanase truncated at site X4 or a *Thermococcus hydrothermalis*/*T. litoralis* hybrid enzyme with truncation site X4 as disclosed in US 61/289,040 published as WO 2011/087836.

The pullulanase may be one comprising an X46 domain disclosed in WO 2011/076123(Novozymes).

It should be understood that these pullulanases are only specific examples. Any pullulanase disclosed above in the "Pullulanase Present and/or Added During Liquefaction" section above may be used as the optional pullulanase component in a composition of the invention.

In a preferred embodiment the composition of the invention comprises

- an alpha-amylase having at least 80% sequence identity to SEQ ID NO: 1 and derived from *Bacillus stearothermophilus*;
- a protease having at least 80% identity to SEQ ID NO: 13, preferably derived from *Pyrococcus furiosus* and having a thermostability value of more than 20% determined as Relative Activity at 80°C/70°C; and
- optionally a glucoamylase derived from *Penicillium oxalicum.*

In a preferred embodiment the composition of the invention comprises

- an alpha-amylase having at least 80% sequence identity to SEQ ID NO: 1, preferably derived from *Bacillus stearothermophilus,* having a T½ (min) at pH 4.5, 85°C, 0.12 mM $CaCl_2$ of at least 10;
- a protease having at least 80% identity to SEQ ID NO: 13, preferably derived from *Pyrococcus furiosus,* having a thermostability value of more than 20% determined as Relative Activity at 80°C/70°C;
- optionally a glucoamylase derived from *Penicillium oxalicum.*

In a preferred embodiment the composition comprises

- an alpha-amylase having at least 80% sequence identity to SEQ ID NO: 1 and derived from *Bacillus stearothermophilus* having a double deletion I181 + G182 and optionally substitution N193F; and optionally further one of the following set of substitutions:
- E129V+K177L+R179E;
- V59A+Q89R+E129V+K177L+R179E+H208Y+K220P+N224L+Q254S;
- E129V+K177L+R179E+K220P+N224L+S242Q+Q254S (using SEQ ID NO: 1 herein for numbering).
- a protease having at least 80% identity to SEQ ID NO: 13, preferably derived from *Pyrococcus furiosus* and having a thermostability value of more than 20% determined as Relative Activity at 80°C/70°C; and
- optionally a *Penicillium oxalicum* glucoamylase in SEQ ID NO: 14 having substitutions selected from the group of:

  - K79V;
  - K79V+P11F+T65A+Q327F; or
  - K79V+P2N+P4S+P11F+T65A+Q327F; or
  - K79V+P11F+D26C+K33C+T65A+Q327F; or
  - K79V+P2N+P4S+P11F+T65A+Q327W+E501V+Y504T; or
  - K79V+P2N+P4S+P11F+T65A+Q327F+E501V+Y504T; or
  - K79V+P11F+T65A+Q327W+E501V+Y504T (using SEQ ID NO: 14 for numbering);

The *Bacillus stearothermophilus* alpha-amylase (SEQ ID NO: 1 herein), or a variant thereof, may be the mature alpha-amylase or corresponding mature alpha-amylase having at least 80% identity, at least 90% identity, at least 95% identity at least 96% identity at least 97% identity at least 99% identity to the SEQ ID NO: 1.

The *Pyrococcus furiosus* protease (SEQ ID NO: 13)may be the mature protease or corresponding mature protease having at least 80% identity, at least 90% identity, at least 95% identity at least 96% identity at least 97% identity at least 99% identity to SEQ ID NO: 13.

The *Penicillium oxalicum* glucoamylase (SEQ ID NO: 14 herein), or a variant thereof, may be the mature glucoamylase or corresponding mature glucoamylase having at least 80% identity, at least 90% identity, at least 95% identity at least 96% identity at least 97% identity at least 99% identity to the SEQ ID NO: 14 herein.

The carbohydrate-source generating enzyme, in particular glucoamylase, may be a *Penicillium oxalicum* glucoamylase. The glucoamylase may optionally be substituted or combined with a pullulanase, as described above in the "Pullulanase"-section, preferably derived from *Thermococcus litoralis* or *Thermococcus hydrothermalis.*

## Materials & Methods

### Materials:

Alpha-Amylase A (AAA): *Bacillus stearothermophilus* alpha-amylase with the mutations I181*+G182*+N193F truncated to 491 amino acids (SEQ ID NO: 1)

Alpha-Amylase 1407 (AA1407): *Bacillus stearothermophilus* alpha-amylase with the mutations I181*+G182*+N193F+V59A+Q89R+E129V+K177L+R179E+H208Y+K220P+N224L+Q254S truncated to 491 amino acids (SEQ ID NO: 1)

Alpha-Amylase 369 (AA369): Bacillus stearothermophilus alpha-amylase with the mutations: I181*+G182*+N193F+V59A+Q89R+E129V+K177L+R179E+Q254S+M284V truncated to 491 amino acids (SEQ ID NO: 1).

Protease WT: Metallo protease derived from *Thermoascus aurantiacus* CGMCC No. 0670 disclosed as amino acids 1-177 in SEQ ID NO: 3 herein and amino acids 1-177 in SEQ ID NO: 2 in WO 2003/048353

Protease 036: Metallo protease derived from *Thermoascus aurantiacus* CGMCC No. 0670 disclosed as amino acids 1-177 in SEQ ID NO: 3 herein and amino acids 1-177 in SEQ ID NO: 2 in WO 2003/048353 with the following mutations: D079L+S87P+0142L.

Protease 050: Metallo protease derived from *Thermoascus aurantiacus* CGMCC No. 0670 disclosed as amino acids 1-177 in SEQ ID NO: 3 herein and amino acids 1-177 in SEQ ID NO: 2 in WO 2003/048353 with the following mutations: D79L+S87P+ A112P+ D142L.
Protease 196: Metallo protease derived from *Thermoascus aurantiacus* CGMCC No. 0670 disclosed as amino acids 1-177 in SEQ ID NO: 3 herein and amino acids 1-177 in SEQ ID NO: 2 in WO 2003/048353 with the following mutations: A27K+D79L+Y82F+S87G+D104P+A112P+A126V+D142L.
Protease Pfu: Protease derived from *Pyrococcus furiosus* purchased from Takara Bio (Japan) as Pfu Protease S (activity 10.5 mg/mL) and also shown in SEQ ID NO: 13 herein.
Glucoamylase PO: Mature part of the *Penicillium oxalicum* glucoamylase disclosed as SEQ ID NO: 2 in PCT/CN10/071753 published as WO 2011/127802 and shown in SEQ ID NO: 9 herein.
Glucoamylase PE001: Variant of the *Penicillium oxalicum* glucoamylase having a K79V substitution using the mature sequence shown in SEQ ID NO: 14 for numbering.
Glucoamylase 493 (GA493): Variant of *Penicillium oxalicum* glucoamylase variant PE001 further having the following mutations: P11F+ T65A+ Q327F (using SEQ ID NO: 14 for numbering).
Glucoamylase BL: Blend of *Talaromyces emersonii* glucoamylase disclosed in WO 99/28448 as SEQ ID NO: 7 and *Trametes cingulata* glucoamylase disclosed in WO 06/069289 in a ratio of about 9:1.
Glucoamylase BL2: Blend comprising *Talaromyces emersonii* glucoamylase disclosed in WO99/28448, *Trametes cingulata* glucoamylase disclosed in WO 06/69289, and *Rhizomucor pusillus* alpha-amylase with *Aspergillus niger* glucoamylase linker and SBD disclosed as V039 in Table 5 in WO 2006/069290 as side activities (ratio about 65:15:1).
Yeast: RED STAR ETHANOL RED™ available from Red Star/Lesaffre, USA.
Substrate in Examples 6 and 20: Ground corn and backset was obtained from commercial plants in the USA.

**Methods**

**Identity:** The relatedness between two amino acid sequences or between two nucleotide sequences is described by the parameter "identity".

For purposes of the present invention the degree of identity between two amino acid sequences, as well as the degree of identity between two nucleotide sequences, may be determined by the program "align" which is a Needleman-Wunsch alignment (i.e. a global alignment). The program is used for alignment of polypeptide, as well as nucleotide sequences. The default scoring matrix BLOSUM50 is used for polypeptide alignments, and the default identity matrix is used for nucleotide alignments. The penalty for the first residue of a gap is -12 for polypeptides and -16 for nucleotides. The penalties for further residues of a gap are -2 for polypeptides, and -4 for nucleotides.

"Align" is part of the FASTA package version v20u6 (see W. R. Pearson and D. J. Lipman (1988), "Improved Tools for Biological Sequence Analysis", PNAS 85:2444-2448, and W. R. Pearson (1990) "Rapid and Sensitive Sequence Comparison with FASTP and FASTA," Methods in Enzymology 183:63- 98). FASTA protein alignments use the Smith-Waterman algorithm with no limitation on gap size (see "Smith-Waterman algorithm", T. F. Smith and M. S. Waterman (1981) J. Mol. Biol. 147:195-197).

**Protease assays**

AZCL-casein assay

A solution of 0.2% of the blue substrate AZCL-casein is suspended in Borax/$NaH_2PO_4$ buffer pH9 while stirring. The solution is distributed while stirring to microtiter plate (100 microL to each well), 30 microL enzyme sample is added and the plates are incubated in an Eppendorf Thermomixer for 30 minutes at 45°C and 600 rpm. Denatured enzyme sample (100°C boiling for 20min) is used as a blank. After incubation the reaction is stopped by transferring the microtiter plate onto ice and the coloured solution is separated from the solid by centrifugation at 3000rpm for 5 minutes at 4°C. 60 microL of supernatant is transferred to a microtiter plate and the absorbance at 595nm is measured using a BioRad Microplate Reader.

pNA-assay

50 microL protease-containing sample is added to a microtiter plate and the assay is started by adding 100 microL 1 mM pNA substrate (5 mg dissolved in 100 microL DMSO and further diluted to 10 mL with Borax/$NaH_2PO_4$ buffer pH 9.0). The increase in $OD_{405}$ at room temperature is monitored as a measure of the protease activity.

**Glucoamylase activity (AGU)**

Glucoamylase activity may be measured in Glucoamylase Units (AGU).

The Novo Glucoamylase Unit (AGU) is defined as the amount of enzyme, which hydrolyzes 1 micromole maltose per minute under the standard conditions 37°C, pH 4.3, substrate: maltose 23.2 mM, buffer: acetate 0.1 M, reaction time 5 minutes.

An autoanalyzer system may be used. Mutarotase is added to the glucose dehydrogenase reagent so that any alpha-D-glucose present is turned into beta-D-glucose. Glucose dehydrogenase reacts specifically with beta-D-glucose in the reaction mentioned above, forming NADH which is determined using a photometer at 340 nm as a measure of the original glucose concentration.

| AMG incubation: | |
|---|---|
| Substrate: | maltose 23.2 mM |
| Buffer: | acetate 0.1 M |
| pH: | 4.30 ± 0.05 |
| Incubation temperature: | 37°C ± 1 |
| Reaction time: | 5 minutes |
| Enzyme working range: | 0.5-4.0 AGU/mL |

| Color reaction: | |
|---|---|
| GlucDH: | 430 U/L |
| Mutarotase: | 9 U/L |
| NAD: | 0.21 mM |
| Buffer: | phosphate 0.12 M; 0.15 M NaCl |
| pH: | 7.60 ± 0.05 |
| Incubation temperature: | 37°C ± 1 |
| Reaction time: | 5 minutes |
| Wavelength: | 340 nm |

A folder (EB-SM-0131.02/01) describing this analytical method in more detail is available on request from Novozymes A/S, Denmark.

**Alpha-amylase activity (KNU)**

The alpha-amylase activity may be determined using potato starch as substrate. This method is based on the break-down of modified potato starch by the enzyme, and the reaction is followed by mixing samples of the starch/enzyme solution with an iodine solution. Initially, a blackish-blue color is formed, but during the break-down of the starch the blue color gets weaker and gradually turns into a reddish-brown, which is compared to a colored glass standard.

One Kilo Novo alpha amylase Unit (KNU) is defined as the amount of enzyme which, under standard conditions (i.e., at 37°C +/- 0.05; 0.0003 M $Ca^{2+}$; and pH 5.6) dextrinizes 5260 mg starch dry substance Merck Amylum solubile.

A folder EB-SM-0009.02/01 describing this analytical method in more detail is available upon request to Novozymes A/S, Denmark.

**Determination of Pullulanase Activity (NPUN)**

Endo-pullulanase activity in NPUN is measured relative to a Novozymes pullulanase standard. One pullulanase unit (NPUN) is defined as the amount of enzyme that releases 1 micro mol glucose per minute under the standard conditions (0.7% red pullulan (Megazyme), pH 5, 40°C, 20 minutes). The activity is measured in NPUN/ml using red pullulan.

1 mL diluted sample or standard is incubated at 40°C for 2 minutes. 0.5 mL 2% red pullulan, 0.5 M KCl, 50 mM citric acid, pH 5 are added and mixed. The tubes are incubated at 40°C for 20 minutes and stopped by adding 2.5 ml 80% ethanol. The tubes are left standing at room temperature for 10-60 minutes followed by centrifugation 10 minutes at 4000 rpm. OD of the supernatants is then measured at 510 nm and the activity calculated using a standard curve.

## EXAMPLES

### Example 1

Stability of Alpha-Amylase Variants

The stability of a reference alpha-amylase (*Bacillus stearothermophilus* alpha-amylase with the mutations I181*+G182*+N193F truncated to 491 amino acids (SEQ ID NO: 1 numbering)) and alpha-amylase variants thereof was determined by incubating the reference alpha-amylase and variants at pH 4.5 and 5.5 and temperatures of 75°C and 85°C with 0.12 mM $CaCl_2$ followed by residual activity determination using the EnzChek® substrate (EnzChek® Ultra Amylase assay kit, E33651, Molecular Probes).

Purified enzyme samples were diluted to working concentrations of 0.5 and 1 or 5 and 10 ppm (micrograms/ml) in enzyme dilution buffer (10 mM acetate, 0.01% Triton X100, 0.12 mM $CaCl_2$, pH 5.0). Twenty microliters enzyme sample was transferred to 48-well PCR MTP and 180 microliters stability buffer (150 mM acetate, 150 mM MES, 0.01% Triton X100, 0.12 mM $CaCl_2$, pH 4.5 or 5.5) was added to each well and mixed. The assay was performed using two concentrations of enzyme in duplicates. Before incubation at 75°C or 85°C, 20 microliters was withdrawn and stored on ice as control samples. Incubation was performed in a PCR machine at 75°C and 85°C. After incubation samples were diluted to 15 ng/mL in residual activity buffer (100 mM Acetate, 0.01% Triton X100, 0.12 mM $CaCl_2$, pH 5.5) and 25 microliters diluted enzyme was transferred to black 384-MTP. Residual activity was determined using the EnzChek substrate by adding 25 microliters substrate solution (100 micrograms/ml) to each well. Fluorescence was determined every minute for 15 minutes using excitation filter at 485-P nm and emission filter at 555 nm (fluorescence reader is Polarstar, BMG). The residual activity was normalized to control samples for each setup.

Assuming logarithmic decay half life time (T½ (min)) was calculated using the equation: T½ (min) = T(min)*LN(0.5)/LN(%RA/100), where T is assay incubation time in minutes, and %RA is % residual activity determined in assay.

Using this assay setup the half life time was determined for the reference alpha-amylase and variant thereof as shown in Table 1.

Table 1

| **Mutations** | **T½ (min) (pH 4.5, 75°C, 0.12 mM $CaCl_2$)** | **T½ (min) (pH 4.5, 85°C, 0.12 mM $CaCl_2$)** | **T½ (min) (pH 5.5, 85°C, 0.12 mM $CaCl_2$)** |
|---|---|---|---|
| Reference Alpha-Amylase A | 21 | 4 | 111 |
| Reference Alpha-Amylase A with the substitution V59A | 32 | 6 | 301 |
| Reference Alpha-Amylase A with the substitution V59E | 28 | 5 | 230 |
| Reference Alpha-Amylase A with the substitution V59I | 28 | 5 | 210 |
| Reference Alpha-Amylase A with the substitution V59Q | 30 | 6 | 250 |
| Reference Alpha-Amylase A with the substitutions V59A+Q89R+ G112D+E129V+K177L+R179E+ K220P+N224L+Q254S | 149 | 22 | ND |
| Reference Alpha-Amylase A with the substitutions V59A+Q89R+E129V+ K177L+R179E+H208Y+K220P+ | >180 | 28 | ND |
| N224L+Q254S | | | |
| Reference Alpha-Amylase A with the substitutions V59A+Q89R+E129V+ K177L+R179E+K220P+N224L+ Q254S+D269E+D281N | 112 | 16 | ND |

(continued)

| Mutations | T½ (min) (pH 4.5, 75°C, 0.12 mM $CaCl_2$) | T½ (min) (pH 4.5, 85°C, 0.12 mM $CaCl_2$) | T½ (min) (pH 5.5, 85°C, 0.12 mM $CaCl_2$) |
|---|---|---|---|
| Reference Alpha-Amylase A with the substitutions V59A+Q89R+E129V+ K177L+R179E+K220P+N224L+ Q254S+I270L | 168 | 21 | ND |
| Reference Alpha-Amylase A with the substitutions V59A+Q89R+E129V+ K177L+R179E+K220P+N224L+ Q254S+H274K | >180 | 24 | ND |
| Reference Alpha-Amylase A with the substitutions V59A+Q89R+E129V+ K177L+R179E+K220P+N224L+ Q254S+Y276F | 91 | 15 | ND |
| Reference Alpha-Amylase A with the substitutions V59A+E129V+ R157Y+K177L+R179E+K220P+ N224L+S242Q+Q254S | 141 | 41 | ND |
| Reference Alpha-Amylase A with the substitutions V59A+E129V+ K177L+R179E+H208Y+K220P+ N224L+S242Q+Q254S | >180 | 62 | ND |
| Reference Alpha-Amylase A with the substitutions V59A+E129V+ K177L+R179E+K220P+N224L+ S242Q+Q254S | >180 | 49 | >480 |
| Reference Alpha-Amylase A with the substitutions V59A+E129V+ K177L+R179E+K220P+N224L+ S242Q+Q254S+H274K | >180 | 53 | ND |
| Reference Alpha-Amylase A with the substitutions V59A+E129V+ K177L+R179E+K220P+N224L+ S242Q+Q254S+Y276F | >180 | 57 | ND |
| Reference Alpha-Amylase A with the substitutions V59A+E129V+ K177L+R179E+K220P+N224L+ S242Q+Q254S+D281 N | >180 | 37 | ND |
| Reference Alpha-Amylase A with the substitutions V59A+E129V+ K177L+R179E+K220P+N224L+ S242Q+Q254S+M284T | >180 | 51 | ND |
| Reference Alpha-Amylase A with the substitutions V59A+E129V+ K177L+R179E+K220P+N224L+ S242Q+Q254S+G416V | >180 | 45 | ND |
| Reference Alpha-Amylase A with the substitutions V59A+E129V+ K177L+R179E+K220P+N224L+ Q254S | 143 | 21 | >480 |
| Reference Alpha-Amylase A with the substitutions V59A+E129V+ K177L+R179E+K220P+N224L+ Q254S+M284T | >180 | 22 | ND |
| Reference Alpha-Amylase A with the substitutions A91L+M961+E129V+ K177L+R179E+K220P+N224L+ S242Q+Q254S | >180 | 38 | ND |
| Reference Alpha-Amylase A with the substitutions E129V+K177L+ R179E | 57 | 11 | 402 |

(continued)

| Mutations | T½ (min) (pH 4.5, 75°C, 0.12 mM $CaCl_2$) | T½ (min) (pH 4.5, 85°C, 0.12 mM $CaCl_2$) | T½ (min) (pH 5.5, 85°C, 0.12 mM $CaCl_2$) |
|---|---|---|---|
| Reference Alpha-Amylase A with the substitutions E129V+K177L+ R179E+K220P+N224L+S242Q+ Q254S | 174 | 44 | >480 |
| Reference Alpha-Amylase A with the substitutions E129V+K177L+ R179E+K220P+N224L+S242Q+ Q254S+Y276F+L427M | >180 | 49 | >480 |
| Reference Alpha-Amylase A with the substitutions E129V+K177L+ R179E+K220P+N224L+S242Q+ Q254S+M284T | >180 | 49 | >480 |
| Reference Alpha-Amylase A with the substitutions E129V+K177L+ R179E+K220P+N224L+S242Q+ Q254S+N376*+1377* | 177 | 36 | >480 |
| Reference Alpha-Amylase A with the substitutions E129V+K177L+ R179E+K220P+N224L+Q254S | 94 | 13 | >480 |
| Reference Alpha-Amylase A with the substitutions E129V+K177L+ R179E+K220P+N224L+Q254S+ M284T | 129 | 24 | >480 |
| Reference Alpha-Amylase A with the substitutions E129V+K177L+ R179E+S242Q | 148 | 30 | >480 |
| Reference Alpha-Amylase A with the substitutions E129V+K177L+ R179V | 78 | 9 | >480 |
| Reference Alpha-Amylase A with | 178 | 31 | >480 |
| the substitutions E129V+K177L+ R179V+K220P+N224L+S242Q+ Q254S | | | |
| Reference Alpha-Amylase A with the substitutions K220P+N224L+ S242Q+Q254S | 66 | 17 | >480 |
| Reference Alpha-Amylase A with the substitutions K220P+N224L+ Q254S | 30 | 6 | 159 |
| Reference Alpha-Amylase A with the substitution M284T | 35 | 7 | 278 |
| Reference Alpha-Amylase A with the substitutions M284V | 59 | 13 | ND |
| ND not determined | | | |

The results demonstrate that the alpha-amylase variants have a significantly greater half-life and stability than the reference alpha-amylase.

**Example 2**

Preparation of Protease Variants and Test of Thermostability

**Strains and plasmids**

*E. coli* DH12S (available from Gibco BRL) was used for yeast plasmid rescue. pJTP000 is a *S. cerevisiae* and *E. coli* shuttle vector under the control of TPI promoter, constructed from pJC039 described in WO 01/92502, in which the *Thermoascus aurantiacus* M35 protease gene (WO 03/048353) has been inserted.

*Saccharomyces cerevisiae* YNG318 competent cells: MATa Dpep4[cir+] ura3-52, leu2-D2, his 4-539 was used for protease variants expression. It is described in J. Biol. Chem. 272 (15), pp 9720-9727, 1997.

**Media and substrates**

10X Basal solution: Yeast nitrogen base w/o amino acids (DIFCO) 66.8 g/l, succinate 100 g/l, NaOH 60 g/l.
SC-glucose: 20 % glucose (i.e., a final concentration of 2 % = 2 g/100 ml)) 100 ml/l, 5% threonine 4 ml/l, 1 % tryptophan10 ml/l, 20 % casamino acids 25 ml/l, 10 X basal solution 100 ml/l. The solution is sterilized using a filter of a pore size of 0.20 micrometer. Agar (2%) and $H_2O$ (approx. 761 ml) is autoclaved together, and the separately sterilized SC-glucose solution is added to the agar solution.
YPD: Bacto peptone 20 g/l, yeast extract 10 g/l, 20 % glucose 100 ml/l.
YPD+Zn: YPD+0.25 mM $ZnSO_4$.
PEG/LiAc solution: 40 % PEG4000 50 ml, 5 M Lithium Acetate 1 ml.
96 well Zein micro titre plate:

Each well contains 200 microL of 0.05-0.1 % of zein (Sigma), 0.25 mM $ZnSO_4$ and 1 % of agar in 20 mM sodium acetate buffer, pH 4.5.

**DNA manipulations**

Unless otherwise stated, DNA manipulations and transformations were performed using standard methods of molecular biology as described in Sambrook et al. (1989) Molecular cloning: A laboratory manual, Cold Spring Harbor lab. Cold Spring Harbor, NY; Ausubel, F. M. et al. (eds.) "Current protocols in Molecular Biology", John Wiley and Sons, 1995; Harwood, C. R. and Cutting, S. M. (Eds.).

**Yeast transformation**

Yeast transformation was performed using the lithium acetate method. 0.5 microL of vector (digested by restriction endnucleases) and 1 microL of PCR fragments is mixed. The DNA mixture, 100 microL of YNG318 competent cells, and 10 microL of YEAST MAKER carrier DNA (Clontech) is added to a 12 ml polypropylene tube (Falcon 2059). Add 0.6 ml PEG/LiAc solution and mix gently. Incubate for 30 min at 30°C, and 200 rpm followed by 30 min at 42°C (heat shock). Transfer to an eppendorf tube and centrifuge for 5 sec. Remove the supernatant and resolve in 3 ml of YPD. Incubate the cell suspension for 45 min at 200 rpm at 30°C. Pour the suspension to SC-glucose plates and incubate 30°C for 3 days to grow colonies. Yeast total DNA are extracted by Zymoprep Yeast Plasmid Miniprep Kit (ZYMO research).

**DNA sequencing**

*E. coli* transformation for DNA sequencing was carried out by electroporation (BIO-RAD Gene Pulser). DNA Plasmids were prepared by alkaline method (Molecular Cloning, Cold Spring Harbor) or with the Qiagen® Plasmid Kit. DNA fragments were recovered from agarose gel by the Qiagen gel extraction Kit. PCR was performed using a PTC-200 DNA Engine. The ABI PRISMTM 310 Genetic Analyzer was used for determination of all DNA sequences.

**Construction of protease expression vector**

The *Themoascus* M35 protease gene was amplified with the primer pair Prot F (SEQ ID NO: 4) and Prot R (SEQ ID NO: 45). The resulting PCR fragments were introduced into S. *cerevisiae* YNG318 together with the pJC039 vector (described in WO2001/92502) digested with restriction enzymes to remove the Humicola insolens cutinase gene.

The Plasmid in yeast clones on SC-glucose plates was recovered to confirm the internal sequence and termed as pJTP001.

**Construction of yeast library and site-directed variants**

Library in yeast and site-directed variants were constructed by SOE PCR method (Splicing by Overlap Extension, see "PCR: A practical approach", p. 207-209, Oxford University press, eds. McPherson, Quirke, Taylor), followed by yeast *in vivo* recombination.

**General primers for amplification and sequencing**

The primers AM34 (SEQ ID NO:5) and AM35 (SEQ ID NO:6) were used to make DNA fragments containing any mutated fragments by the SOE method together with degenerated primers (AM34 + Reverse primer and AM35 + forward primer) or just to amplify a whole protease gene (AM34 + AM35).

| PCR reaction system: | Conditions: | |
|---|---|---|
| 48.5 microL $H_2O$ | 1 | 94°C 2 min |
| 2 beads puRe Taq Ready-To-Go PCR (Amersham Biosciences) | 2 | 94°C 30 sec |
| 0.5 micro L X 2 100 pmole/microL of primers | 3 | 55°C 30 sec |
| 0.5 microL template DNA | 4 | 72°C 90 sec |
| | 2-4 | 25 cycles |
| | 5 | 72°C 10 min |

DNA fragments were recovered from agarose gel by the Qiagen gel extraction Kit. The resulting purified fragments were mixed with the vector digest. The mixed solution was introduced into *Saccharomyces cerevisiae* to construct libraries or site-directed variants by *in vivo* recombination.

**Relative activity assay**

Yeast clones on SC-glucose were inoculated to a well of a 96-well micro titre plate containing YPD+Zn medium and cultivated at 28°C for 3 days. The culture supernatants were applied to a 96-well zein micro titer plate and incubated at at least 2 temperatures (ex. 60°C and 65°C, 70°C and 75°C, 70°C and 80°C) for more than 4 hours or overnight. The turbidity of zein in the plate was measured as A630 and the relative activity (higher/lower temperatures) was determined as an indicator of thermoactivity improvement. The clones with higher relative activity than the parental variant were selected and the sequence was determined.

**Remaining activity assay**

Yeast clones on SC-glucose were inoculated to a well of a 96-well micro titre plate and cultivated at 28°C for 3 days. Protease activity was measured at 65°C using azo-casein (Megazyme) after incubating the culture supernatant in 20 mM sodium acetate buffer, pH 4.5, for 10 min at a certain temperature (80°C or 84°C with 4°C as a reference) to determine the remaining activity. The clones with higher remaining activity than the parental variant were selected and the sequence was determined.

**Azo-casein assay**

20 microL of samples were mixed with 150 microL of substrate solution (4 ml of 12.5% azo-casein in ethanol in 96 ml of 20 mM sodium acetate, pH 4.5, containing 0.01 % triton-100 and 0.25 mM $ZnSO_4$) and incubated for 4 hours or longer.

After adding 20 microL/well of 100 % trichloroacetic acid (TCA) solution, the plate was centrifuge and 100 microL of supernatants were pipette out to measure A440.

**Expression of protease variants in *Aspergillus oryzae***

The constructs comprising the protease variant genes were used to construct expression vectors for *Aspergillus.* The *Aspergillus* expression vectors consist of an expression cassette based on the *Aspergillus niger* neutral amylase II promoter fused to the *Aspergillus nidulans* triose phosphate isomerase non translated leader sequence (Pna2/tpi) and the *Aspergillus niger* amyloglycosidase terminator (Tamg). Also present on the plasmid was the *Aspergillus selective* marker amdS from *Aspergillus nidulans* enabling growth on acetamide as sole nitrogen source. The expression plasmids for protease variants were transformed into *Aspergillus* as described in Lassen et al. (2001), Appl. Environ. Microbiol. 67, 4701-4707. For each of the constructs 10-20 strains were isolated, purified and cultivated in shake flasks.

**Purification of expressed variants**

1. Adjust pH of the 0.22 μm filtered fermentation sample to 4.0.
2. Put the sample on an ice bath with magnetic stirring. Add (NH4)2SO4 in small aliquots (corresponding to approx. 2.0-2.2 M (NH4)2SO4 not taking the volume increase into account when adding the compound).
3. After the final addition of (NH4)2SO4, incubate the sample on the ice bath with gentle magnetic stirring for min. 45 min.
4. Centrifugation: Hitachi himac CR20G High-Speed Refrigerated Centrifuge equipped with R20A2 rotor head, 5°C, 20,000 rpm, 30 min.
5. Dissolve the formed precipitate in 200 ml 50 mM Na-acetate pH 4.0.
6. Filter the sample by vacuum suction using a 0.22 μm PES PLUS membrane (IWAKI).
7. Desalt/buffer-exchange the sample to 50 mM Na-acetate pH 4.0 using ultrafiltration (Vivacell 250 from Vivascience equipped with 5 kDa MWCO PES membrane) overnight in a cold room. Dilute the retentate sample to 200 ml using 50 mM Na-acetate pH 4.0. The conductivity of sample is preferably less than 5 mS/cm.
8. Load the sample onto a cation-exchange column equilibrated with 50 mM Na-acetate pH 4.0. Wash unbound sample out of the column using 3 column volumes of binding buffer (50 mM Na-acetate pH 4.0), and elute the sample using a linear gradient, 0-100% elution buffer (50 mM Na-acetate + 1 M NaCl pH 4.0) in 10 column volumes.
9. The collected fractions are assayed by an endo-protease assay (cf. below) followed by standard SDS-PAGE (reducing conditions) on selected fractions. Fractions are pooled based on the endo-protease assay and SDS-PAGE.

**Endo-protease assay**

1. Protazyme OL tablet/5 ml 250 mM Na-acetate pH 5.0 is dissolved by magnetic stirring (substrate: endo-protease Protazyme AK tablet from Megazyme - cat. # PRAK 11/08).
2. With stirring, 250 microL of substrate solution is transferred to a 1.5 ml Eppendorf tube.
3. 25 microL of sample is added to each tube (blank is sample buffer).
4. The tubes are incubated on a Thermomixer with shaking (1000 rpm) at 50°C for 15 minutes.
5. 250 microL of 1 M NaOH is added to each tube, followed by vortexing.
6. Centrifugation for 3 min. at 16,100 × G and 25°C.
7. 200 microL of the supernatant is transferred to a MTP, and the absorbance at 590 nm is recorded.

**Results**

Table 2. Relative activity of protease variants. Numbering of substitution(s) starts from N-terminal of the mature peptide in amino acids 1 to 177 of SEQ ID NO: 2.

| Variant | Substitution(s) | Relative activity 65°C/60°C |
|---|---|---|
| WT | none | 31% |
| JTP004 | S87P | 45% |
| JTP005 | A112P | 43% |
| JTP008 | R2P | 71% |
| JTP009 | D79K | 69% |
| JTP010 | D79L | 75% |
| JTP011 | D79M | 73% |
| JTP012 | D79L/S87P | 86% |
| JTP013 | D79L/S87P/A112P | 90% |
| JTP014 | D79L/S87P/A112P | 88% |
| JTP016 | A73C | 52% |
| JTP019 | A126V | 69% |
| JTP021 | M152R | 59% |

Table 3. Relative activity of protease variants. Numbering of substitution(s) starts from N-terminal of the mature peptide in amino acids 1 to 177 of SEQ ID NO: 2.

| Variant | Substitution(s) and/or deletion (S) | Relative activity | | |
|---|---|---|---|---|
| | | 70°C/65°C | 75°C/65°C | 75°C/70°C |
| WT | none | 59% | 17% | |
| JTP036 | D79L/S87P/D142L | 73% | 73% | |
| JTP040 | T54R/D79L/S87P | | 71% | |
| JTP042 | Q53K/D79L/S87P/I173V | | 108% | |
| JTP043 | Q53R/D79L/S87P | | 80% | |
| JTP045 | S41 R/D79L/S87P | | 82% | |
| JTP046 | D79L/S87P/Q158W | | 96% | |
| JTP047 | D79L/S87P/S157K | | 85% | |
| JTP048 | D79L/S87P/D104R | | 88% | |
| JTP050 | D79L/S87P/A112P/D142L | | 88% | |
| JTP051 | S41 R/D79L/S87P/A112P/D142L | | | 102% |
| JTP052 | D79L/S87P/A112P/D142L/S157K | | | 111% |
| JTP053 | S41 R/D79L/S87P/A112P/D142L/S157K | | | 113% |
| JTP054 | ΔS5/D79L/S87P | | | 92% |
| JTP055 | ΔG8/D79L/S87P | | | 95% |
| JTP059 | C6R/D79L/S87P | | | 92% |
| JTP061 | T46R/D79L/S87P | | | 111% |
| JTP063 | S49R/D79L/S87P | | | 94% |
| JTP064 | D79L/S87P/N88R | | | 92% |
| JTP068 | D79L/S87P/T114P | | | 99% |
| JTP069 | D79L/S87P/S115R | | | 103% |
| JTP071 | D79L/S87P/T116V | | | 105% |
| JTP072 | N26R/D79L/S87P | | 92% | |
| JTP077 | A27K/D79L/S87P/A112P/D142L | | 106% | |
| JTP078 | A27V/D79L/S87P/A112P/D142L | | 100% | |
| JTP079 | A27G/D79L/S87P/A112P/D142L | | 104% | |

Table 4. Relative activity of protease variants. Numbering of substitution(s) starts from N-terminal of the mature peptide in amino acids 1 to 177 of SEQ ID NO: 2.

| Variant | Substitution(s) and/or deletion(s) | Relative activity 75°C/65°C | Remaining activity | |
|---|---|---|---|---|
| | | | 80°C | 84°C |
| JTP082 | ΔS5/D79L/S87P/A112P/D142L | 129% | | 53% |
| JTP083 | T46R/D79L/S87P/A112P/D142L | 126% | | |
| JTP088 | Y43F/D79L/S87P/A112P/D142L | 119% | | |

(continued)

| Table 4. Relative activity of protease variants. Numbering of substitution(s) starts from N-terminal of the mature peptide in amino acids 1 to 177 of SEQ ID NO: 2. | | | | |
|---|---|---|---|---|
| **Variant** | **Substitution(s) and/or deletion(s)** | **Relative activity 75°C/65°C** | **Remaining activity** | |
| | | | **80°C** | **84°C** |
| JTP090 | D79L/S87P/A112P/T124L/D142L | 141% | | |
| JTP091 | D79L/S87P/A112P/T124V/D142L | 154% | 43% | |
| JTP092 | ΔS5/N26R/D79L/S87P/A112P/D142L | | | 60% |
| JTP095 | N26R/T46R/D79L/S87P/A112P/D142L | | | 62% |
| JTP096 | T46R/D79L/S87P/T116V/D142L | | | 67% |
| JTP099 | D79L/P81 R/S87P/A112P/D142L | | | 80% |
| JTP101 | A27K/D79L/S87P/A112P/T124V/D142L | | 81% | |
| JTP116 | D79L/Y82F/S87P/A112P/T124V/D142L | | 59% | |
| JTP117 | D79L/Y82F/S87P/A112P/T124V/D142L | | 94% | |
| JTP127 | D79L/S87P/A112P/T124V/A126V/D142L | | 53% | |

| Table 5. Relative activity of protease variants. Numbering of substitution(s) starts from N-terminal of the mature peptide in amino acids 1 to 177 of SEQ ID NO: 2. | | | | |
|---|---|---|---|---|
| **Variant** | **Substitutions** | **Relative activity** | | |
| | | **75°C/70°C** | **80°C/70°C** | **85°C/70°C** |
| JTP050 | D79L S87P A112P D142L | 55% | 23% | 9% |
| JTP134 | D79L Y82F S87P A112P D142L | | 40% | |
| JTP135 | S38T D79L S87P A112P A126V D142L | | 62% | |
| JTP136 | D79L Y82F S87P A112P A126V D142L | | 59% | |
| JTP137 | A27K D79L S87P A112P A126V D142L | | 54% | |
| JTP140 | D79L S87P N98C A112P G135C D142L | 81% | | |
| JTP141 | D79L S87P A112P D142L T141C M161C | 68% | | |
| JTP143 | S36P D79L S87P A112P D142L | 69% | | |
| JTP144 | A37P D79L S87P A112P D142L | 57% | | |
| JTP145 | S49P D79L S87P A112P D142L | 82% | 59% | |
| JTP146 | S50P D79L S87P A112P D142L | 83% | 63% | |
| JTP148 | D79L S87P D104P A112P D142L | 76% | 64% | |
| JTP161 | D79L Y82F S87G A112P D142L | | 30% | 12% |
| JTP180 | S70V D79L Y82F S87G Y97W A112P D142L | | 52% | |
| JTP181 | D79L Y82F S87G Y97W D104P A112P D142L | | 45% | |
| JTP187 | S70V D79L Y82F S87G A112P D142L | | 45% | |
| JTP188 | D79L Y82F S87G D104P A112P D142L | | 43% | |

| | | | | |
|---|---|---|---|---|
| JTP189 | D79L Y82F S87G A112P A126V D142L | | 46% | |
| JTP193 | Y82F S87G S70V D79L D104P A112P D142L | | | 15% |
| JTP194 | Y82F S87G D79L D104P A112P A126V D142L | | | 22% |
| JTP196 | A27K D79L Y82F S87G D104P A112P A126V D142L | | | 18% |

Table 5. Relative activity of protease variants. Numbering of substitution(s) starts from N-terminal of the mature peptide in amino acids 1 to 177 of SEQ ID NO: 2.

| Variant | Substitutions | Relative activity | |
|---|---|---|---|
| | | **75°C/70°C** | **80°C/70°C** |
| JTP196 | A27K D79L Y82F S87G D104P A112P A126V D142L | 102% | 55% |
| JTP210 | A27K Y82F S87G D104P A112P A126V D142L | 107% | 36% |
| JTP211 | A27K D79L Y82F D104P A112P A126V D142L | 94% | 44% |
| JTP213 | A27K Y82F D104P A112P A126V D142L | 103% | 37% |

**Example 3**

Temperature profile of selected variants using purified enzymes

Selected variants showing good thermo-stability were purified and the purified enzymes were used in a zein-BCA assay as described below. The remaining protease activity was determined at 60°C after incubation of the enzyme at elevated temperatures as indicated for 60 min.

Zein-BCA assay:

Zein-BCA assay was performed to detect soluble protein quantification released from zein by variant proteases at various temperatures.

Protocol:

1) Mix 10 microliters of 10 micrograms/ml enzyme solutions and 100ul of 0.025% zein solution in a micro titer plate (MTP).
2) Incubate at various temperatures for 60min.
3) Add 10 microliters of 100% trichloroacetic acid (TCA) solution.
4) Centrifuge MTP at 3500 rpm for 5 min.
5) Take out 15 microliters to a new MTP containing 100 microliters of BCA assay solution (Pierce Cat#:23225, BCA Protein Assay Kit).
6) Incubate for 30 min. at 60°C.
7) Measure A562.

The results are shown in Table 5. All of the tested variants showed an improved thermo-stability as compared to the wt protease.

Table 5. Zein-BCA assay

| WT/Variant | Sample incubated 60 min at indicated temperatures (°C) (μg/ml Bovine serum albumin equivalent peptide released) | | | | | | |
|---|---|---|---|---|---|---|---|
| | 60°C | 70°C | 75°C | 80°C | 85°C | 90°C | 95°C |
| WT | 94 | 103 | 107 | 93 | 58 | 38 | |
| JTP050 | 86 | 101 | 107 | 107 | 104 | 63 | 36 |
| JTP077 | 82 | 94 | 104 | 105 | 99 | 56 | 31 |
| JTP188 | 71 | 83 | 86 | 93 | 100 | 75 | 53 |
| JTP196 | 87 | 99 | 103 | 106 | 117 | 90 | 38 |

**Example 4**

Characterization of *Penicillium oxalicum* glucoamylase

The *Penicillium oxalicum* glucoamylase is disclosed in SEQ ID NO: 9 herein.

**Substrate.** Substrate: 1% soluble starch (Sigma S-9765) in deionized water
Reaction buffer: 0.1 M Acetate buffer at pH 5.3
Glucose concentration determination kit: Wako glucose assay kit (LabAssay glucose, WAKO, Cat# 298-65701).
**Reaction condition.** 20 microL soluble starch and 50 microL acetate buffer at pH 5.3 were mixed. 30 microL enzyme solution (50 micro g enzyme protein/ml) was added to a final volume of 100 microL followed by incubation at 37°C for 15 min.
The glucose concentration was determined by Wako kits.
All the work carried out in parallel.
**Temperature optimum.** To assess the temperature optimum of the *Penicillium oxalicum* glucoamylase the "Reaction condition"-assay described above was performed at 20, 30, 40, 50, 60, 70, 80, 85, 90 and 95°C. The results are shown in Table 6.

Table 6 Temperature optimum

| Temperature (°C) | 20 | 30 | 40 | 50 | 60 | 70 | 80 | 85 | 90 | 95 |
|---|---|---|---|---|---|---|---|---|---|---|
| Relative activity (%) | 63.6 | 71.7 | 86.4 | 99.4 | 94.6 | 100.0 | 92.9 | 92.5 | 82.7 | 82.8 |

From the results it can be seen that the optimal temperature for *Penicillium oxalicum* glucoamylase at the given conditions is between 50°C and 70°C and the glucoamylase maintains more than 80% activity at 95°C.
**Heat stability.** To assess the heat stability of the *Penicillium oxalicum* glucoamylase the Reaction condition assay was modifed in that the the enzyme solution and acetate buffer was preincubated for 15 min at 20, 30, 40, 50, 60, 70, 75, 80, 85, 90 and 95°C. Following the incubation 20 microL of starch was added to the solution and the assay was performed as described above.
The results are shown in Table 7.

Table 7 Heat stability

| Temperature (°C) | 20 | 30 | 40 | 50 | 60 | 70 | 80 | 85 | 90 | 95 |
|---|---|---|---|---|---|---|---|---|---|---|
| Relative activity (%) | 91.0 | 92.9 | 88.1 | 100.0 | 96.9 | 86.0 | 34.8 | 36.0 | 34.2 | 34.8 |

From the results it can be seen that *Penicillium oxalicum* glucoamylase is stable up to 70 °C after preincubation for 15 min in that it maintains more than 80% activity.
**pH optimum.** To assess the pH optimum of the *Penicillium oxalicum* glucoamylase the Reaction condition assay described above was performed at pH 2.0, 3.0, 3.5, 4.0, 4.5, 5.0, 6.0 7.0, 8.0, 9.0, 10.0 and 11.0. Instead of using the acetate buffer described in the Reaction condition assay the following buffer was used 100mM Succinic acid, HEPES, CHES, CAPSO, 1 mM $CaCl_2$, 150mM KCl, 0.01% Triton X-100, pH adjusted to 2.0, 3.0, 3.5, 4.0, 4.5, 5.0, 6.0 7.0, 8.0, 9.0, 10.0 or 11.0 with HCl or NaOH.

The results are shown in Table 8.

Table 8 pH optimum

| pH | 2.0 | 3.0 | 3.5 | 4.0 | 4.5 | 5.0 | 6.0 | 7.0 | 8.0 | 9.0 | 10.0 | 11.0 |
|---|---|---|---|---|---|---|---|---|---|---|---|---|
| Relative activity (%) | 71.4 | 78.6 | 77.0 | 91.2 | 84.2 | 100.0 | 55.5 | 66.7 | 30.9 | 17.8 | 15.9 | 16.1 |

From the results it can be seen that *Penicillium oxalicum* glucoamylase at the given conditions has the highest activity at pH 5.0. The *Penicillium oxalicum* glucoamylase is active in a broad pH range in the it maintains more than 50% activity from pH 2 to 7.

**pH stability.** To assess the heat stability of the *Penicillium oxalicum* glucoamylase the Reaction condition assay was modifed in that the enzyme solution (50micro g/mL) was preincubated for 20 hours in buffers with pH 2.0, 3.0, 3.5, 4.0, 4.5, 5.0, 6.0 7.0, 8.0, 9.0, 10.0 and 11.0 using the buffers described under pH optimum. After preincubation, 20 microL soluble starch to a final volume of 100 microL was added to the solution and the assay was performed as described above.

The results are shown in Table 9.

Table 9 pH stability

| pH | 2.0 | 3.0 | 3.5 | 4.0 | 4.5 | 5.0 | 6.0 | 7.0 | 8.0 | 9.0 | 10.0 | 11.0 |
|---|---|---|---|---|---|---|---|---|---|---|---|---|
| Relative activity (%) | 17.4 | 98.0 | 98.0 | 103.2 | 100.0 | 93.4 | 71.2 | 90.7 | 58.7 | 17.4 | 17.0 | 17.2 |

From the results it can be seen that *Penicillium oxalicum* glucoamylase, is stable from pH 3 to pH 7 after preincubation for 20 hours and it decreases its activity at pH 8.

**Example 5**

Thermostability of Protease Pfu.

The thermostability of the *Pyrococcus furiosus* protease (Pfu S) purchased from Takara Bio Inc, (Japan) was tested using the same methods as in Example 2. It was found that the thermostability (Relative Activity) was 110% at (80°C/70°C) and 103% (90°C/70°C) at pH 4.5.

**Example 6** (Embodiment of the invention)

Ethanol Production Using Alpha-Amylase 1407 and Protease Pfu for Liquefaction

The purpose of this experiment was to evaluate the application performance of Protease Pfu derived from *Pyrococcus furiosus* added during liquefaction at pH 5.4 and 5.8, respectively, at 85°C for 2 hours.

**Liquefaction (Labomat)**

Each liquefaction received ground corn (84.19% DS), backset (6.27% DS), and tap water targeting a total weight of 100 g at 32.50% Dry Solids (DS). Backset was blended at 30% w/w of total slurry weight. Initial slurry pH was approximately 5.2 and was adjusted to pH 5.4 or 5.8 with 50% w/w sodium hydroxide prior to liquefaction. All enzymes were added according to the experimental design listed in Table 11 below. Liquefaction took place in a Labomat using the following conditions: 5°C/min. Ramp, 17 minute Ramp, 103 minute hold time at 85°C, 40 rpm for the entire run, 200 mL stainless steel canisters. After liquefaction, all canisters were cooled in an ice bath and prepared for fermentation based on the protocol listed below under SSF.

**Simultaneous Saccharification and Fermentation (SSF)**

Each mash was adjusted to pH 5.0 with 50% w/w Sodium Hydroxide or 40% v/v sulfuric acid. Penicillin was applied to each mash to a total concentration of 3 ppm. The tubes were prepared with mash by aliquoting approximately 4.5 g of mash per 15 mL pre-drilled test tubes to allow $CO_2$ release. The test tubes sat, overnight, at 4°C until the next morning.

All test tubes of mash were removed from cold storage and warmed up to 32°C in the walk-in incubation chamber. Once warmed, Glucoamylase BL2, was dosed to each tube of mash at 0.50 AGU/g DS, water was added so that all tubes received 120 µL of liquid and each mash sample received 100 µL of rehydrated yeast. Rehydrated yeast was prepared by mixing 5.5 g of Fermentis RED STAR into 100 mL of 32°C tap water for at least 15 minutes.

In monitoring $CO_2$ weight-loss over time, each unit of $CO_2$ generated and lost is converted to gram ethanol produced per gram of dry solids (g EtOH/g DS) by the following:

$$\text{g ethanol/ g DS} = \frac{\text{g CO2 weight loss} \times \frac{\text{1mol CO2}}{\text{44.0098g CO2}} \times \frac{\text{1mol ethanol}}{\text{1mol CO2}} \times \frac{\text{46.094g ethanol}}{\text{1mol ethanol}}}{\text{g mash in tube} \times \text{\%DS of mash}}$$

**HPLC analysis**

Fermentation sampling took place after 54 hours of fermentation by taking 3 tubes per treatment. Each sample was deactivated with 50 µL of 40% v/v $H_2SO_4$, vortexing, centrifuging at 1460xg for 10 minutes, and filtering through a 0.45 µm Whatman PP filter. 54 hour samples were analyzed under HPLC without further dilution. Samples were stored at 4°C prior to and during HPLC analysis.

| | |
|---|---|
| HPLC system | Agilent's 1100/1200 series with Chem station software Degasser, Quaternary Pump, Auto-Sampler, Column Compartment /w Heater<br>Refractive Index Detector (RI) |
| Column | Bio-Rad HPX- 87H Ion Exclusion Column 300mm x 7.8mm part# 125-0140<br>Bio-Rad guard cartridge cation H part# 125-0129, Holder part# 125-0131 |
| Method | 0.005M $H_2SO_4$ mobile phase<br>Flow rate: 0.6 ml/min<br>Column temperature: 65°C<br>RI detector temperature: 55°C |

The method quantified analyte(s) using calibration standards for ethanol (% w/v). A four point calibration including the origin is used for quantification.

Where applicable, data were analyzed using JMP software (Cary, NC) with Oneway ANOVA of pairs using Tukey-Kramer HSD or Dunnett's. Error bars denoting the 95% confidence level were established by multiplying the standard error of Oneway Anova analysis by 1.96.

Table 11. Experimental Plan

| Liquefaction at 85°C for 2 hours | | | | | | |
|---|---|---|---|---|---|---|
| pH | Alpha-Amylase | Dose µg/g DS | Protease | Dose µg/g DS | Glucoamylase | Dose µg/g DS |
| 5.4 | 1407 | 1.4 | - | - | - | - |
| 5.4 | 1407 | 1.4 | Pfu | 2 | - | - |
| 5.4 | 1407 | 1.4 | Pfu | 2 | PE001 | 10 |
| 5.8 | 1407 | 1.4 | - | - | - | - |
| 5.8 | 1407 | 1.4 | Pfu | 2 | - | - |
| 5.8 | 1407 | 1.4 | Pfu | 2 | PE001 | 10 |

Results:

| Treatment | pH | EtOH (%w/v) | EtOH (%) | JMP Std Error | 95%CI |
|---|---|---|---|---|---|
| Control | 5.8 | 8.6 | 100% | 0.015 | 0.029 |
| | 5.4 | 8.9 | 100% | 0.012 | 0.024 |
| Pfu | 5.8 | 11.0 | 128% | 0.015 | 0.029 |
| | 5.4 | 10.8 | 122% | 0.012 | 0.024 |
| Pfu+PE001 | 5.8 | 11.0 | 128% | 0.015 | 0.029 |
| | 5.4 | 11.0 | 124% | 0.012 | 0.024 |

**Example 7**

Ethanol production with Alpha-Amylase A and Thermostable Protease 050 and Protease 036

The purpose of this experiment was to evaluate the application performance of two thermostable protease variants of the *Thermoascus auranticus* protease added during liquefaction at pH 5.4 at 85°C for 2 hours.

Liquefaction

Ground corn, backset and tap water were blended to 32.50 % DS and adjusted to pH 5.4 with 50% v/v sodium hydroxide. Each respective protease was added, mixed well, and followed by Alpha-Amylase A addition at a dose of 0.02% (w/w) per g corn. Samples were incubated in a water bath set to 85°C for two hours and received frequent mixing during the first 15 minutes of incubation, every 15 minutes thereafter. All mashes were refrigerated after liquefaction and remained there until fermentation.

Simultaneous Saccharification and Fermentation (SSF)

Mashes were adjusted to 32% DS with tap water prior to SSF as needed and dosed to a total concentration of 500 ppm urea and 3 ppm penicillin. No pH adjustment was made for SSF after liquefaction. Approximately 4.5g of mash was added to 15mL test tubes that were pre-drilled in the top to allow for $CO_2$ release. Glucoamylase BL2 was dosed at 0.50 AGU/g DS, and water was added to each tube to ensure all samples were processed at equal solids. Rehydrated yeast was prepared by mixing 5.5 g of Fermentis RED STAR in 100mL of 32°C tap water for at least 15 minutes and each test tube was inoculated with 100$\mu$L, corresponding to 30 million cells per mL of mash.

All 54 hour fermentation samples selected for HPLC analysis and were deactivated with 50 $\mu$L of 40% v/v $H_2SO_4$, vortexed, centrifuged at 1460xg for 10 min., and then filtered through a 0.45 $\mu$m Whatman filter. Samples were stored at 4°C prior to HPLC analysis. HPLC analysis was done as described in Example 6.

In monitoring $CO_2$ weight-loss over time, each unit of $CO_2$ lost is converted to gram Ethanol produced per gram of dry solids (g EtOH/g DS) using the formula in Example 6.

All liquefactions included Alpha-Amylase A.

| Treatment | EtOH (%w/v) | EtOH (%?) |
|---|---|---|
| Control | 12.26 | 100.0 |
| Protease WT | 12.39 | 101.0 |
| Protease 036 | 12.55 | 102.4 |
| Protease 050 | 12.67 | 103.3 |

**Example 8**

**Cloning of *Penicillium oxalicum* strain glucoamylase gene**

**Preparation of *Penicillium oxalicum* strain cDNA.**

The cDNA was synthesized by following the instruction of 3' Rapid Amplifiction of cDNA End System (Invitrogen Corp., Carlsbad, CA, USA).

**Cloning of *Penicillium oxalicum* strain glucoamylase gene.**

The *Penicillium oxalicum* glucoamylase gene was cloned using the oligonucleotide primer shown below designed to amplify the glucoamylase gene from 5' end.

Sense primer: 5'-ATGCGTCTCACTCTATTATCAGGTG-3' (SEQ ID NO: 15)

The full length gene was amplified by PCR with Sense primer and AUAP (supplied by 3' Rapid Amplifiction of cDNA End System) by using Platinum HIFI Taq DNA polymerase (Invitrogen Corp., Carlsbad, CA, USA). The amplification reaction was composed of 5 μl of 10x PCR buffer, 2 μl of 25mM $MgCl_2$, 1 μl of 10mM dNTP, 1 μl of 10uM Sense primer, 1 μl of 10 uM AUAP, 2 μl of the first strand cDNA, 0.5 μl of HIFI Taq, and 37.5 μl of deionized water. The PCR program was: 94°C, 3mins; 10 cycles of 94°C for 40secs, 60°C 40secs with 1°C decrease per cycle, 68°C for 2min; 25 cycles of 94°C for 40secs, 50°C for 40secs, 68°C for 2min; final extension at 68°C for 10 mins.

The obtained PCR fragment was cloned into pGEM-T vector (Promega Corporation, Madison, WI, USA) using a pGEM-T Vector System (Promega Corporation, Madison, WI, USA) to generate plasmid AMG 1. The glucoamylase gene inserted in the plasmid AMG 1 was sequencing confirmed. *E. coli* strain TOP10 containing plasmid AMG 1 (designated NN059173), was deposited with the Deutsche Sammlung von Mikroorganismen und Zellkulturen GmbH (DSMZ) on November 23, 2009, and assigned accession number as DSM 23123.

**Example 9**

**Expression of cloned *Penicillium oxalicum* glucoamylase**

The *Penicillium oxalicum* glucoamylase gene was re-cloned from the plasmid AMG 1 into an *Aspergillus* expression vector by PCR using two cloning primer F and primer R shown below, which were designed based on the known sequence and added tags for direct cloning by IN-FUSION™ strategy.

Primer F: 5' ACACAACTGGGGATCCACCATGCGTCTCACTCTATTATC (SEQ ID NO: 16)
Primer R: 5' AGATCTCGAGAAGCTTAAAACTGCCACACGTCGTTGG (SEQ ID NO: 17)

A PCR reaction was performed with plasmid AMG 1 in order to amplify the full-length gene. The PCR reaction was composed of 40 μg of the plasmid AMG 1 DNA, 1 μl of each primer (100 μM); 12.5 μl of 2X Extensor Hi-Fidelity master mix (Extensor Hi-Fidelity Master Mix, ABgene, United Kingdom), and 9.5 μl of PCR-grade water. The PCR reaction was performed using a DYAD PCR machine (Bio-Rad Laboratories, Inc., Hercules, CA, USA) programmed for 2 minutes at 94°C followed by a 25 cycles of 94°C for 15 seconds, 50°C for 30 seconds, and 72°C for 1 minute; and then 10 minutes at 72°C.

The reaction products were isolated by 1.0% agarose gel electrophoresis using 1 x TAE buffer where an approximately 1.9 kb PCR product band was excised from the gel and purified using a GFX® PCR DNA and Gel Band Purification Kit (GE Healthcare, United Kingdom) according to manufacturer's instructions. DNA corresponding to the *Penicillium oxalicum* glucoamylase gene was cloned into an *Aspergillus* expression vector linearized with BamHI and *Hind*III, using an IN-FUSION™ Dry-Down PCR Cloning Kit (BD Biosciences, Palo Alto, CA, USA) according to the manufacturer's instructions. The linearized vector construction is as described in WO 2005/042735 A1.

A 2 μl volume of the ligation mixture was used to transform 25 μl of Fusion Blue *E. coli* cells (included in the IN-FUSION™ Dry-Down PCR Cloning Kit). After a heat shock at 42°C for 45 sec, and chilling on ice, 250 μl of SOC medium was added, and the cells were incubated at 37°C at 225 rpm for 90 min before being plated out on LB agar plates containing 50 μg of ampicillin per ml, and cultivated overnight at 37°C. Selected colonies were inoculated in 3 ml of LB medium supplemented with 50 μg of ampicillin per ml and incubated at 37°C at 225 rpm overnight. Plasmid DNA from the selected colonies was purified using Mini JETSTAR (Genomed, Germany) according to the manufacturer's instructions. *Penicillium oxalicum* glucoamylase gene sequence was verified by Sanger sequencing before heterologous expression. One of the plasmids was selected for further expression, and was named XYZ XYZ1471-4.

Protoplasts of *Aspergillus niger* MBin118 were prepared as described in WO 95/02043. One hundred μl of protoplast suspension were mixed with 2.5 μg of the XYZ1471-4 plasmid and 250 microliters of 60% PEG 4000 (Applichem) (polyethylene glycol, molecular weight 4,000), 10 mM $CaCl_2$, and 10 mM Tris-HCl pH 7.5 were added and gently mixed. The mixture was incubated at 37°C for 30 minutes and the protoplasts were mixed with 6% low melting agarose (Biowhittaker Molecular Applications) in COVE sucrose (Cove, 1996, Biochim. Biophys. Acta 133:51-56) (1 M) plates supplemented with 10 mM acetamid and 15 mM CsCl and added as a top layer on COVE sucrose (1 M) plates supplemented with 10 mM acetamid and 15 mM CsCl for transformants selection (4 ml topagar per plate). After incubation for 5 days at 37°C spores of sixteen transformants were picked up and seed on 750 μl YP-2% Maltose medium in 96 deepwell MT plates. After 5 days of stationary cultivation at 30°C, 10 μl of the culture-broth from each well was analyzed on a SDS-PAGE (Sodium dodecyl sulfate-polyacrylamide gel electrophoresis) gel, Griton XT Precast gel (BioRad, CA, USA) in order to identify the best transformants based on the ability to produce large amount of glucoamylase. A selected transformant was identified on the original transformation plate and was preserved as spores in a 20% glycerol stock and stored frozen (-80°C).

**Cultivation.** The selected transformant was inoculated in 100ml of MLC media and cultivated at 30 °C for 2 days in 500 ml shake flasks on a rotary shaker. 3 ml of the culture broth was inoculated to 100ml of M410 medium and cultivated at 30°C for 3 days. The culture broth was centrifugated and the supernatant was filtrated using 0.2 μm membrane filters.

**Alpha-cyclodextrin affinity gel.** Ten grams of Epoxy-activated Sepharose 6B (GE Healthcare, Chalfont St. Giles, U.K) powder was suspended in and washed with distilled water on a sintered glass filter. The gel was suspended in coupling solution (100 ml of 12.5 mg/ml alpha-cyclodextrin, 0.5 M NaOH) and incubated at room temperature for one day with gentle shaking. The gel was washed with distilled water on a sintered glass filter, suspended in 100 ml of 1 M ethanolamine, pH 10, and incubated at 50 °C for 4 hours for blocking. The gel was then washed several times using 50 mM Tris-HCl, pH 8 and 50 mM NaOAc, pH 4.0 alternatively. The gel was finally packed in a 35-40 ml column using equilibration buffer (50 mM NaOAc, 150 mM NaCl, pH 4.5).

**Purification of glucoamylase from culture broth.** Culture broth from fermentation of *A. niger* MBin118 harboring the glucoamylase gene was filtrated through a 0.22 μm PES filter, and applied on a alpha-cyclodextrin affinity gel column previously equilibrated in 50 mM NaOAc, 150 mM NaCl, pH 4.5 buffer. Unbound material was washed off the column with equilibration buffer and the glucoamylase was eluted using the same buffer containing 10 mM beta-cyclodextrin over 3 column volumes.

The glucoamylase activity of the eluent was checked to see, if the glucoamylase had bound to the alpha-cyclodextrin affinity gel. The purified glucoamylase sample was then dialyzed against 20 mM NaOAc, pH 5.0. The purity was finally checked by SDS-PAGE, and only a single band was found.

## Example 10

### Construction and expression of a site-directed variant of *Penicillium oxalicum* glucoamylase (PE001)

Two PCR reactions were performed with plasmid XYZ1471-4, described in Example 9, using primers K79V F and K79VR shown below, which were desined to substitute lysine K at position 79 from the mature seequence to varin V and primers F-NP003940 and R-NP003940 shown below, which were designed based on the known sequence and added tags for direct cloning by IN-FUSION™ strategy.

Primer K79V F 18mer GCAGTCTTTCCAATTGAC (SEQ ID NO: 18)
Primer K79V R 18mer AATTGGAAAGACTGCCCG (SEQ ID NO: 19)
Primer F-NP003940: 5' ACACAACTGGGGATCCACCATGCGTCTCACTCTATTATC (SEQ ID NO: 20)
Primer R-NP003940: 5' AGATCTCGAGAAGCTTAAAACTGCCACACGTCGTTGG (SEQ ID NO: 21)

The PCR was performed using a PTC-200 DNA Engine under the conditions described below.

<table>
<tr><th>PCR reaction system:</th><th colspan="3">Conditions:</th></tr>
<tr><td rowspan="6">48.5 micro L H2O<br>2 beads puRe Taq Ready-To-Go PCR Beads (Amersham bioscineces)<br>0.5micro L X 2100 pmole/micro L Primers (K79V F + Primer R-NP003940, K79V R + Primer F-NP003940) 0.5 micro L Template DNA</td><td>1</td><td>94°C</td><td>2 min</td></tr>
<tr><td>2</td><td>94°C</td><td>30 sec</td></tr>
<tr><td>3</td><td>55°C</td><td>30 sec</td></tr>
<tr><td>4</td><td>72°C</td><td>90 sec</td></tr>
<tr><td>2-4</td><td colspan="2">25 cycles</td></tr>
<tr><td>5</td><td>72°C</td><td>10 min</td></tr>
</table>

DNA fragments were recovered from agarose gel by the Qiagen gel extraction Kit according to the manufacturer's instruction. The resulting purified two fragments were cloned into an *Aspergillus* expression vector linearized with *Bam*HI and *Hind*III, using an IN-FUSION™ Dry-Down PCR Cloning Kit (BD Biosciences, Palo Alto, CA, USA) according to the manufacturer's instructions. The linearized vector construction is as described in WO 2005/042735 A1.

The ligation mixture was used to transform *E. coli* DH5α cells (TOYOBO). Selected colonies were inoculated in 3 ml of LB medium supplemented with 50 μg of ampicillin per ml and incubated at 37°C at 225 rpm overnight. Plasmid DNA from the selected colonies was purified using Qiagen plasmid mini kit (Qiagen) according to the manufacturer's instructions. The sequence of *Penicillium oxalicum* glucoamylase site-directed variant gene sequence was verified before heterologous expression and one of the plasmids was selected for further expression, and was named pPoPE001.

Protoplasts of *Aspergillus niger* MBin118 were prepared as described in WO 95/02043. One hundred microliters of protoplast suspension were mixed with 2.5 μg of the pPoPE001 plasmid and 250 microliters of 60% PEG 4000 (Applichem) (polyethylene glycol, molecular weight 4,000), 10 mM $CaCl_2$, and 10 mM Tris-HCl pH 7.5 were added and gently mixed. The mixture was incubated at 37°C for 30 minutes and the protoplasts were mixed with 1% agarose L (Nippon Gene) in COVE sucrose (Cove, 1996, Biochim. Biophys. Acta 133:51-56) supplemented with 10 mM acetamid and 15 mM CsCl and added as a top layer on COVE sucrose plates supplemented with 10 mM acetamid and 15 mM CsCl for transformants selection (4 ml topagar per plate). After incubation for 5 days at 37°C spores of sixteen transformants were picked up and seed on 750 μl YP-2% Maltose medium in 96 deepwell MT plates. After 5 days of stationary cultivation at 30°C, 10 μl of the culture-broth from each well was analyzed on a SDS-PAGE gel in order to identify the best transformants based on the ability to produce large amount of the glucoamylase.

**Example 11**

**Purification of site-directed Po AMG variant PE001**

The selected transformant of the variant and the strain expressing the wild type *Penicillium oxalicum* glucoamylase described in Example 8 was cultivated in 100 ml of YP- 2% maltose medium and the culture was filtrated through a 0.22 μm PES filter, and applied on a alpha-cyclodextrin affinity gel column previously equilibrated in 50 mM NaOAc, 150 mM NaCl, pH 4.5 buffer. Unbound materias was washed off the column with equilibration buffer and the glucoamylase was eluted using the same buffer containing 10 mM beta-cyclodextrin over 3 column volumes.

The glucoamylase activity of the eluent was checked to see, if the glucoamylase had bound to the alpha-cyclodextrin affinity gel. The purified glucoamylase samples were then dialyzed against 20 mM NaOAc, pH 5.0.

**Example 12**

**Characterization of PE001 Protease stability**

40 μl enzyme solutions (1 mg/ml) in 50 mM sodium acetate buffer, pH 4.5, was mixed with 1/10 volume of 1 mg/ml protease solutions such as aspergillopepsinI described in Biochem J. 1975 Apr; 147(1): 45-53 or the commercially availble product from Sigma and aorsin descrived in Biochemical journal [0264-6021] Ichishima, 2003, 371(2): 541 and incubated at 4 or 32°C overnight. As a control experiment, $H_2O$ was added to the sample instead of proteases. The samples were loaded on SDS-PAGE to see if the glucoamylases are cleaved by proteases.

In SDS-PAGE, PE001 only showed one band corresponding to the intact molecule, while the wild type glucoamylase was degraded by proteases and showed a band at lower molecular size at 60 kCa.

TABLE 12 The result of SDS-PAGE after protease treatment

| | Wild type glucoamylase | | | | PE001 | | | | control |
|---|---|---|---|---|---|---|---|---|---|
| Protease | aspergillopepsin I | | aorsin | | aspergillopepsin I | | aorsin | | |
| Incubation temperature (°C) | 4 | 32 | 4 | 32 | 4 | 32 | 4 | 32 | 4 |
| intact glucoamylase (ca. 70 kDa) | 100% | 90% | 40% | 10% | 100% | 100% | 100 % | 100 % | 100% |
| cleaved glucoamylase (ca. 60 kDa) | N.D. | 10% | 60% | 90% | N.D. | N.D. | N.D | N.D | N.D. |
| N.D.: not detected. | | | | | | | | | |

**Example 13**

**Less cleavage during cultivation**

*Aspergillus* transformant of the variant and the wild type *Penicillium oxalicum* glucoamylase were cultivated in 6-well MT plates containing 4X diluted YP-2% maltose medium supplemented with 10 mM sodium acetate buffer, pH4.5, at 32°C for 1 week.

The culture supernatants were loaded on SDS-PAGE.

TABLE 13 The result of SDS-PAGE of the culture supernatants

| | Wild type glucoamylase | PE001 |
|---|---|---|
| intact glucoamylase(ca. 70 kDa) | 90% | 100% |
| cleaved glucoamylase (ca. 60 kDa) | 10% | N.D. |
| N.D.: not detected. | | |

The wild type glucoamylase was cleaved by host proteasaes during fermentation, while the variant yielded only intact molecule.

**Example 14**

**Glucoamylase activity of variant PE001 compared to parent**

The glucoamylase activity measures as AGU as described above was checked for the purified enzymes of the wild type *Penicillium oxalicum* and the variant glucoamylase.

The Glucoamylase Unit (AGU) was defined as the amount of enzyme, which hydrolyzes 1 micromole maltose per minute under the standard conditions (37°C, pH 4.3, substrate: maltose 100 mM, buffer: acetate 0.1 M, reaction time 6 minutes).

Table 14

| Relative specific activity | AGU/mg |
|---|---|
| *Penicillium oxalicum* wt | 100 % |
| *Penicillium oxalicum* PE001 (SEQ ID NO: 14 + V79K) | 102 % |

**Example 15**

**Purification of Glycoamylase variants having increased thermostability**

The variants showing increased thermostability may be constructed and expressed similar to the procedure described in Example 10. All variants were derived from the PE001. After expression in YPM medium, variants comprising the T65A or Q327F substitution was micro-purified as follows:

Mycelium was removed by filtration through a 0.22 μm filter. 50 μl column material (alpha-cyclodextrin coupled to Mini-Leak divinylsulfone-activated agarose medium according to manufacturers recommendations) was added to the wells of a filter plate (Whatman, Unifilter 800 μl, 25-30 μm MBPP). The column material was equilibrated with binding buffer (200 mM sodium acetate pH 4.5) by two times addition of 200 μl buffer, vigorous shaking for 10 min (Heidolph, Titramax 101, 1000 rpm) and removal of buffer by vacuum (Whatman, UniVac 3). Subsequently, 400 μl culture supernatant and 100 μl binding buffer was added and the plate incubated 30 min with vigorous shaking. Unbound material was removed by vacuum and the binding step was repeated. Normally 4 wells were used per variant. Three washing steps were then performed with 200 μl buffer of decreasing ionic strength added (50/10/5 mM sodium acetate, pH 4.5), shaking for 15 min and removal of buffer by vacuum. Elution of the bound AMG was achieved by two times addition of 100 μl elution buffer (250 mM sodium acetate, 0.1 % alpha-cyclodextrin, pH 6.0), shaking for 15 min and collection of eluted material in a microtiter plate by vacuum. Pooled eluates were concentrated and buffer changed to 50 mM sodium acetate pH 4.5 using centrifugal filter units with 10 kDa cut-off (Millipore Microcon Ultracel YM-10). Micropurified samples were stored at -18°C until testing of thermostability.

**Example 16**

**Protein thermal unfolding analysis (TSA, Thermal shift assay)**

Protein thermal unfolding of the T65A and Q327F variants, was monitored using Sypro Orange (In-vitrogen, S-6650) and was performed using a real-time PCR instrument (Applied Biosystems; Step-One-Plus).

In a 96-well plate, 25 microliter micropurified sample in 50 mM Acetate pH4,5 at approx. 100 microgram/ml was mixed (5:1) with Sypro Orange (resulting conc. = 5X; stock solution from supplier = 5000X). The plate was sealed with an optical PCR seal. The PCR instrument was set at a scan-rate of 76°C pr. hr, starting at 25°C and finishing at 96°C.

Protein thermal unfolding of the E501V + Y504T variant, was monitored using Sypro Orange (In-vitrogen, S-6650) and was performed using a real-time PCR instrument (Applied Biosystems; Step-One-Plus).

In a 96-well plate, 15 microliter purified sample in 50 mM Acetate pH4,5 at approx. 50 microgram/ml was mixed (1:1) with Sypro Orange (resulting conc. = 5X; stock solution from supplier = 5000X) with or without 200 ppm Acarbose (Sigma A8980). The plate was sealed with an optical PCR seal. The PCR instrument was set at a scan-rate of 76 degrees C pr. hr, starting at 25°C and finishing at 96°C.

Fluorescence was monitored every 20 seconds using in-built LED blue light for excitation and ROX-filter (610 nm, emission).

Tm-values were calculated as the maximum value of the first derivative (dF/dK) (ref.: Gregory et al., 2009, J. Biomol. Screen. 14: 700).

Table 15a.

| Sample | Tm (Deg. Celsius) +/- 0.4 |
|---|---|
| PO-AMG (PE001) | 80.3 |
| Variant Q327F | 82.3 |
| Variant T65A | 81.9 |

Table 15b.

| Sample | Tm (Deg. Celsius) +/- 0.4 | |
|---|---|---|
| Acarbose: | - | + |
| PO-AMG (PE001) | 79.5 | 86.9 |
| Variant E501V Y504T | 79.5 | 95.2 |

**Example 17**

**Thermostability analysis by Differential Scanning Calorimitry (DSC)**

Additional site specific variants having substitutions and /or deletions at specific positions were constructed basically as described in Example 10 and purified as described in Example 11.

The thermostability of the purified Po-AMG PE001 derived variants were determined at pH 4.0 or 4.8 (50 mM Sodium Acetate) by Differential Scanning Calorimetry (DSC) using a VP-Capillary Differential Scanning Calorimeter (MicroCal Inc., Piscataway, NJ, USA). The thermal denaturation temperature, Td (°C), was taken as the top of the denaturation peak (major endothermic peak) in thermograms (Cp vs. T) obtained after heating enzyme solutions in selected buffers (50 mM Sodium Acetate, pH 4.0 or 4.8)at a constant programmed heating rate of 200 K/hr.

Sample- and reference-solutions (approximately 0.3 ml) were loaded into the calorimeter (reference: buffer without enzyme) from storage conditions at 10°C and thermally pre-equilibrated for 10 minutes at 20°C prior to DSC scan from 20°C to 110°C. Denaturation temperatures were determined with an accuracy of approximately +/- 1°C.

The isolated variants and the DSC data are disclosed in Table 16 below.

Table 16.

| Po-AMG name | Mutations | DSC Td (°C) @ | DSC Td (°C) @ |
|---|---|---|---|
| | Mutations relative to PE001 | **pH 4.0** | **pH 4.8** |
| PE001 (SEQ ID NO: 3) | | 82.1 | 83.4 |
| GA167 | E501V Y504T | 82.1 | |
| GA481 | T65AK161S | 84.1 | 86.0 |
| GA487 | T65A Q405T | 83.2 | |
| GA490 | T65A Q327W | 87.3 | |
| GA491 | T65A Q327F | 87.7 | |
| GA492 | T65A Q327Y | 87.3 | |
| GA493 | P11 F T65A Q327F | 87.8 | 88.5 |
| GA497 | R1K D3W K5Q G7V N8S T10K P11S T65A Q327F | 87.8 | 88.0 |
| GA498 | P2N P4S P11 F T65A Q327F | 88.3 | 88.4 |
| GA003 | P11 F D26C K33C T65A Q327F | 83.3 | 84.0 |
| GA009 | P2N P4S P11F T65A Q327W E501V Y504T | 88.8 | |
| GA002 | R1E D3N P4G G6R G7A N8A T10D P11 D T65A Q327F | 87.5 | 88.2 |
| GA005 | P11 F T65A Q327W | 87.4 | 88.0 |
| GA008 | P2N P4S P11F T65A Q327F E501V Y504T | 89.4 | 90.2 |
| GA010 | P11F T65A Q327W E501 V Y504T | | 89.7 |
| GA507 | T65A Q327F E501V Y504T | | 89.3 |
| GA513 | T65A S105P Q327W | | 87.0 |
| GA514 | T65A S105P Q327F | | 87.4 |
| GA515 | T65A Q327W S364P | | 87.8 |
| GA516 | T65A Q327F S364P | | 88.0 |
| GA517 | T65A S103N Q327F | | 88.9 |
| GA022 | P2N P4S P11 F K34Y T65A Q327F | | 89.7 |
| GA023 | P2N P4S P11F T65A Q327F D445N V447S | | 89.9 |
| GA032 | P2N P4S P11 F T65A I172V Q327F | | 88.7 |
| GA049 | P2N P4S P11 F T65A Q327F N502* | | 88.4 |
| GA055 | P2N P4S P11F T65A Q327F N502T P563S K571E | | 88.0 |
| GA057 | P2N P4S P11F R31S K33V T65A Q327F N564D K571S | | 89.5 |
| GA058 | P2N P4S P11 F T65A Q327F S377T | | 88.6 |
| GA064 | P2N P4S P11 F T65A V325T Q327W | | 88.0 |
| GA068 | P2N P4S P11F T65A Q327F D445N V447S E501V Y504T | | 90.2 |
| GA069 | P2N P4S P11F T65A I172V Q327F E501V Y504T | | 90.2 |
| GA073 | P2N P4S P11F T65A Q327F S377T E501V Y504T | | 90.1 |
| GA074 | P2N P4S P11F D26N K34Y T65A Q327F | | 89.1 |
| GA076 | P2N P4S P11F T65A Q327F I375A E501V Y504T | | 90.2 |
| GA079 | P2N P4S P11F T65A K218A K221D Q327F E501V Y504T | | 90.9 |
| GA085 | P2N P4S P11F T65A S103N Q327F E501V Y504T | | 91.3 |
| GA086 | P2N P4S T10D T65A Q327F E501V Y504T | | 90.4 |
| GA088 | P2N P4S F12Y T65A Q327F E501V Y504T | | 90.4 |
| GA097 | K5A P11F T65A Q327F E501V Y504T | | 90.0 |
| GA101 | P2N P4S T10E E18N T65A Q327F E501V Y504T | | 89.9 |
| GA102 | P2N T10E E18N T65A Q327F E501V Y504T | | 89.8 |
| GA084 | P2N P4S P11F T65A Q327F E501V Y504T T568N | | 90.5 |
| GA108 | P2N P4S P11F T65A Q327F E501V Y504T K524T G526A | | 88.6 |
| GA126 | P2N P4S P11F K34Y T65A Q327F D445N V447S E501V Y504T | | 91.8 |

(continued)

| Po-AMG name | Mutations | DSC Td (°C) @ | DSC Td (°C) @ |
|---|---|---|---|
| | Mutations relative to PE001 | **pH 4.0** | **pH 4.8** |
| GA129 | P2N P4S P11F R31S K33V T65A Q327F D445N V447S E501V Y504T | | 91.7 |
| GA087 | P2N P4S P11F D26N K34Y T65A Q327F E501V Y504T | | 89.8 |
| GA091 | P2N P4S P11F T65A F80* Q327F E501V Y504T | | 89.9 |
| GA100 | P2N P4S P11F T65A K112S Q327F E501V Y504T | | 89.8 |
| GA107 | P2N P4S P11F T65A Q327F E501V Y504T T516P K524T G526A | | 90.3 |
| GA110 | P2N P4S P11F T65A Q327F E501V N502T Y504* | | 90.6 |

## Example 18

### Thermostability analysis by thermo-stress test and pNPG assay

Starting from one of the identified substitution variants from Example 10, identified as PE008, additional variants were tested by a thermo-stress assay in which the supernatant from growth cultures were assayed for glucoamylase (AMG) activity after a heat shock at 83°C for 5 min.

After the heat-shock the residual activity of the variant was measured as well as in a non-stressed sample.

Description of Po-AMG pNPG activity assay:

The *Penicillium oxalicum* glucoamylase pNPG activity assay is a spectrometric endpoint assay where the samples are split in two and measured thermo-stressed and non-thermo-stressed. The data output is therefore a measurement of residual activity in the stressed samples.

Growth:

A sterile micro titer plate (MTP) was added 200 microliters rich growth media (FT X-14 without Dowfax) to each well. The strains of interest were inoculated in triplicates directly from frozen stocks to the MTP. Benchmark was inoculated in 20 wells. Non-inoculated wells with media were used as assay blanks. The MTP was placed in a plastic box containing wet tissue to prevent evaporation from the wells during incubation. The plastic box was placed at 34°C for 4 days.

Assay:

50 microliters supernatant was transferred to 50 microliters 0.5 M NaAc pH 4.8 to obtain correct sample pH.

50 microliters dilution was transferred to a PCR plate and thermo-stressed at 83°C for 5 minutes in a PCR machine. The remaining half of the dilution was kept at RT.

20 microliters of both stressed and unstressed samples was transferred to a standard MTP. 20 microliters pNPG-substrate was added to start the reaction. The plate was incubated at RT for 1h.

The reaction was stopped and the colour developed by adding 50 microliters 0.5 M $Na_2CO_3$. The yellow colour was measured on a plate reader (Molecular Devices) at 405 nm.

Buffers:

0.5 M NaAc pH 4.8
0.25 M NaAc pH 4.8

Substrate, 6 mM pNPG:

15 mg 4-nitrophenyl D-glucopyranoside in 10 mL 0.25 NaAc pH 4.8

Stop/developing solution:

0.5 M $Na_2CO_3$

Data treatment:

In Excel the raw Abs405 data from both stressed and unstressed samples were blank subtracted with their respective blanks. The residual activity (% res. act. = ($Abs_{unstressed}$ ($Abs_{unstressed}$ - $Abs_{stressed}$))/$Abs_{unstressed}$ *100%) was calculated and plotted relative to benchmark, Po-amg0008.

Table 17

| Po-AMG name | Mutations | % residual activity |
|---|---|---|
| GA008 | P2N P4S P11 F T65A Q327F E501V Y504T | 100 |
| GA085 | P2N P4S P11F T65A S103N Q327F E501V Y504T | 127 |
| GA097 | K5A P11F T65A Q327F E501V Y504T | 106 |
| GA107 | P2N P4S P11F T65A Q327F E501V Y504T T516P K524T G526A | 109 |
| GA130 | P2N P4S P11F T65A V79A Q327F E501V Y504T | 111 |
| GA131 | P2N P4S P11F T65A V79G Q327F E501V Y504T | 112 |
| GA132 | P2N P4S P11 F T65A V79I Q327F E501V Y504T | 101 |
| GA133 | P2N P4S P11 F T65A V79L Q327F E501V Y504T | 102 |
| GA134 | P2N P4S P11F T65A V79S Q327F E501V Y504T | 104 |
| GA150 | P2N P4S P11 F T65A L72V Q327F E501V Y504T | 101 |
| GA155 | S255N Q327F E501V Y504T | 105 |

Table 18

| Po-AMG name | Mutations | % residual activity |
|---|---|---|
| GA008 | P2N P4S P11 F T65A Q327F E501V Y504T | 100 |
| GA179 | P2N P4S P11 F T65A E74N V79K Q327F E501V Y504T | 108 |
| GA180 | P2N P4S P11F T65A G220N Q327F E501V Y504T | 108 |
| GA181 | P2N P4S P11F T65A Y245N Q327F E501V Y504T | 102 |
| GA184 | P2N P4S P11F T65A Q253N Q327F E501V Y504T | 110 |
| GA185 | P2N P4S P11F T65A D279N Q327F E501V Y504T | 108 |
| GA186 | P2N P4S P11F T65A Q327F S359N E501V Y504T | 108 |
| GA187 | P2N P4S P11F T65A Q327F D370N E501V Y504T | 102 |
| GA192 | P2N P4S P11F T65A Q327F V460S E501V Y504T | 102 |
| GA193 | P2N P4S P11F T65A Q327F V460T P468T E501V Y504T | 102 |
| GA195 | P2N P4S P11F T65A Q327F T463N E501V Y504T | 103 |
| GA196 | P2N P4S P11F T65A Q327F S465N E501V Y504T | 106 |
| GA198 | P2N P4S P11F T65A Q327F T477N E501V Y504T | 106 |

**Example 19**

**Test for Glucoamylase activity of thermo-stable variants according to the invention**

All of the above described variants disclosed in tables 16, 17, and 18 have been verified for Glucoamylase activity on culture supernatants using the pNPG assay described in Example 18.

**Example 20** (Embodiment of the invention)

Ethanol Production Using (Alpha-Amylase 1407 or 369) and Protease Pfu for Liquefaction

The purpose of this experiment was to evaluate the application performance of Alpha-Amylase 1407 and Alpha-Amylase 369 in combination with Protease Pfu derived from *Pyrococcus furiosus* and added during liquefaction at pH 4.8, 5.3 and 5.8, at 85°C for 2 hours.

Liquefaction (Labomat)

Each liquefaction received ground corn (85.6% DS), backset (4.9% DS), and tap water targeting a total weight of 140 g at 32.50% Dry Solids (DS). Backset was blended at 30% w/w of total slurry weight. Initial slurry pH was approximately 5.2 and was adjusted to pH 4.8, 5.3 or 5.8 with 50% w/w sodium hydroxide or 40% v/v sulfuric acid prior to liquefaction. All enzymes were added according to the experimental design listed in Table 19 below. Liquefaction took place in a Labomat using the following conditions: 5°C/min. Ramp, 17 minute Ramp, 103 minute hold time, 40 rpm for the entire run, 200 mL stainless steel canisters. After liquefaction, all canisters were cooled in an ice bath and prepared for fermentation based on the protocol listed below under SSF.

Simultaneous Saccharification and Fermentation (SSF)

Each mash was adjusted to pH 5.0 with 50% w/w Sodium Hydroxide or 40% v/v sulfuric acid. Penicillin was applied to each mash to a total concentration of 3 ppm. The tubes were prepared with mash by aliquoting approximately 4.5 g of mash per 15 mL pre-drilled test tubes to allow $CO_2$ release.

Glucoamylase BL2 was dosed to each tube of mash at 0.54 AGU/g DS, minimal water was added to each tube to normalize solids, and each mash sample received 100 μL of rehydrated yeast. Rehydrated yeast was prepared by mixing 5.5 g of Fermentis RED STAR into 100 mL of 32°C tap water for at least 15 minutes.

HPLC analysis

Fermentation sampling took place after approximately 54 hours of fermentation. Each sample was deactivated with 50 μL of 40% v/v $H_2SO_4$, vortexing, centrifuging at 1460xg for 10 minutes, and filtering through a 0.45 μm Whatman PP filter. 54 hour samples were analyzed under HPLC without further dilution. Samples were stored at 4°C prior to and during HPLC analysis.

| | |
|---|---|
| HPLC system | Agilent's 1100/1200 series with Chem station software Degasser, Quaternary Pump, Auto-Sampler, Column Compartment /w Heater Refractive Index Detector (RI) |
| Column | Bio-Rad HPX- 87H Ion Exclusion Column 300mm x 7.8mm part# 125-0140<br>Bio-Rad guard cartridge cation H part# 125-0129, Holder part# 125-0131 |
| Method | 0.005 M $H_2SO_4$ mobile phase<br>Flow rate: 0.6 ml/min<br>Column temperature: 65°C<br>RI detector temperature: 55°C |

The method quantified analyte(s) using calibration standards for ethanol (% w/v). A four point calibration including the origin is used for quantification.

Where applicable, data were analyzed using JMP software (Cary, NC) with Oneway ANOVA of pairs using Tukey-Kramer HSD or Dunnett's. Error bars denoting the 95% confidence level were established by multiplying the standard error of Oneway Anova analysis by 1.96.

Table 19. Liquefaction Experiment Design

| | pH | Amylase | Dose | | PoAMG | Dose | Protease | Dose |
|---|---|---|---|---|---|---|---|---|
| **1** | 4.8 | | | | | | | |
| **2** | 5.3 | A | 0.02% | %w/w corn | none | | none | |
| **3** | 5.8 | | | | | | | |

(continued)

| | pH | Amylase | Dose | | PoAMG | Dose | Protease | Dose |
|---|---|---|---|---|---|---|---|---|
| **4**<br>**5** | 4.8<br>5.3 | AA1407 | 0.07718 | KNU-S/g corn | GA493 | 5 µg/gDS | Pfu | 1 µg/gDS |
| **6**<br>**7** | 4.8<br>5.3 | AA369 | 1.65 | KNU-S/g corn | GA493 | 5 µg/gDS | Pfu | 1 µg/gDS |
| **8**<br>**9**<br>**10** | 4.8<br>5.3<br>5.8 | A | 0.02% | %w/w corn | none | | none | |
| **11** | 4.8 | AA1407 | 0.07718 | KNU-S/g corn | GA493 | 5 µg/gDS | Pfu | 1 µg/gDS |
| **12** | 5.3 | | | | | | | |
| **13**<br>**14** | 4.8<br>5.3 | AA369 | 1.65 | KNU-S/g corn | GA493 | 5 µg/gDS | Pfu | 1 µg/gDS |

Results:

| **Enzymes** | **pH** | EtOH g/L | Level |
|---|---|---|---|
| AAA | 4.8 | 131.35 | B |
| AAA | 5.3 | 131.89 | B |
| AAA | 5.8 | 131.26 | B |
| AA1407 + GA493 + Pfu | 4.8 | 133.44 | A |
| AA1407 + GA493 + Pfu | 5.3 | 133.09 | A |
| AA369 + GA493 + Pfu | 4.8 | 131.64 | B |
| AA369 + GA493 + Pfu | 5.3 | 133.34 | A |

These results demonstrate that the thermostable alpha-amylase, glucoamylase, and thermostable protease can be used together in liquefaction to increase ethanol yield compared to Alpha-Amylase A (AAA) alone.

## Claims

**1.** A process for producing fermentation products from starch-containing material comprising the steps of:

i) liquefying the starch-containing material at a pH in the range between from above 5.0 to 7.0 at a temperature above the initial gelatinization temperature using:

- an alpha-amylase having at least 80% sequence identity to SEQ ID NO: 1;
- a protease having at least 80% identity to SEQ ID NO: 13 and having a thermostability value of more than 20% determined as Relative Activity at 80°C/70°C; and

ii) saccharifying using a carbohydrate-source generating enzyme;
iii) fermenting using a fermenting organism.

**2.** The process of claim 1, wherein the alpha-amylase is the *Bacillus stearothermophilus* alpha-amylase shown in SEQ ID NO: 1 herein.

**3.** The process of claim 1 or 2, wherein the alpha-amylase has a T½ (min) at pH 4.5, 85°C, 0.12 mM $CaCl_2$ of at least 10.

**4.** The process of any of claims 1-3, wherein the protease has a thermostability of more than 25% determined as Relative Activity at 80°C/70°C.

**5.** The process of any of claims 1-4, wherein the protease is derived from a strain of *Pyrococcus furiosus.*

**6.** The process of any of claims 1-5, further wherein a glucoamylase is present and/or added during liquefaction step i) and has a heat stability at 85°C, pH 5.3, of at least 20%.

**7.** The process of any of claims 1-6, further wherein a pullulanase is present during liquefaction and/or saccharification.

**8.** An enzyme composition comprising:

i) an alpha-amylase having at least 80% sequence identity to SEQ ID NO: 1;
ii) a protease having at least 80% identity to SEQ ID NO: 13 and having a thermostability value of more than 20% determined as Relative Activity at 80°C/70°C.

**9.** The composition of claim 8, wherein the alpha-amylase is the *Bacillus stearothermophilus* alpha-amylase shown in SEQ ID NO: 1 herein.

**10.** The composition of claim 8 or 9, wherein the alpha-amylase has a T½ (min) at pH 4.5, 85°C, 0.12 mM $CaCl_2$ of at least 10.

**11.** The composition of any of claims 8-10, wherein the protease has a thermostability of more than 25% determined as Relative Activity at 80°C/70°C.

**12.** The composition of any of claims 8-11, wherein the protease is derived from a strain of *Pyrococcus furiosus.*

**13.** The composition of any of claims 8-12, further comprising a glucoamylase.

**14.** The composition of claim 13, wherein the glucoamylasehas a heat stability at 85°C, pH 5.3, of at least 20%.

**15.** The composition of any of claims 8-14, further comprising a pullulanase.

## Patentansprüche

**1.** Verfahren zum Herstellen von Fermentationsprodukten aus stärkehaltigem Material, umfassend die Schritte:

i) Verflüssigen des stärkehaltigen Materials bei einem pH im Bereich von über 5,0 bis 7,0 bei einer Temperatur über der anfänglichen Geliertemperatur unter Verwendung von:

- einer alpha-Amylase mit mindestens 80% Sequenzidentität zu SEQ ID NO: 1;
- einer Protease mit mindestens 80% Identität zu SEQ ID NO: 13 und mit einem Thermostabilitätswert von mehr als 20%, bestimmt als relative Aktivität bei 80°C/70°C; und

ii) Verzuckern unter Verwendung eines eine Kohlenhydratquelle erzeugenden Enzyms;
iii) Fermentieren unter Verwendung eines fermentierenden Organismus.

**2.** Verfahren nach Anspruch 1, wobei die alpha-Amylase die in SEQ ID NO: 1 hierin gezeigte *Bacillus stearothermophilus* alpha-Amylase ist.

**3.** Verfahren nach Anspruch 1 oder 2, wobei die alpha-Amylase eine T½ (min) bei pH 4,5, 85°C, 0,12 mM $CaCl_2$ von mindestens 10 aufweist.

**4.** Verfahren nach einem beliebigen der Ansprüche 1-3, wobei die Protease eine Thermostabilität von mehr als 25%, bestimmt als relative Aktivität bei 80°C/70°C, aufweist.

**5.** Verfahren nach einem beliebigen der Ansprüche 1-4, wobei die Protease aus einem Stamm von *Pyrococcus furiosus* abgeleitet ist.

**6.** Verfahren nach einem beliebigen der Ansprüche 1-5, wobei weiterhin eine Glucoamylase vorhanden ist und/oder während Verflüssigungsschritt i) zugegeben wird und eine Hitzestabilität bei 85°C, pH 5,3 von mindestens 20% aufweist.

**7.** Verfahren nach einem beliebigen der Ansprüche 1-6, wobei weiterhin eine Pullulanase während Verflüssigung und/oder Verzuckerung vorhanden ist.

**8.** Enzymzusammensetzung, umfassend:

i) eine alpha-Amylase mit mindestens 80% Sequenzidentität zu SEQ ID NO: 1;
ii) eine Protease mit mindestens 80% Identität zu SEQ ID NO: 13 und mit einem Thermostabilitätswert von mehr als 20%, bestimmt als relative Aktivität bei 80°C/70°C.

**9.** Zusammensetzung nach Anspruch 8, wobei die alpha-Amylase die in SEQ ID NO: 1 hierin gezeigte *Bacillus stearothermophilus* alpha-Amylase ist.

**10.** Zusammensetzung nach Anspruch 8 oder 9, wobei die alpha-Amylase eine T½ (min) bei pH 4,5, 85°C, 0,12 mM $CaCl_2$ von mindestens 10 aufweist.

**11.** Zusammensetzung nach einem beliebigen der Ansprüche 8-10, wobei die Protease eine Thermostabilität von mehr als 25%, bestimmt als relative Aktivität bei 80°C/70°C, aufweist.

**12.** Zusammensetzung nach einem beliebigen der Ansprüche 8-11, wobei die Protease aus einem Stamm von *Pyrococcus furiosus* abgeleitet ist.

**13.** Zusammensetzung nach einem beliebigen der Ansprüche 8-12, weiterhin umfassend eine Glucoamylase.

**14.** Zusammensetzung nach Anspruch 13, wobei die Glucoamylase eine Hitzestabilität bei 85°C, pH 5,3 von mindestens 20% aufweist.

**15.** Zusammensetzung nach einem beliebigen der Ansprüche 8-14, weiterhin umfassend eine Pullulanase.

## Revendications

**1.** Procédé pour la production de produits de fermentation à partir d'une matière contenant de l'amidon, comprenant les étapes de :

i) liquéfaction de la matière contenant de l'amidon à un pH compris dans la plage comprise au-dessus de 5,0 à 7,0 à une température supérieure à la température initiale de gélatinisation, en utilisant :

- une alpha-amylase ayant une identité de séquence d'au moins 80 % avec la SEQ ID NO : 1 ;
- une protéase ayant une identité de séquence d'au moins 80 % avec la SEQ ID NO : 13 et ayant une valeur de thermostabilité supérieure à 20 %, déterminée en tant qu'activité relative à 80 °C/70 °C ; et

ii) saccharification en utilisant une enzyme génératrice d'une source d'hydrates de carbone ;
ii) fermentation en utilisant un organisme de fermentation.

**2.** Procédé selon la revendication 1, dans lequel l'alpha-amylase est l'alpha-amylase de *Bacillus stearothermophilus* indiquée ici dans la SEQ ID NO : 1.

**3.** Procédé selon la revendication 1 ou 2, dans lequel l'alpha-amylase a une T ½ (min) à pH 4,5, 85 °C, 0,12 mm de $CaCl_2$ d'au moins 10.

**4.** Procédé selon l'une quelconque des revendications 1 à 3, dans lequel la protéase a une thermostabilité supérieure à 25 %, déterminée en tant qu'activité relative à 80 °C/70 °C.

**5.** Procédé selon l'une quelconque des revendications 1 à 4, dans lequel la protéase est dérivée d'une souche de *Pyrococcus furiosus.*

**6.** Procédé selon l'une quelconque des revendications 1 à 5, dans lequel en outre une glucoamylase est présente et/ou ajoutée au cours de l'étape de liquéfaction i) et possède une stabilité à la chaleur à 85 °C, pH 5,3, d'au moins 20 %.

**7.** Procédé selon l'une quelconque des revendications 1 à 6, dans lequel en outre une pullulanase est présente au cours de la liquéfaction et/ou saccharification.

**8.** Composition enzymatique comprenant :

i) une alpha-amylase ayant une identité de séquence d'au moins 80 % avec la SEQ ID NO : 1 ;
ii) une protéase ayant une identité de séquence d'au moins 80 % avec la SEQ ID NO : 13 et ayant une valeur de thermostabilité supérieure à 20 %, déterminée en tant qu'activité relative à 80 °C/70 °C.

**9.** Composition selon la revendication 8, dans laquelle l'alpha-amylase est l'alpha-amylase de *Bacillus stearothermophilus* indiquée ici dans la SEQ ID NO : 1.

**10.** Composition selon la revendication 8 ou 9, dans laquelle l'alpha-amylase a une T ½ (min) à pH 4,5, 85 °C, 0,12 mm de $CaCl_2$ d'au moins 10.

**11.** Composition selon l'une quelconque des revendications 8 à 10, dans laquelle la protéase a une thermostabilité supérieure à 25 %, déterminée en tant qu'activité relative à 80 °C/70 °C.

**12.** Composition selon l'une quelconque des revendications 8 à 11, dans laquelle la protéase est dérivée d'une souche de *Pyrococcus furiosus.*

**13.** Composition selon l'une quelconque des revendications 8 à 12, comprenant en outre une glucoamylase.

**14.** Composition selon la revendication 13, dans laquelle la glucoamylase possède une thermostabilité à 85 °C, pH 5,3 d'aumoins 20 %.

**15.** Composition selon l'une quelconque des revendications 8 à 14, comprenant en outre une pullulanase.

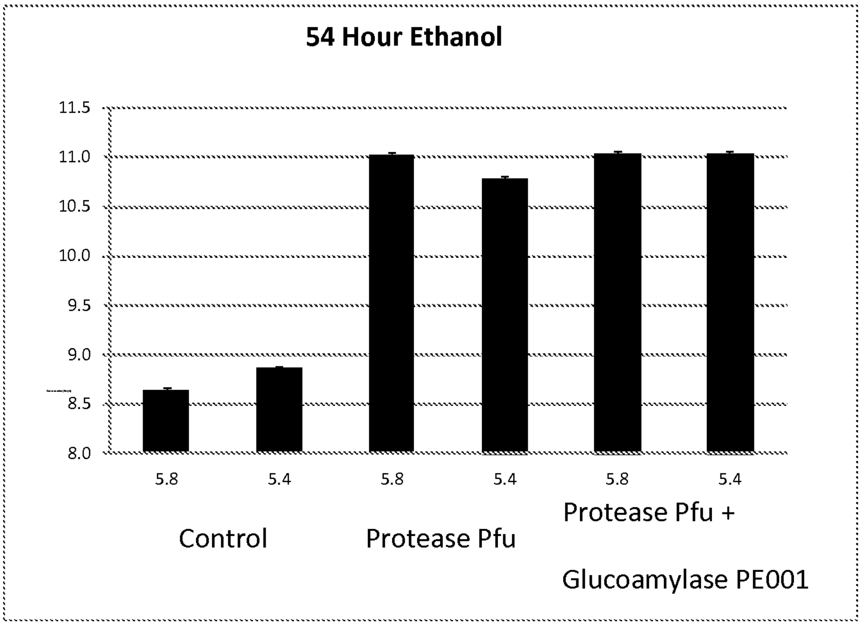
54 Hour Ethanol
11.5
11.0
10.5
10.0
9.5
9.0
8.5
8.0
5.8
5.4
5.8
5.4
5.8
5.4
Control
Protease Pfu
Protease Pfu +
Glucoamylase PE001

**Fig. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011072191 A
- WO 2006086792 A
- WO 2012088303 A
- WO 9919467 A
- WO 9623873 A
- WO 9623874 A
- WO 9741213 A
- WO 0060059 A
- WO 0210355 A
- US 6093562 A
- US 6187576 B
- US 6297038 B
- US 7713723 B
- US 6358726 B1
- CN 10071753 W
- WO 2011127802 A
- US 4560651 A
- WO 01151620 A
- WO 9202614 A
- US 61289040 B
- WO 2011087836 A
- WO 2011076123 A
- WO 9200381 A
- WO 0004136 A
- WO 0104273 A
- WO 8402921 A
- US 4727026 A
- WO 9928448 A
- US RE32153 E
- US 4587215 A
- EP 135138 A
- WO 8601831 A
- WO 2006069289 A
- WO 2007124285 A
- WO 2005045018 A
- US 61264977 B
- WO 2011066576 A
- US 61406741 B
- WO 2011068803 A
- WO 2012064351 A
- US 4598048 A
- US 4604355 A
- US 6162628 A
- US 6358726 B
- WO 2003048353 A
- WO 06069289 A
- WO 0669289 A
- WO 2006069290 A
- WO 0192502 A
- WO 03048353 A
- WO 200192502 A
- WO 2005042735 A1
- WO 9502043 A


### Non-patent literature cited in the description


- Handbook of Proteolytic Enzymes. Academic Press, 1998
- *FEMS Mic. Let.,* 1994, vol. 115, 97-106
- **BOEL et al.** *EMBO J.,* 1984, vol. 3 (5), 1097-1102
- *Agric. Biol. Chem.,* 1991, vol. 55 (4), 941-949
- **CHEN et al.** *Prot. Eng.,* 1996, vol. 9, 499-505
- **CHEN et al.** *Prot. Eng.,* 1995, vol. 8, 575-582
- **CHEN et al.** *Biochem. J.,* 1994, vol. 301, 275-281
- **FIEROBE et al.** *Biochemistry,* 1996, vol. 35, 8698-8704
- **LI et al.** *Protein Eng.,* 1997, vol. 10, 1199-1204
- **NAGASAKA et al.** Purification and properties of the raw-starch-degrading glucoamylases from Corticium rolfsii. *Appl Microbiol Biotechnol,* 1998, vol. 50, 323-330
- **W. R. PEARSON ; D. J. LIPMAN.** Improved Tools for Biological Sequence Analysis. *PNAS,* 1988, vol. 85, 2444-2448
- **W. R. PEARSON.** Rapid and Sensitive Sequence Comparison with FASTP and FASTA. *Methods in Enzymology,* 1990, vol. 183, 63-98
- **T. F. SMITH ; M. S. WATERMAN.** Smith-Waterman algorithm. *J. Mol. Biol.,* 1981, vol. 147, 195-197
- *J. Biol. Chem.,* 1997, vol. 272 (15), 9720-9727
- **SAMBROOK et al.** Molecular cloning: A laboratory manual. Cold Spring Harbor lab, 1989
- Current protocols in Molecular Biology. John Wiley and Sons, 1995
- PCR: A practical approach. Oxford University press, 207-209

- **LASSEN et al.** *Appl. Environ. Microbiol.,* 2001, vol. 67, 4701-4707
- **COVE.** *Biochim. Biophys. Acta,* 1996, vol. 133, 51-56
- *Biochem J.,* April 1975, vol. 147 (1), 45-53
- **ICHISHIMA.** *Biochemical journal,* 2003, vol. 371 (2), 541
- **GREGORY et al.** *J. Biomol. Screen.,* 2009, vol. 14, 700